# FASCICULE DE BREVET EUROPEEN

(11) **EP 3 160 517 B1**
(45) Date de publication et mention de la délivrance du brevet: **12.06.2024**
(21) Numéro de dépôt: 15742341.9
(22) Date de dépôt: 24.06.2015
(51) Int. Cl.: A61K 49/00, C07D 311/04, C07D 239/72, C07D 249/04, A61K 47/22

(54) **SONDES MOLÉCULAIRES ACTIVABLES HYDROSOLUBLES, INTERMÉDIAIRES POUR LEUR SYNTHÈSE ET PROCÉDÉS DE DÉTECTION ASSOCIÉS**
AKTIVIERBARE WASSERLÖSLICHE MOLEKULARE SONDEN, ZWISCHENPRODUKTE ZUR SYNTHESE DAVON UND ZUGEHÖRIGE DETEKTIONSVERFAHREN
WATER-SOLUBLE ACTIVATABLE MOLECULAR PROBES, INTERMEDIATES FOR THE SYNTHESIS THEREOF AND ASSOCIATED DETECTION METHODS

(30) Priorité: 26.06.2014 FR 1456014
(43) Date de publication de la demande: 03.05.2017
(73) Titulaire: Ecole Normale Supérieure de Lyon, 69342 Lyon Cedex 07 (FR); Université Claude Bernard Lyon I, 69622 Villeurbanne Cedex (FR); Centre National de la Recherche Scientifique (C.N.R.S.), 75794 Paris Cedex 16 (FR)
(72) Inventeur: HASSERODT, Jens, 69364 Lyon (FR); PROST, Maxime, 69007 Lyon (FR)
(74) Mandataire: Cabinet Becker et Associés
(86) Numéro de dépôt international: PCT/FR2015/051705
(87) Numéro de publication internationale: WO 2015/197981

(56) Documents cités:
- WO-A1-00/59510
- WO-A1-2013/045854
- WO-A1-2014/020285
- WO-A2-2007/146066
- WO-A2-2012/122420
- Maxime Prost: "Sondes moléculaires comprenant des espaceurs auto-effondrables multifonctionnels pour la détection d'activités enzymatiques - Ecole Normale Supérieure de Lyon", , 3 juillet 2014 (2014-07-03), XP055164724, Extrait de l'Internet: URL:http://www.ens-lyon.eu/recherche/sonde s-moleculaires-comprenant-des-espaceurs-au to-effondrables-multifonctionnels-pour-la- detection-d-activites-enzymatiques-232636. kjsp?RH=THESE_SOUT [extrait le 2015-01-26]

## Description

L'invention concerne des sondes moléculaires activables utiles dans le domaine du diagnostic, incorporant un bras espaceur, qui en réponse à un stimulus qui peut notamment être la présence d'une enzyme, d'un composé chimique ou une caractéristique physicochimique du milieu dans lequel se trouve la sonde, va s'auto-effondrer pour libérer une entité détectable du type fluorophore ou chromophore.

Dans l'analyse d'un échantillon biologique ou chimique, la détection d'une activité enzymatique peut être très utile. Les organismes entiers, les cellules ou les extraits cellulaires, les liquides biologiques ou les mélanges chimiques sont des exemples d'échantillons biologiques ou chimiques dans lesquels une activité enzymatique peut être détectée. Les enzymes constituent de nombreux biomarqueurs de diverses pathologies. Elles sont impliquées aussi dans de nombreux processus cellulaires et font donc l'objet d'études innombrables de la part des biologistes cellulaires. Ainsi, leur détection peut donner des informations concernant un état métabolique ou morbide particulier, par exemple. De ce fait, des sondes capables de détecter une activité enzymatique sont très utiles et ont fait l'objet de nombreux travaux.

On peut notamment citer les travaux de l'un des inventeurs de la présente demande de brevet correspondant aux demandes WO 2013/045854 qui concerne des substrats de peptidases et WO 2014/020285 qui concerne des substrats de glycosidases qui utilisent un espaceur auto-effondrable reliant un substrat de l'enzyme d'intérêt à un groupe aryle entraînant, après clivage du substrat, la cyclisation de l'espaceur et la libération d'un fluorophore ESIPT (fluorophore conduisant à un transfert de proton intramoléculaire dans un état excité, nommé ESIPT pour « Excited State Intramolecular Proton Transfer »).

Or, une bonne solubilité aqueuse des sondes moléculaires répondant à une activité enzymatique est un des critères essentiels pour s'assurer de la fiabilité des résultats qu'elles délivrent. En effet, l'ajout de co-solvant organique pour dissoudre une molécule hydrophobe diminue généralement l'activité des enzymes de manière importante. De plus, alors que l'utilisation de solutions faiblement concentrées est acceptable pour certains tests *in vitro*, le passage à des incubations cellulaires ou des injections systémiques *in vivo* requiert l'utilisation de solutions à des concentrations bien plus élevées car elles vont être diluées de manière importante une fois injectées. De plus, l'utilisation d'un co- solvant *in vivo* est exclue. Enfin, une molécule trop hydrophobe risque de s'accumuler dans les tissus adipeux, et non pas dans les tissus ciblés.

Cette question de solubilité est donc depuis longtemps une préoccupation pour les chimistes et plusieurs solutions ont été développées. La première a été d'incorporer des groupements ayant un caractère salin en tant que substrats enzymatiques. Cette solution, telle que rencontrée dans des sondes pour phosphatase (Haugland et at Anal. Biochem. 1992, 207, 32) ou caspase-3 (Liu et al. J. Am. Chem. Soc. 2012, 134, 17972) par exemple, fonctionne très bien mais elle limite le choix des enzymes visées puisque celles qui reconnaissent des substrats apolaires ne peuvent être ciblées. De plus, les molécules chargées négativement traversent difficilement la membrane cellulaire ce qui limite donc l'application au ciblage d'enzymes extracellulaires. Une seconde solution est le greffage de groupements hydrosolubilisants sur le signalophore utilisé, souvent des groupements sulfonates (Tung et al. Tetrahedrun 2006, 62, 578 ; Shabat et al. J. Am. Chem. Soc 2011, 133, 10960). Encore une fois, l'hydrosolubilisation apportée par ces motifs est excellente, mais cette mesure est peu attractive car elle risque d'abaisser les performances du signalophore une fois relâché de la sonde initiale suite à l'activité enzymatique. De plus, une telle solution est tout simplement inenvisageable lorsque l'on veut employer des signalophores à l'état solide. Ces deux possibilités ne sont donc pas généralisables au plus grand nombre de sondes moléculaires répondant à une activité enzymatique.

A ce jour, il n'existe que très peu de systèmes qui permettent l'association versatile d'une espèce hydrosolubilisants à un substrat enzymatique dans le but de relarguer une molécule optiquement active. Ceux-ci sont généralement basés sur un espaceur éliminant substitué (Shabat et al. J. am. Chem. Soc. 2008, 130, 5434) ou sur une combinaison d'un espaceur cyclisant et d'espaceurs éliminants (Shabat et al. Bioorg. Med. Chem. 2007, 15, 7318). Le problème est que les espaceurs éliminants substitués sont connus pour produire des intermédiaires réactionnels hautement toxiques (Rokita, Quinone Methides, 1ère éd. 2009, John Wiley and Sons Inc. ; Bolton et al. Chem. Res. Toxicol. 1995, 8, 323 ; Thatcher et al. J. Org. Chem. 1997, 62, 1820)*.* D'autre part, la combinaison cyclisation/élimination est loin d'être idéale car elle introduit une forte complexité (nécessité d'utiliser deux espaceurs en série) tout en démontrant des cinétiques d'effondrement qui sont loin d'être satisfaisantes (Wu et al. Bioorg. Med. Chem. 2012, 20, 3465), ou bien une instabilité face à l'hydrolyse spontanée non négligeable (Romieu et al Org. Lett 2008, 10, 1517). Certaines constructions livrent leur molécule active en plusieurs dizaines d'heures (Shabat et al. Bioconjugate Chem. 2006, 17, 1432), ce qui les rend difficilement utilisables pour détecter rapidement la présence ou l'absence de certains analytes dans un échantillon donné.

Il est donc souhaitable de développer une nouvelle génération d'espaceurs cyclisant permettant l'incorporation versatile de n'importe quel substrat enzymatique, qu'il soit polaire ou apolaire, et qui répondent aux exigences de solubilité. Néanmoins, cet espaceur devra garder une cinétique d'effondrement rapide et devra permettre une reconnaissance enzymatique efficace.

Dans le cadre de l'invention, les inventeurs proposent une nouvelle génération de sondes qui, d'une part, offre une solubilité dans l'eau satisfaisante et, d'autre part, sont facilement synthétisables et modulables, du fait de la présence d'un groupe pipérazine dont l'un des atomes d'azote peut porter divers groupes hydrosolubilisants. Les inventeurs ont choisi de proposer une solution qui incorpore une partie hydrosolubilisante sur une 3^{ème} composante de la sonde, à savoir dans un espaceur reliant le signalophore et le substrat ce qui permet de choisir à volonté n'importe quel substrat enzymatique et n'importe quelle molécule rapportrice.

L'invention est exposée dans le jeu de revendications joint.

La présente invention permet de détecter un analyte chimique ou enzymatique capable de cliver la liaison **X₁-SE,** par la détection du chromophore ou fluorophore qui va être libéré suite à la cyclisation de l'espaceur du type pipérazinyle de formule :

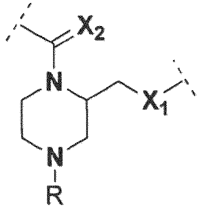

Le substrat selon l'invention agit comme une sonde moléculaire capable de révéler la présence d'un stimulus tel qu'une enzyme, un composé chimique ou une caractéristique physicochimique du milieu dans lequel se trouve la sonde (notamment un changement de pH). Quand la sonde est modifiée chimiquement par ledit stimulus, elle se fragmente via une réaction en cascade pour libérer un chromophore ou fluorophore détectable. Il est possible que le groupement **A** se comporte également comme un chromophore ou un fluorophore lorsqu'il est lié au reste de la molécule dans les sondes de formule (I). Dans ce cas de sondes « ratiométriques », ses propriétés spectrales, et en particulier ses propriétés d'absorbance et/ou de fluorescence seront différentes de l'entité libérée sous l'action d'un stimulus tel qu'une enzyme, un composé chimique ou une caractéristique physicochimique du milieu dans lequel se trouve la sonde. De manière avantageuse mais non obligatoire, le groupement **A** pourra être choisi, de manière à ce que la sonde de formule (I) ne soit pas détectable par absorption ou émission de lumière, avant de rencontrer le stimulus, sous la forme notamment d'une enzyme, d'un composé chimique ou d'une caractéristique physicochimique du milieu dans lequel se trouve la sonde, (c'est-à-dire que la sonde est dite « furtive »).

Le groupe labile **SE** est tel que son élimination, par exemple par voie enzymatique ou consécutivement à une action purement chimique (telle qu'une variation de pH ou la présence d'un agent réducteur), assure, par réarrangement intramoléculaire de l'espaceur auto-effondrable, une libération du fluorophore ou chromophore, notamment sous la forme **A-**OH ou **A-**O⁻, ou **A**'=O (**A'**=O étant une forme obtenue après réarrangement de **A**-OH, une forme tautomérique ou polymérisée de **A**-OH). Le clivage de la liaison **SE-X₁** déclenche la libération du fluorophore ou chromophore, notamment sous **A**-OH ou **A**-O⁻, ou **A'**=O (**A'**=O étant une forme obtenue après réarrangement de **A**-OH ou **A**-O⁻, une forme tautomérique ou polymérisée de **A**-OH), par cyclisation de l'espaceur comme illustré sur le **Schéma 1** ci-dessous :

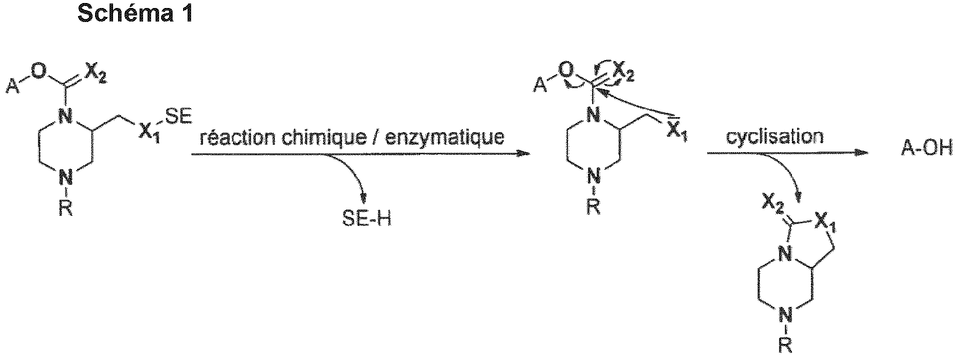

La sonde est composée de quatre composants moléculaires i) un espaceur intelligent qui porte, à une extrémité, ii) un groupe labile **SE** et, à l'autre, iii) un groupe organique **A-O** relié par un groupement carbamate ou thiocarbamate de l'espaceur qui, quand il est libéré par fragmentation du conjugué espaceur-**A**-O, pour donner le composé énolique ou énolate correspondant, conduit à la formation d'un chromophore ou fluorophore détectable, iv) un groupement hydrosolubilisant.

Dans le cadre de l'invention, la présence de l'atome d'azote porteur du groupement **R** permet de moduler les propriétés de la sonde et d'introduire une troisième fonctionnalisation, à côté du groupe labile **SE** et du groupe **A** conduisant, après élimination du groupe **SE** et à la cyclisation de l'espaceur, à la libération d'un chromophore ou fluorophore détectable.

En effet, les sondes de formule (I) selon l'invention comportent un espaceur de type pipérazine multifonctionnalisable qui permet l'incorporation d'un large choix de substrats enzymatiques, et d'un groupe annexe **R** qui permettra, en fonction de sa nature, de rendre la molécule encore plus hydrosoluble. La présence de l'atome d'azote, permet à partir d'un même intermédiaire de synthèse de conduire à différentes fonctionnalisations **R** hydrosolubilisantes.

De plus, les sondes selon l'invention présentent une solubilité améliorée, par rapport à leurs homologues comprenant un espaceur du type pipéridinyle, exemplifiés dans les demandes WO 2013/045854 et WO 2014/020285 et par rapport aux réactifs commerciaux ne comprenant pas du tout d'espaceur tels que la 4-méthyl-7-(phénylacétamido)coumarine ou l'acétate de 4-méthylumbelliferyle par exemple :

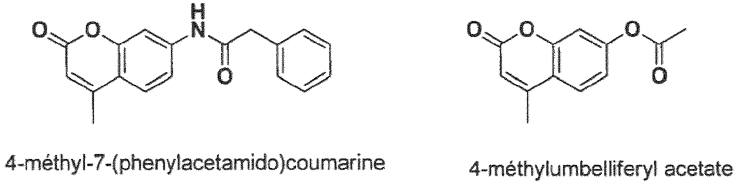

Par ailleurs, comme dans le cas des demandes WO 2013/045854 et WO 2014/020285, la sonde comporte un espaceur pré-organisé pour sa cyclisation après clivage de la liaison **SE-X₁** qui accélère le processus d'auto-effondrement de l'espaceur, tout en assurant une bonne reconnaissance enzymatique. Or, Il n'était nullement évident qu'avec la modification de l'espaceur opérée dans le cadre de l'invention dans laquelle des groupements sont introduits pour augmenter l'hydrosolubilité, il n'y ait pas d'impact sur l'activité rapportrice des sondes. Or, dans le cadre de l'invention, il a été démontré que l'introduction d'une charge ou l'augmentation de l'encombrement stérique au niveau du groupe **R,** et donc sur une position proche du site d'action du stimulus (te. une enzyme, un composé chimique ou une variation d'une caractéristique physicochimique du milieu dans lequel se trouve la sonde, en fonction de la nature du groupe **SE**), ne modifiait pas la réaction obtenue sous l'action du stimulus correspondant.

La présente invention concerne donc les composés de formule (I), quelle que soit leur variante de mise en oeuvre décrite dans la présente demande de brevet, pour la détection *in vivo,* chez l'homme ou l'animal, *in vitro, in cellulo* ou *ex vivo*, d'un stimulus tel qu'une enzyme, un composé chimique ou une caractéristique physicochimique du milieu dans lequel se trouve la sonde, dont la présence entraîne le clivage de la liaison **SE-X₁.**

Selon un autre aspect, l'invention concerne un procédé in vitro pour détecter, au moyen d'une sonde de formule (I) selon l'invention, la présence d'un stimulus tel qu'une enzyme, un composé chimique ou une caractéristique physicochimique du milieu dans lequel se trouve la sonde, dont la présence entraîne le clivage de la liaison **SE-X₁.** Plus précisément, l'invention concerne un procédé pour détecter la présence d'un stimulus tel qu'une enzyme, un composé chimique ou une caractéristique physicochimique du milieu dans lequel se trouve la sonde, dont la présence entraîne le clivage de la liaison **SE-X₁,** comprenant les étapes de :
- mise en contact d'un échantillon suspecté de contenir ledit composé chimique ou ladite enzyme ou de présenter ladite caractéristique physicochimique avec une sonde de formule (I) selon l'invention ;
- l'application de conditions appropriées pour permettre le clivage de la liaison covalente entre **X₁** et **SE,** sous l'action dudit stimulus, qui est suivi d'un clivage de la liaison C-O entre l'atome de carbone porteur de l'atome (**X₂**), et l'atome d'oxygène porteur du groupement A suite à une cyclisation de l'espaceur présent dans la sonde de formule (I), et
- analyse quantitative ou qualitative du chromophore ou fluorophore libéré.

L'échantillon pourra être tout échantillon biologique approprié. Il pourra s'agir notamment d'un prélèvement de fluide biologique, notamment un échantillon de sang total, de sérum, plasma, urine, d'un prélèvement tissulaire ou de cellules isolées, et en particulier d'un milieu cellulaire. Ce prélèvement peut être utilisé tel quel, ou bien être soumis avant mise en présence de la sonde, à une préparation de type enrichissement ou culture, bien connue de l'homme de l'art.

De manière avantageuse, les composés de formule (I) se comportent comme des sondes fonctionnant selon un mode éteint/allumé, c'est-à-dire qu'avant clivage par le stimulus (ie. une enzyme, un composé chimique ou une variation d'une caractéristique physicochimique du milieu dans lequel se trouve la sonde, en fonction de la nature du groupe **SE**), la sonde est « invisible ». Il est également possible de choisir le groupement **A,** de manière à obtenir des propriétés spectrales différentes entre i) le groupement libéré après clivage de la liaison C-O entre l'atome de carbone porteur de l'atome (**X₂**) et l'atome d'oxygène porteur du groupement A et ii) la sonde de formule (I).

En particulier, le procédé de détection selon l'invention peut être mis en oeuvre dans des conditions physiologiques, notamment dans un milieu aqueux tamponné à un pH appartenant à la gamme allant de 4 à 9, et notamment à un pH de l'ordre de 7,4.

Dans un mode de réalisation de l'invention, un fluorophore est libéré et son analyse comprend les étapes suivantes :
- exposition du fluorophore à une source lumineuse capable de produire une lumière à une longueur d'onde d'absorption du fluorophore ; et
- détection de la fluorescence résultante.

Dans un autre mode de réalisation de l'invention, un chromophore est libéré et son analyse comprend les étapes suivantes :
- exposition du chromophore à une source lumineuse capable de produire une lumière à une longueur d'onde d'absorption du chromophore ; et
- détection de la lumière absorbée par le chromophore.

Dans le cas où un chromophore est libéré, on détectera un changement de couleur suite à l'action du stimulus (ie. une enzyme, un composé chimique ou une variation d'une caractéristique physicochimique du milieu dans lequel se trouve la sonde, en fonction de la nature du groupe **SE**).

De manière préférée, mais non nécessaire, le fluorophore ou le chromophore libéré, en particulier sous la forme **A**-OH ou **A**-O⁻, ou **A'**=O (**A'**=O étant une forme obtenue après réarrangement de **A**-OH ou **A**-O⁻, une forme tautomérique ou polymérisée de **A**-OH), forme un précipité en solution aqueuse.

L'invention va maintenant être décrite de manière plus détaillée.

### Définition des groupes labiles SE

**SE** est un groupe labile, éliminable en solution aqueuse sous l'action d'un stimulus qui peut notamment être la présence d'une enzyme, d'un composé chimique ou d'une caractéristique physicochimique du milieu dans lequel se trouve la sonde. L'élimination de **SE** peut notamment se faire en milieu aqueux. A titre d'exemple d'une propriété physicochimique, on peut citer une variation de pH, ou la variation du potentiel redox, la dernière clivant les ponts disulfures présents notamment dans les cas où **X₁** = S. De manière préférée, notamment dans les cas où **X₁** = NH ou O, le groupe labile **SE** est susceptible à un composé chimique (un réactif cible) ou une enzyme. Une enzyme est définie comme une protéine naturelle qui catalyse une réaction chimique.

De manière préférée, le groupe labile **SE** assure la reconnaissance d'une cible (composé chimique ou enzymatique, paramètre physico-chimique du milieu), et notamment d'une enzyme, sélectivement localisée dans un tissu ou dans un type de cellules visé.

Les groupements **SE** ont la caractéristique de pouvoir être clivés du reste de la molécule sous l'action d'un stimulus qui peut notamment être la présence d'une enzyme, d'un composé chimique ou d'une caractéristique physicochimique du milieu dans lequel se trouve la sonde. Dans le cas où **SE** est un substrat enzymatique, l'enzyme joue alors le rôle de catalyseur de la coupure entre **SE** et l'atome **X₁** auquel il est lié. Une telle coupure est la conséquence d'une hydrolyse en milieu aqueux pour laquelle l'enzyme va jouer le rôle de catalyseur. Bien que dans ce cas également, la coupure mette en jeu une hydrolyse, on parlera de clivage sous l'action d'une enzyme qui est catalytiquement active pour la réaction d'hydrolyse.

Le choix de **X₁** sera adapté en fonction du groupe **SE,** et notamment du substrat d'enzyme sélectionné, de manière à obtenir le clivage souhaité. En particulier, **X₁** sera un oxygène dans le cas où **SE** sera un substrat d'estérase, de glycosidase, de phosphatase, de sulfatase, ou de glucuronidase, **X₁** sera NH dans le cas où **SE** sera un substrat de protéase, notamment d'amidase, ou de transférase.

En particulier, l'homme du métier, en fonction de l'application visée pour les sondes selon l'invention, pourra choisir un substrat pour au moins une enzyme choisie parmi les estérases telles que les carboxylestérases ou les lipases ; les alkaline-phosphatases ; les glucuronidases telles que la β- glucuronidase ; les glycosidases ; les protéases telles que les métalloprotéases ; les peptidases et les amidases.

Le groupement **SE** sera, de préférence, choisi de manière à être spécifique d'une enzyme d'intérêt. En revanche, certaines enzymes ont la capacité de cliver un ensemble de groupements **SE** différents ; parmi elles, on peut citer la hexosaminidase, et les estérases.

De tels substrats d'enzyme seront notamment choisis parmi les groupes glycosyle liés par leur carbone anomérique au reste de la molécule, des acides gras liés par leur fonction acyle au reste de la molécule, les groupes peptidyle liés au reste de la molécule par une fonction acyle portée par leur carbone terminal ou par un chaînon latéral, ou un groupe sulfonyle formant un sulfonamide avec **X₁** qui représente alors NH.

En particulier, **SE** peut être un substrat de glycosidase, et notamment de galactosidase et correspondre à groupement glycosyle lié par son carbone anomérique au reste de la molécule.

Par « glycosidase », on entend une enzyme glycoside hydrolase qui a la capacité de catalyser l'hydrolyse de liaisons glycosidiques, de manière à libérer au moins un composé osidique.

Par groupe « glycosyle », on entend tout sucre, mono ou polysaccharide lié au reste de la molécule par un lien glycosyle, c'est-à-dire via son carbone anomérique. Le carbone anomérique peut adopter la configuration alpha ou beta. A titre d'exemple de groupe **SE,** on peut citer les groupements mono-glycosyle, c'est-à-dire formé d'une seule unité saccharidique et poly-glycosyle, c'est-à-dire formé de plusieurs unités saccharidiques identiques ou différentes. Les unités saccharidiques peuvent être notamment du type hexose ou pentose, et choisies parmi le galactose, le glucose, le mannose, le gulose, l'allose, l'altrose, l'idose, le talose, le fucose, le fructose, l'arabinose, le lyxose, le ribose et le xylose, par exemple. Les unités saccharidiques peuvent être de stéréochimie L ou D. Tous les groupements glycosyle possibles constituant des substrats de glycosidase peuvent être utilisés en tant que **SE.** Les unités glycosyle peuvent être fonctionnalisées ou non, notamment avec un groupement acétyl ou amino. Les N-acétylhexosamines sont des exemples de groupe glycosyle. Le plus souvent, le groupe **SE** comprendra de 1 à 50 unités saccharidiques. Dans le cas d'un poly-glycosyle, il pourra s'agir d'un homopolymère ou d'un copolymère avec une structure aléatoire, alternée ou bloc.

Des exemples de tels groupes **SE** se comportant comme des substrats de glycosidase sont donnés ci-après : les groupements mono-glycosylés choisis parmi le galactosyle, le glucosyle, le mannosyle, le gulosyle, l'allosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle et les groupements polyglycosylés constitués de plusieurs (notamment 2 à 20, de préférence de 3 à 10, et plus particulièrement de 4 à 6) de ces groupements monoglycosylés identiques ou différents.

A titre d'exemples d'enzyme glycosidase qui peuvent êtres ciblés par les sondes selon l'invention, on peut citer la N-acétyl-β-galactosaminidase ; la N-acétyl-β-glucosaminidase ; la α-amylase ; la α-arabinofuranosidase ; la α-arabinosidase ; la β-cellobiosidase ; la β-chltobiosidase ; la α-galactosidase ; la β-galactosidase ; la α-glucosidase ; la β-glucosidase ; la β-glucuronidase ; la α-maltosidase ; la α-mannosidase ; la β-mannosidase ; la β-xylosidase ; la β-D-fucosidase ; la α-L-fucosidase ; la β-L-fucosidase ; la L-iduronidase et la cellulase (Orenga, S., James, A. L, Manafi, M., Perry, J. D., & Pincus, D. H. (2009). Enzymatic substrates in microbiology. Journal of Microbiological Methods, 75(2), 139-155).

**SE** peut également être un substrat de galactosidase, et en particulier de la β-galactosidase, de l'induronidase, de la glucosidase, de la N-acétyl-D-glucosaminidase, de la N-acétyl-D-galactosaminidase, de la mannosidase, de la fucosidase ou de la glucuronidase, notamment de la β-glucuronidase.

Des exemples de tels groupes **SE** se comportant comme des substrats de galactosidase sont donnés ci-après : les groupements mono-glycosylés choisis parmi le D-glucuronyle, le L-iduronyle, le D-glucopyranosyle, le D- galactopyranosyle, le N-acétyl-D-glucosaminyle, le N-acétyl-D- galactosaminyle, le D-mannopyranosyle, le L-fucopyranosyle et les groupements polyglycosylés constitués de plusieurs (notamment 2 à 20, de préférence de 3 à 10, et plus particulièrement de 4 à 6) de ces groupements monoglycosylés identiques ou différents.

**SE** peut aussi être un substrat d'estérase, notamment de lipase. Dans ce cas, **SE** est un groupe acyle, lié par sa fonction C(O) au reste de la molécule. En particulier, **SE** peut être -C(O)Ri avec Ri qui représente, par exemple, un groupe alkyle de préférence de 1 à 20 atomes de carbone, alcényle de préférence de 1 à 20 atomes de carbone, un groupe benzyle, aryle ou hétéroaryle.

SE peut aussi être un substrat d'une protéase ou d'une peptidase, notamment un substrat de cathepsine ou de métalloprotéase, notamment de la PSMA (Prostate Spécifie Membrane Antigen).

Dans ce cas, **SE** est un groupe peptidyle ou aminoacide lié par une fonction acyle portée par son carbone terminal ou par un chaînon latéral au reste de la molécule.

Par groupe « peptidyle », on entend un enchaînement d'au moins deux acides aminés liés entre eux par une liaison peptidique. Dans le cadre de l'invention, le ou les acides aminés présents dans **SE** peuvent être des acides aminés naturels ou non, mais seront, de préférence, choisis parmi les 20 acides aminés naturels ( = protéinogéniques), éventuellement sous une forme salifiée ou protégée. La fonction N-terminale de l'acide aminé terminal pourra éventuellement être salifiée ou fonctionnalisée. A titre d'exemple de forme salifiée, on peut citer la forme chlorhydrate, tosylate ou trifluoroacétate.

Tous les groupes peptidyle ou aminoacides possibles constituant des substrats de peptidases peuvent être utilisés en tant que groupe **SE.** Les aminoacides peuvent être fonctionnalisés ou non, en particulier sur l'extrémité N-terminale de **SE.** Selon des modes de réalisation particuliers, le résidu peptidyle **SE** a au plus 10 aminoacides qui peuvent être identiques ou différents. Les aminoacides du résidu peptidyle SE sont, de préférence, choisis parmi les aminoacides naturels. Néanmoins, l'extrémité N-terminale de l'aminoacide ou du groupe peptidyle peut être fonctionnalisée avec un groupe acyle -COR₀, Ra étant un groupe (C₁-C₆)alkyle ou un groupe -O-(C₁-C₆)alkyle. La fonctionnalisation possible de l'extrémité N-terminale d'une sonde donnée avec un groupe acyle (R₀CO-) provient du fait que certaines endoprotéases n'interagiront pas avec un substrat ayant un groupe amino libre à son extrémité. Aussi, cette fonctionnalisation N-terminale sera préférée dans le cas où **SE** représente un groupe peptidyle, alors que dans le cas où **SE** représente un acide aminé, sa fonction N-terminale sera libre, ou de préférence salifiée sous la forme d'un ammonium. La synthèse peptidique en phase solide (SPPS) au moyen de groupes protecteurs de carbamate (qui constituent aussi des groupes "acyle" dans ce contexte) permet souvent de synthétiser plus simplement une sonde présentant un carbamate à cette extrémité N-terminale. Dans le cas des amino-peptidases, on préférera utiliser un groupe **SE** qui représente un acide aminé, alors que dans le cas des endopeptidases, on préférera utiliser un groupe SE qui représente un groupe peptidyle.

A titre d'exemples, les groupes peptidyle suivants peuvent être cités : Leu (pour la leucine aminopeptidase), Ser-Gln-Asn-Tyr (la partie N-terminale de la séquence de clivage préférée de la peptidase du VI H-1), Asp-Glu-Val-Asp- (pour la caspase 3), His-Ser-Ser-Lys-Leu-GIn (pour l'antigène spécifique à la prostate « PSA ») où l'extrémité N-terminale peut être libre ou être substituée par un groupe acyle -COR₀, Ra étant un groupe (C₁-C₆)alkyte ou un groupe -O- (C₁-C₆)alkyle (par exemple un groupe -COMe).

Le groupe peptidyle ou aminoacide est choisi pour son adéquation avec la sélectivité de séquence connue de la peptidase ciblée, qui le reconnaîtra. Le groupe peptidyle ou aminoacide peut être choisi pour l'action préférée qu'une peptidase impliquée dans certaines maladies va avoir sur ce dernier, comme présenté, notamment, dans le **Tableau 1.**

**Tableau 1 : Exemples de peptidases qui peuvent servir de biomarqueurs pour des phénomènes biologiques ou des maladies pendant l'imagerie**

| Peptidase | Classe | Fonction | Maladie |
|---|---|---|---|
| Leudne aminopeptidase | Zinc | Maturation post-protéasomale de peptides présentés de classe I | - |
| Caspase-3 | Cys | Apoptose | Cancer |
| Peptidase de VIH-1 | Asp | Réplication de VIH | SIDA |
| Rénine | Asp | Production d'angiotensine I | Hypertension |
| Thrombine | Ser | Coagulation sanguine | Infarctus du myocarde |
| Tryptase | Ser | Phagocytose | Asthme |
| Cathepsine K | Cys | Résorption osseuse | Ostéoporose |
| ACE | Zinc | Production d'angiotensine II | Hypertension |
| Plasmepsines I et II | Asp | Dégradation de l'hémoglobine | Malaria |
| β-Secrétase | Asp | Synthèse d'amyloïde β | Maladie d'Alzheimer |
| PSA (kallikrein III) | Ser | Liquification de l'éjaculat de sperme | Cancer de la prostate |

**SE** peut aussi être un substrat d'amidase, catalyseur de l'hydrolyse de liaisons amides non peptidiques, notamment un substrat des pénicilline amidases, d'hydrolase d'amide d'acides gras, ou encore des malonamidases.

**SE** peut aussi être un substrat de transférase, catalyseur du transfert de groupement fonctionnel d'un substrat à un autre, notamment un substrat des transaminases, ou de la glutathione transférase ou de la gamma- glutamyl/transférase. Dans ce cas, **SE** correspond à un groupe peptidyle ou aminoacide lié par une fonction acyle portée par son carbone terminal ou par un chaînon latéral ou un groupe sulfonyle formant un sulfonamide avec **X₁** qui représente alors NH.

Les substrats de protéases sont bien connus et notamment décrits dans le « Handbook of proteolytic enzymes » (Ralings et Salvesen, 2013, 3ème édition, Elsevier Ltd) et dans la base de données MEROPS accessible en ligne (http://merops.sanger.ac.uk/index.shtml, Bateman et al. Nucleic acid res. 2014, 42, D503), auquel on pourra se référer pour plus de détails.

**SE** peut aussi être un substrat réagissant avec un composé chimique, et notamment un groupe formant un pont disulfure avec **X₁** qui est un atome de soufre qui sera réduit/clivé par un agent réducteur, notamment par le dithiothréitol ou par la cystéine présent dans l'échantillon à analyser, ou un substrat réagissant par clivage d'une liaison labile en milieu acide lors de l'acidification de l'échantillon analysé et notamment un groupe formant une liaison acétate avec **X₁** qui est un atome d'oxygène, tels que des groupements **SE** correspondant à méthyloxyméthyle (-CH₂-O-CH₃), méthyloxyéthyle (-CH₂-O-CH₂CH₃), méthyloxyphényl (-CH₂-O-Ph), méthyloxyallyle (-CH₂-O-Allyl).

### Définition des groupes A

**A** est un groupement aromatique qui après clivage de la liaison C**X₂**-O permet la formation d'un chromophore ou fluorophore. Ce chromophore ou fluorophore peut correspondre directement au composé libéré **A**-OH ou à sa forme **A**-O⁻ qui se forme en solution aqueuse, ou à un composé résultant d'une réaction post-clivage du composé **A**-OH ou **A**-O⁻ libéré telle qu'une dimérisation ou à un composé résultant d'un réarrangement post clivage du composé **A**-OH ou **A**-O⁻, par exemple pour conduire à une forme tautomérique. Un tel composé sera nommé **A'**=O.

Par « groupe aromatique », on entend un groupe comprenant un ou plusieurs cycles aromatiques substitués ou non substitués, lesdits cycles pouvant comprendre un ou plusieurs hétéroatomes choisis parmi les atomes d'azote, d'oxygène ou de soufre et/ou un ou plusieurs atomes de carbone sous la forme d'un carbonyle C=O. **A** comprend, par exemple, de préférence de 4 à 40 atomes de carbone et de 0 à 10 hétéroatomes choisis parmi les atomes d'azote, d'oxygène ou de soufre. En particulier, un groupe aromatique **A** peut être un carbocycle monocydique, un carbocycle bicyclique ou un carbocycle polycyclique avec plus de deux cycles, et comportant de préférence de 5 à 40 chaînons, et préférentiellement de 6 à 15 chaînons, et comprenant au moins un cycle aromatique, ledit carbocycle comprenant éventuellement au moins un hétéroatome choisi parmi les atomes d'oxygène, azote ou soufre intégré au sein du carbocycle et/ou un ou plusieurs atomes de carbone formant le carbocycle sous la forme d'un carbonyle C=0, un tel carbocycle pouvant être non substitué ou porteur de un ou plusieurs substituants. Les groupes aromatiques comprennent notamment les groupes aryle et hétéroaryle, non substitués ou substitués. A titre d'exemple de tels carbocycles, sous leur forme non-substituée, on peut citer les groupes phényle, naphtyle, 2-, 3- ou 4-pyridinyle, 2- ou 3-furoyle, 2- ou 3-thiophényle, pyrrolyle, imidazolyle, pyrazolyle, thiazolyle, oxazolyle, isoxazolyle, pyridinyle, pyrazinyle, pyrimidinyle, tétrazolyle, thiadiazolyle, oxadiazolyle, triazolyle, pyridazinyle, indolyle, pyronyle.

Un chromophore est une molécule capable d'absorber une partie du spectre de la lumière visible. On peut citer, comme exemple de chromophore, le paranitrophénol et ses dérivés, les colorants du type Indigoïdes obtenus après dimérisation du dérivé énol correspondant (et correspondant donc à **A'**=O) qui forment un précipité en milieu aqueux aérobique, ou encore les composés tels que la cyclohexenoesculetine (CHE), l'alizarine ou encore l'hydroxyflavone qui forment un précipité coloré en présence de certains métaux :

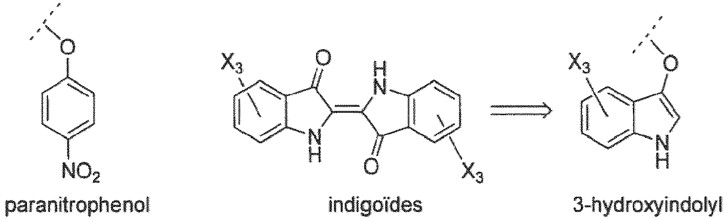

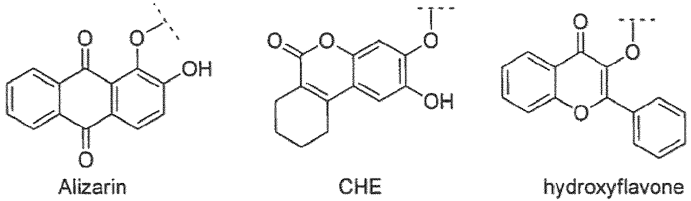

Les hydroxyindolyles peuvent être substitués par un ou plusieurs X₃ identiques ou différents choisis parmi les atomes de chlore, brome, iode ou fluor et positionné(s) sur n'importe quelle position du cycle benzénique.

Un fluorophore est une molécule capable de générer de la fluorescence, lorsqu'elle est soumise à une excitation lumineuse de longueur d'onde adaptée. La « fluorescence » est la propriété par laquelle une molécule qui est excitée par de la lumière d'une longueur d'onde donnée émet de la lumière à une plus grande longueur d'onde. La fluorescence est un phénomène qui résulte de l'Interaction d'un fluorophore avec un photon incident Ce processus est appelé excitation. L'absorption du photon amène un électron dans le fluorophore, à passer de son état fondamental, à un niveau d'énergie supérieur. Ensuite, l'électron revient à son niveau original en émettant un photon. Ce processus est appelé émission de fluorescence. Le fluorophore émet ensuite de la lumière à une plus grande longueur (fonde que celle du photon absorbé. Ceci est dû simplement au fait que l'énergie du photon émis est inférieure à celle du photon absorbé, du fait de la dissipation d'énergie pendant la durée de vie de l'état excité. Cette définition est donnée dans la demande de brevet WO 2004/058787.

Des exemples de groupements -O**A**, permettant de libérer de tels fluorophores sont donnés ci-dessous :

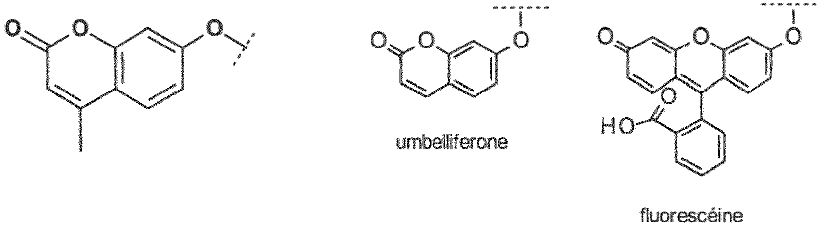

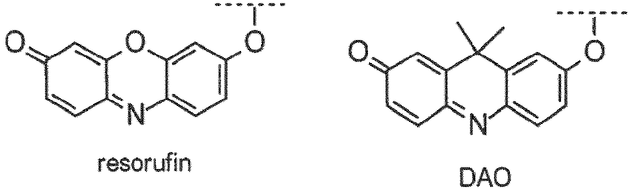

A titre d'exemple de groupe -O**A** permettant la libération d'un fluorophore après réarrangement de la molécule libérée **A**-OH ou **A**-O⁻, on peut citer les fluorophores suivants (le deuxième est décrit par : Shabat et al. J. Am. Chem. Soc 2012, 134, 20412) :

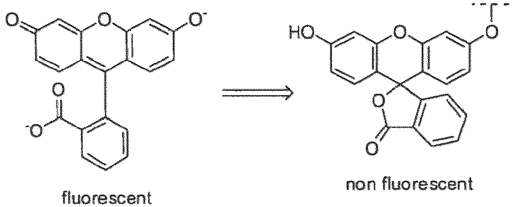

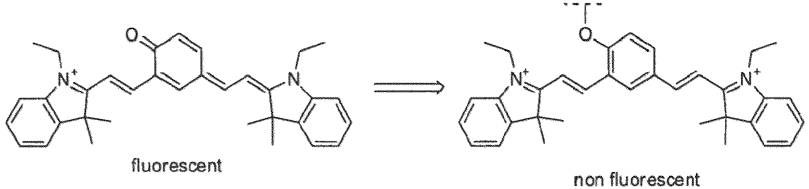

Selon des modes de réalisation particuliers, le groupe -O**A** est sélectionné de manière à obtenir, après clivage de la liaison C-0 entre l'atome de carbone porteur de l'atome (X₂) et l'atome d'oxygène porteur du groupement A dans les composés de formule (I), un composé **A**OH qui correspond à un fluorophore ESIPT, qui peut ou non précipiter en solution aqueuse. Un fluorophore ESIPT est un composé qui comprend au moins un groupement hydroxyle qui intervient dans le transfert de proton intra moléculaire, permettant une tautomérisation de la molécule fluorophore et l 'émission de fluorescence. Des exemples de tels groupements -O**A** répondent à la formule (**AA**): 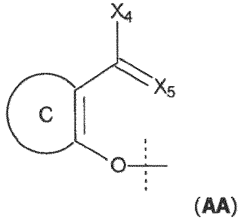 dans laquelle :
soit **X₅** est un atome d'oxygène et **X₄** est un groupe -NH₂, -OH, -SH, alkyle, aryle, -O-alkyle, -O-phényle, -NH-alkyle, -NH-phényle, -S-alkyle ou -S-aryle, lesdits groupes alkyle et phényle pouvant être substitués ou non substitués,
soit **X₅** représente un atome d'azote et est lié à **X₄** qui représente alors CH, O, S, N ou NH pour former un hétéroaryle substitué ou non substitué, ledit hétéroaryle substitué ou non substitué étant de préférence choisi parmi le quinazole, l'imidazole, le benzoimidazole, le thiazole, le benzothiazole, l'oxazole, le benzooxazole, la pyridine et la quinoline,
et 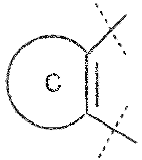 représente un aryle ou un hétéroaryle, substitué ou non substitué, par exemple choisi parmi les groupes phényle, naphthyle, et : 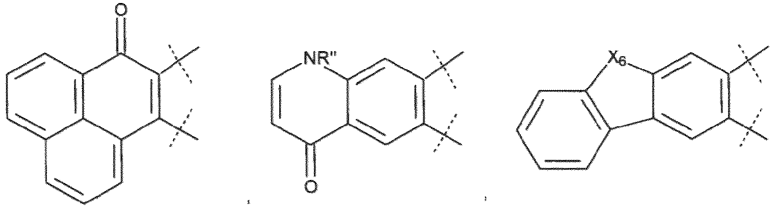
lesdits groupes pouvant être substitués ou non substitués,
   avec **X₆** qui représente S, O ou **NR",** et **R"** qui représente un atome d'hydrogène ou un groupe (C₁-C₄)alkyle.

Lorsque dans un groupe -OA, qui représente sous forme AOH un fluorophore ESIPT, comprend plusieurs groupements hydroxyle, alors l'atome d'oxygène du groupe -OA est l'atome d'oxygène du groupement hydroxyle qui intervient dans le transfert de proton intramoléculaire. L'incorporation de cet hydroxyle dans le groupement carbamate des composés de formule (I) empêche la formation de la liaison hydrogène interne et la tautomérisation du fluorophore.

Les fluorophores ESIPT montrent un déplacement de Stokes qui dépasse les 100 nm et atteint souvent 200 nm. Tous les fluorophores ESIPT perdent cette émission de fluorescence correspondant à un déplacement de Stokes supérieur à 100 nm, si leur groupement OH du type phénolique est alkylé, acylé ou autrement fonctionnalisé, ce qui empêche de transférer un atome d'hydrogène à l'hétéroatome **X₅** sur l'illustration donnée avec la formule (**AA**), lors de l'excitation par irradiation, et ainsi empêche l'émission de fluorescence caractéristique du processus de transfert de proton.

Le plus souvent dans un tel cas, le groupe **A** correspond à un groupe phényle qui est non substitué ou substitué et/ou qui est fusionné avec un ou plusieurs carbocycles insaturés comprenant éventuellement un hétéroatome tel que l'azote. Ce groupe sous sa forme phénoxy -O**A**, lorsqu'il n'est pas lié au substrat, correspond sous sa forme protonée à un composé phénolique HO-**A** qui appartient à la classe des fluorophores ESIPT.

Des groupes -O**A** du type phénoxy, correspondent, par exemple, aux structures préférées (BB) ou (CC) suivantes : 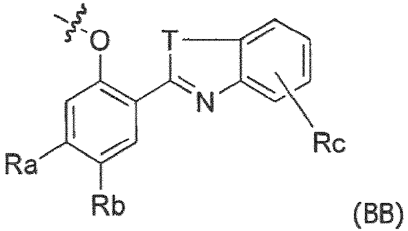 dans laquelle
- T est -NH-C(O)-, -S-, -O-, -NH, N-alkyle ou N-aryle,
- Ra est l'hydrogène ou un substituant carboné attracteur d'électrons, comme - CN ou -COORd, avec Rd qui représente un groupe (C₁-C₄)alkyle, ou bien Ra est - CONReRf, avec Re et Rf, identiques ou différents, qui représentent l'hydrogène ou un groupe (C₁-C₄)alkyle, ou bien Ra est -CF₃ ou un groupe 2-oxazolyle, 2-thiazolyle, 2-imidazolyle, 2-benzoimidazolyle, 4-pyrimidinon -2-yle ou quinazolinon-2-yle,
- Rb est l'hydrogène, un atome de chlore, brome, iode ou fluor, -OH, -NH₂, - NRgRh, -NHRg ou -ORg, avec Rg et Rh qui représentent chacun indépendamment un (C₁-C₄)alkyle,
- ou bien Ra et Rb sont liés entre eux pour former une chaîne hydrocarbonée comprenant 4 ou 5 chaînons, saturée ou insaturée, substituée ou non substituée, éventuellement interrompue par un ou plusieurs hétéroatomes choisis parmi N, S et O,
- Rc est l'hydrogène, Br, Cl, I ou F, 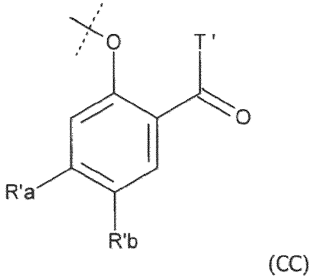 dans laquelle :
   - T ' est NH₂, OH, un groupe aryle, un groupe (C₁-C₄)alkyle, SH, NHR'c, OR'c, NR'cR'd ou SR'c, avec R'c et R'd, identiques ou différents, qui représentent un groupe (C₁-C₄)alkyle ou aryle,
   - R'a est l'hydrogène ou un substituant carboné attracteur d'électrons comme - CN, ou -COOR'e, avec R'e qui représente un groupe (C₁-C₄)alkyle, ou R'a est - CONR'fR'g, avec R'f et R'g, identiques ou différents, qui représentent l'hydrogène ou un groupe (C₁-C₄)alkyle, ou bien R'a est -CF₃ ou un 2-oxazolyle, 2-thiazolyle, 2-Imidazolyle, 2-benzoimidazolyle, 4-pyrimidinon-2-yle ou quinazolinon-2-yle,
   - R'b est l'hydrogène, un atome de chlore, brome, iode ou fluor, -OH, -NH₂ - NR'hR'i ou -OR'h, avec R'h et R'i, identiques ou différents, qui représentent un groupe (C₁-C₄)alkyle,
   - ou bien R'a et R'b sont liés entre eux pour former une chaîne hydrocarbonée comprenant 4 ou 5 chaînons, saturée ou insaturée, substituée ou non substituée, éventuellement interrompue par un ou plusieurs hétéroatomes choisis parmi N, S et O.

On pourra notamment se référer aux demandes WO 2013/045854 et WO 2014/020285A qui donnent des exemples de tels fluorophores ESIPT pour plus de détails.

A titre d'exemple de groupe O**A**, correspondant à un fluorophore de type ESIPT, on peut citer, plus particulièrement les groupements suivants :

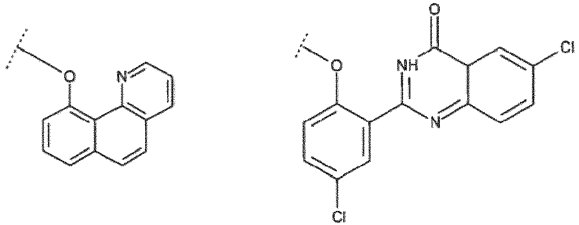

Le très grand déplacement de Stokes de tels fluorophores (approximativement 170 nm pour la HPQ (Hydroxyphénylquinazolinone) présentée à droite ou de tout analogue de la HPQ contribuera à l'excellente sensibilité de la sonde et rendra le fluorophore libéré aisément distinguable de la fluorescence native pouvant provenir de l'échantillon biologique sur lequel l'analyse va être menée.

### Définition des groupes R

**R** peut représenter un atome d'hydrogène ou -(**L**)**n**-GP, avec **n** qui est égal à 0 ou 1.

Bien souvent, pour des raisons de synthèse, **n** = 1 et **L** est un bras de liaison, et notamment un bras **-(L1)ml-(L2)m2-(L'1)m'1-** (dans le sens pipérazine -> groupe **GP**) avec :
**L1** et **L'1,** identiques ou différents, qui sont choisis parmi -O-, -NH-,
-N(C₁₋₆) alkyl- -N(phényl)-, -N(aryl)-, -C(O)-, -C(O)O-, -OC(O)-, -OC(O)-O-, - NHC(O)-O-, -OC(O)-NH-, -NHC(O)-NH-, -S-, -SO₂-, -N=N-, -NHC(O)- et -CONH- ;
**L2** qui est choisi parmi les groupes bivalents suivants : (C₁₋₂₀)alkyle, (C₁₋₂₀)alcényle, (C₁₋₂₀)alcynyle, (C₆₋₂₄)aryle, (C₇₋₄₄)alkylaryle, (C₇₋₄₄)alcénylaryle, (C₇₋₄₄)alcynylaryle, (C₇₋₄₄)alkylcycloalkyle, (C₇₋₄₄)alcénylcycloalkyle, (C₇₋₄₄)alcynylcycloalkyle, (C₇₋₄₄)alkylhétérocycloalkyle, (C₇₋₄₄)alcénylhétérocycloalkyle,
(C₇₋₄₄)alcynylhétérocycloalkyle; lesdits groupes pouvant être interrompus ou se terminer par un groupe triazole et pouvant être non substitués ou substitués, notamment par un ou plusieurs substituants choisis parmi les (C₁₋₁₀)alcoxy, (C₁₋₁₀)alkyle, (C₆₋₁₀)aryle, amido, imido, phosphido, nitrido, (C₁₋₁₀)alcényle, (C₁₋₁₀)alcynyle et -OH ; et
**m1, m'1** et **m2,** identiques ou différents, qui sont égaux à 0 ou 1.

Le bras **L**, lorsqu'il est présent, sera choisi pour éloigner le groupe **GP** de la pipérazine ou pour des questions de synthèse. Selon un mode de réalisation préféré, **L** représente **-(L1)ml-(L2)m2-(L'1)m'1** avec **L1** = -C(O)-, **m1 = m2** = 1, **m'1** = 1 ou 0 et **L2** et **L'1** tels que définis précédemment et notamment L représente -C(O)-(CH₂)**p-L3-** avec p qui est égal à 1, 2, 3 ou 4 et L3 qui est un groupe triazole et notamment un groupe 1H-1,2,3-triazole.

**GP** est un groupe hydrosolubilisant.

Par « groupe hydrosolubilisant », on entend un groupe hydrophile qui permet d'améliorer la solubilité de la sonde, par rapport notamment à une sonde qui n'en diffère que par le remplacement de l'atome d'azote porteur de L-GP par un CH₂, pour obtenir une pipéridine telle que décrite dans les demandes WO 2013/045854 et WO 2014/020285. A titre d'exemple de groupe hydrosolubilisant, on peut citer les groupes capables de former une espèce chargée en solution aqueuse. A titre d'exemple de groupe hydrosolubilisant **GP,** on peut citer les fonctions **F₁** choisies parmi les aminé (primaire, secondaire, ou tertiaire), amidine, guanidine ou tétrazole ; les fonctions **F₂** choisies parmi les fonctions anioniques du type carboxylate, sulfonate ou phosphate ; les groupements comprenant une ou plusieurs de ces fonctions **F₁** et/ou **F₂** ; les polyéthylèneglycols ; les sucres ou polysaccharides tels que le glucose, le galactose et le mannose ; les groupes peptidiques tels que la poly-lysine, la poly-arginine, les TAT-peptides... A titre d'exemple de fonctions aminé, on peut citer -MH₂, -NH (C₁-C₄)alkyle, et les dialkylamines dans lesquelles les groupes alkyle sont identiques ou différents et comprennent de 1 à 4 atomes de carbone. Par « groupements comprenant une ou plusieurs de ces fonctions **F₁** et/ou **F₂** », on entend un groupement alkyle (tel que les (C₃₋₆)alkyles), un groupement aryle (tel qu'un groupement aryle comportant 6 atomes de carbone), un groupement hétéroaryle (tel qu'un hétéroaryle comportant 6 atomes de carbone) dans lequel un ou plusieurs atomes d'hydrogène de ces groupement a(ont) été substitué(s) par une ou plusieurs fonctions **F₁,** ou par une ou plusieurs fonctions **F₂** ou bien au moins un atome d'hydrogène a été substitué par une fonction **F₁** et un autre atome d'hydrogène a été substitué par une fonction **F₂.**

### Définition des termes utilisés dans la définition des sondes

Par fonction acyle, on entend une fonction :

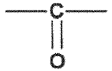

Par groupe « alkyle », à moins qui) n'en soit spécifié autrement, on entend une chaîne hydrocarbonée, saturée, linéaire ou ramifiée. Les groupes alkyle comprenant de 1 à 6 atomes de carbone seront préférés. A titre d'exemple de groupe alkyle comprenant de 1 à 6 atomes de carbone, nommés C₁₋₆ alkyle ou (C₁-C₆) alkyle, on peut citer, notamment, les groupes méthyle, éthyle, n-propyle, iso-propyle, n-butyle, tert-butyle, sec-butyle, n-pentyle, n-hexyle.

Par groupe « aryle », on entend un carbocycle mono-, bi- ou polycyclique, comprenant au moins un groupe aromatique, par exemple, un groupe phényle, cinnaamyle ou naphtyle. Les groupes aryle comprenant de 6 à 12 atomes de carbone seront préférés. Le phényle est le groupe aryle particulièrement préféré.

Lorsqu'il est indiqué qu'un groupe est substitué, cela signifie qu'il est substitué par un ou plusieurs substituants, notamment choisis parmi les atomes de chlore, brome, iode ou fluor, les groupes cyano, alkyle, fluoroalkyle, trifluorométhyle, alcényle, alcynyle, cycloalkyle, aryle, hétérocycloalkyle, amino, alkylamino, dialkylamino, hydroxy, akoxy, aryloxy, -COOH, nitro, les groupes acyle, les groupes aromatiques (notamment aryle et hétéroaryle), lesdits groupes alcényle, alcynyle, cycloalkyle, hétérocycloalkyle, groupes acyle et groupes aromatiques peuvent eux-mêmes être non substitués ou substitués. Les termes utilisés pour (a définition de ces substituants sont ceux usuellement reconnus par l'homme du métier.

De manière classique, le terme « alcényle » désigne une chaîne hydrocarbonée, linéaire ou ramifiée comprenant au moins une double liaison, et présentant de préférence de 2 à 20 atomes de carbone, et préférentiellement de 2 à 6 atomes de carbone ; le terme « alcynyle » désigne une chaîne hydrocarbonée, linéaire ou ramifiée comprenant au moins une triple liaison, et présentant de préférence de 2 à 12 atomes de carbone, et préférentiellement de 2 à 6 atomes de carbone ; le terme « fluoroalkyle » désigne une chaîne hydrocarbonée, saturée, linéaire ou ramifiée dans laquelle au moins un atome d'hydrogène a été remplacé par un atome de fluor, et présentant de préférence de 2 à 12 atomes de carbone, et préférentiellement de 2 à 6 atomes de carbone ; les termes « alcoxy » et « aryloxy » désignent respectivement un O-alkyle et un O-aryle.

Par « cycloalkyle », on entend un groupe hydrocarboné saturé constitué d'au moins un cycle, éventuellement ponté. Les groupes alkyle comprenant de 3 à 12 atomes de carbone seront préférés. A titre d'exemple, on peut citer les groupes cyclopropyle, cyclobutyle, cyclopentyle, cyclohexyle, cycloheptyle et adamantyle.

Par « hétérocycloalkyle », on entend un groupe cycloalkyle tel que défini ci-dessus, dans lequel au moins un atome de carbone est remplacé par un hétéroatome choisi parmi 0, S ou N. A titre d'exemple d'hétérocycloalkyle, on peut citer les groupes pipéridinyle, pipérazinyle, morpholinyle, tétérahydropyranyle.

Par groupe « hétéroaryle », on entend un carbocycle mono-, bi- ou polycydique, comportant de préférence de 6 à 12 chaînons, et comprenant au moins un groupe aromatique et au moins un hétéroatome choisi parmi les atomes d'oxygène, azote ou soufre intégré au sein du carbocycle. A titre d'exemple de groupe hétéroaryle, on peut citer les 2-, 3- ou 4-pyridinyle, 2- ou 3-furoyle, 2- ou 3-thiophényle, pyrrolyle, imidazolyle, pyrazolyle, thiazolyle, oxazolyle, isoxazolyle, pyridinyle, pyrazinyle, pyrimidinyle, tétrazolyle, thiadiazolyle, oxadiazolyle, triazolyle, pyridazinyle, indolyle. Les hétéroaryles comprennent également de tels groupes dans lesquels un ou plusieurs atome(s) de carbone du cycle est(sont) sous la forme d'une fonction carbonyle C=O.

Par « groupe acyle », on entend un groupe lié par le carbone d'une fonction acyle C=O, et notamment un groupe -CONH₂, -COOH, -COaryle, -CO(C₁₋₄)alkyle, - COSH, -CONHR"_{a,} -CONR "ₐR"_{b} ou -COSR"ₐ avec R"ₐ et R"_{b,} identiques ou différents, qui représentent un groupe (C₁-C₄) alkyle ou aryle.

Par « (C₇₋₄₄)alkylaryle », « (C₇₋₄₄)alcénylaryle », « (C₇₋₄₄)alcynylaryle », on entend respectivement une chaîne alkyle, alcényle ou alcynyle, commençant, interrompue ou se terminant par un groupe aryle. On préférera les groupes alkylaryle, alcénylaryle et alcynylaryle comprenant de 7 à 22 et de préférence de 7 à 16 atomes de carbone.

### Préparation des sondes selon l'invention

Les composés utilisés dans le cadre de l'invention sont préparés selon des techniques classiques. Ils peuvent, notamment être obtenus selon des procédés analogues à ceux utilisés dans les exemples.

Les composés selon l'invention peuvent être obtenus selon le **Schéma 2** ci-après, dans lequel **A, X₁, X₂** et **SE** sont tels que définis pour les sondes de formule (I) et **P₁** et **P₂** sont des groupes protecteurs temporaires des fonctions aminé en particulier dans les conditions de réaction mises en oeuvre, **Y** est un groupe partant du type Cl, para-nitrophénol, imidazole ou N-méthylimidazolium, ou N-hydroxysuccinimide, et **Rp** représente **R** ou un groupe protecteur temporaire des fonctions aminé en particulier dans tes conditions de réaction mise en oeuvre ou **Rp** est un groupe précurseur du groupe **R.**

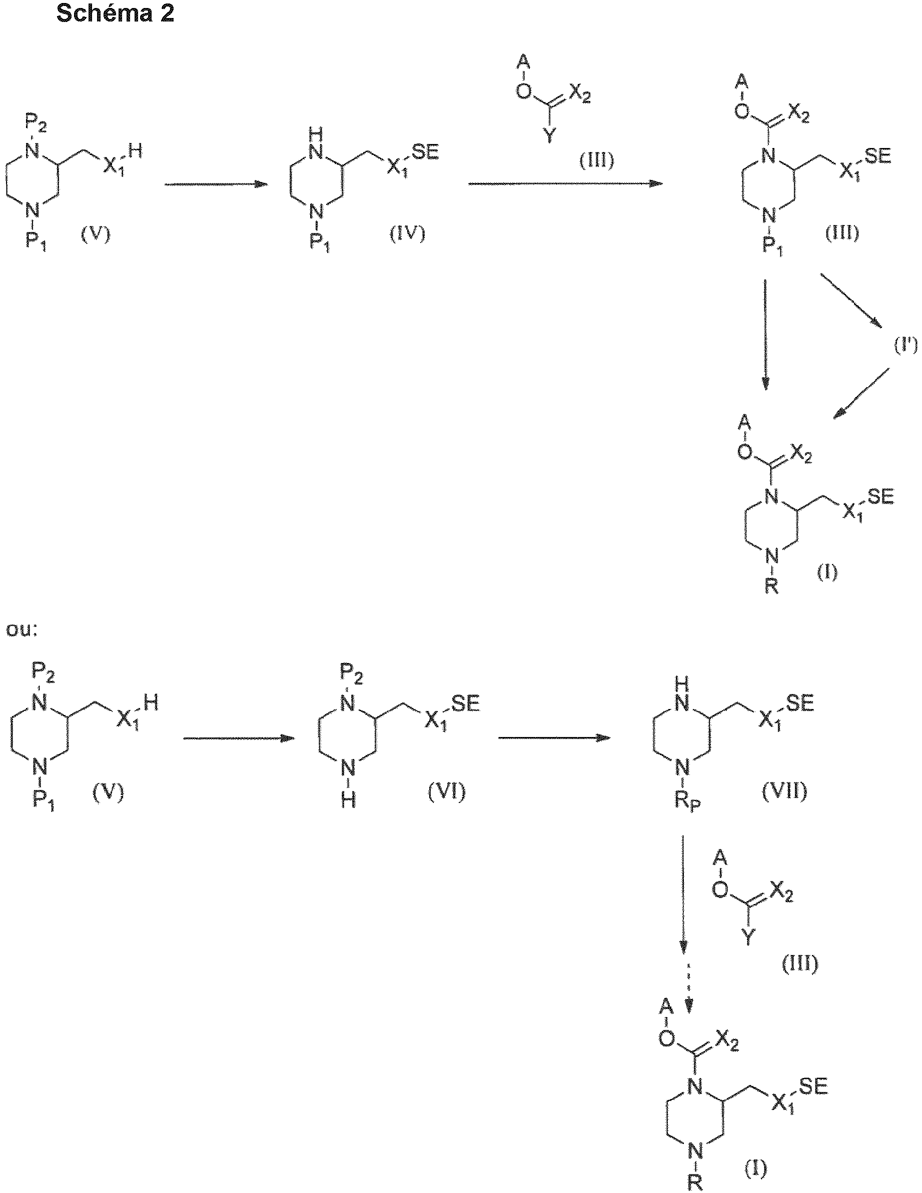

Par groupe protecteur temporaire des aminés, on entend les groupes protecteurs tels que ceux décrits dans Protective Groups in Organic Synthesis, Greene T.W. et Wuts P.G.M., ed John Wiley et Sons, 2006 et dans Protecting Groups, Kocienski PJ., 1994, Georg Thieme Verlag. A titre d'exemple de tels groupements, on peut citer les groupes benzyle (Bn), les groupes -C(O)O**R'₁** avec **R'₁** qui représente un groupe alkyle ou alcényle de 1 à 12 atomes de carbone ou un groupe - (CH₂)ₘ₃**R"₁** avec **R"₁** qui
représente un groupe aryle, cycloalkyle ou fluorényle et m3 qui est égal à 0, 1, 2 ou 3, et en particulier, les groupes carbobenzyloxy (Cbz), ter-butyloxycarbonyl (Boc) et 9-fluorénylméthyloxycarbonyl (Fmoc), allyloxycarbonyle (Alloc).

Les composés (V) pourront notamment être obtenus à partir de la pipérazine commerciale. En suivant une procédure similaire, mais modifiée de celle décrite dans Org. Lett 2010, 12, 4176, on peut former le composé (V) à partir de la pipérazine bis-protégée, dont l'un des groupements est un groupement Butyloxycarbonyl (Boc), grâce à une réaction d'ortholithiation, suivie d'une réaction avec du paraformaldéhyde pour former le composé (V) où **X₁** = 0. Le dérivé aminé (V) où **X₁** = NH peut être ensuite obtenu par exemple grâce à une synthèse de Gabrielle, tandis que le composé (V) où **X₁** = S peut être obtenu par substitution nucléophile.

Il est également possible, de manière préférée, d'utiliser les procédés illustrés dans les **Schémas 3** et **4** qui nécessitent moins d'étapes dans les cas où **X₁** = O ou NH pour la préparation des composés de formule (VII). Dans les **Schémas 3** et **4, A, X₁, X₂** et **SE** sont tels que définis pour les sondes de formule (I) et **R₂** est un groupe alkyle de 1 à 4 atomes de carbone, **R₃** est groupe protecteur temporaire des fonctions aminé en particulier dans les conditions de réactions mise en oeuvre, et **Y** est un groupe partant du type Cl ou paranitrophénol, imidazole ou N-méthylimidazolium, ou N-hydroxysuccinimide et **Rp** représente R ou un groupe protecteur temporaire des fonctions aminé en particulier dans les conditions de réaction mises en oeuvre ou **Rp** est un groupe précurseur du groupe **R.**

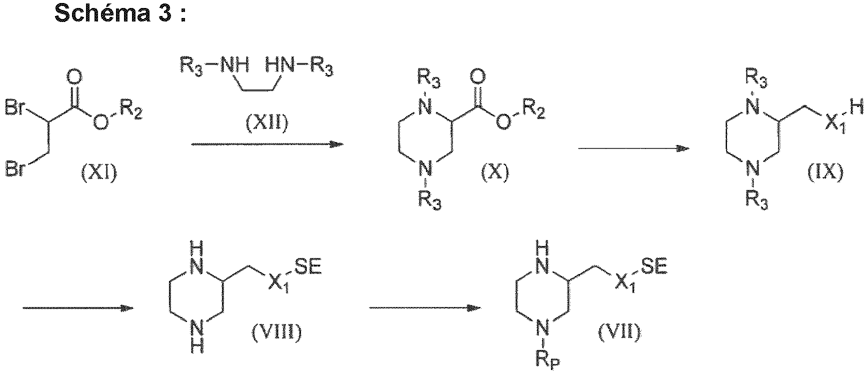

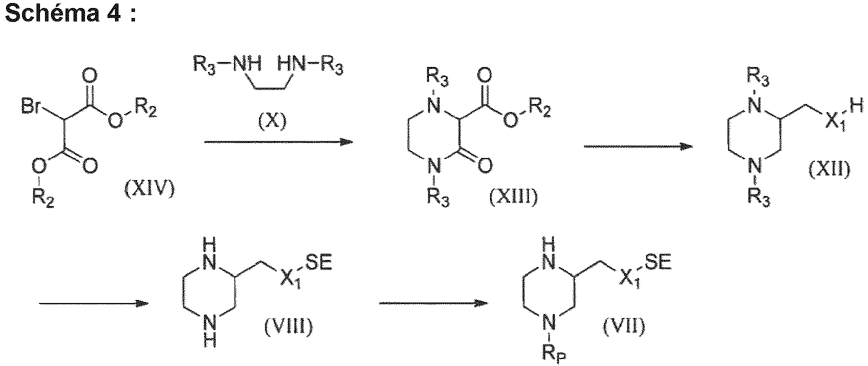

Dans la procédure décrite dans le **Schéma 3,** le cycle pipérazine est obtenu par une double substitution nucléophile d'un composé du type éthylène diamine (X) sur des dérivés du produit de départ commercial du type (XI).

Dans la procédure décrite dans le **Schéma 4,** le cycle pipérazine est obtenu par une réduction du cycle pipérazinone (XIII), lui-même synthétisé à partir d'un bromure de malonate (XIV) et d'un composé du type éthylène diamine (X).

Les groupements **R** différents de H, qui correspondent à un groupe **L- GP,** peuvent être introduits grâce à des réactions du type substitution nucléophile, amination réductrice ou formation de liaison amide. La liaison entre les groupements **L** et **GP** peut être obtenue par d'innombrables voies de synthèse selon les fonctions chimiques présentes sur le bras de liaison **L.** On peut par exemple citer des réactions de ligation telles que des substitutions nucléophiles, des couplages peptidiques, des cycloadditions telles que la cycloaddition de Huisgen ou de Diels-Alder, des couplages paladocatalysés tels que le couplage de Sonogashira ou de Heck.

Il est possible de former intermédiairement un composé intermédiaire de formule (l') dans laquelle **Rp** est un groupe précurseur d'un groupe **R** ou **Rp** est un groupe protecteur temporaire des aminés. En particulier, font partie intégrante de l'invention, les intermédiaires de formule : 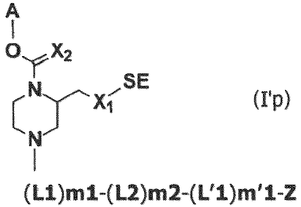 et 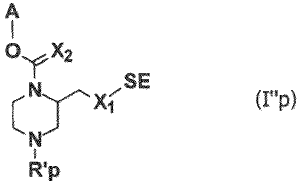 ainsi que leurs sels, solvats ou hydrates,
dans lesquels :
**A, X₁, X₂, SE, L1, L'1, L2, m1, m'1,** et **m2** sont tels que définis pour (I),
**Z** représente C=CH, N₃, une fonction N-oxysucdnimide ou maléimide, et
**R'p** représente un groupe protecteur des fonctions aminés, de préférence choisi parmi les groupes benzyle, les groupes -C(O)O**R'₁** avec **R'₁** qui représente un groupe alkyle ou alcényle de 1 à 12 atomes de carbone ou un groupe -(CH₂)ₘ₃**R"₁** avec **R"₁** qui représente un groupe aryle, cycloalkyle ou fluorényle et m3 qui est égal à 0, 1, 2 ou 3. A titre d'exemple de groupe **R'p** préféré, on peut citer le carbobenzyloxy, ter-butyloxycarbonyl, 9- fluorénylméthyloxycarbonyl, et allyloxycarbonyle.

Dans certains cas, également, un composé de formule (I) pourra servir d'intermédiaire dans la préparation d'un autre composé de formule (I). C'est notamment le cas des composé de formule (I) dans lesquels **GP** = -COOH ou -NH₂ qui pourront être utilisés notamment pour former des composés dans lesquels **L'1** = - CONH- ou -NHCO- et **m'1** = 1.

Les réactifs de départ sont disponibles dans le commerce ou facilement accessibles. Les molécules de formule (I) sont donc d'accès chimique relativement simples, et peuvent être obtenues à un relativement faible coût de préparation, pour le domaine technique considéré.

Les sels des composés selon l'invention sont préparés selon des techniques bien connues de l'homme de l'art. Les sels des composés de formule (I) selon la présente invention comprennent ceux avec des acides ou des bases, en fonction des substituants présents. Ces acides ou bases peuvent être choisis parmi les acides et bases minéraux et organiques qui permettent une séparation ou une cristallisation convenable des composés de formule (I), ainsi que des sels physiologiquement acceptables, c'est-à-dire compatibles avec des applications *in vivo* et *in vitro.* En tant qu'acide approprié, on peut citer : l'acide oxalique ou un acide optiquement actif, par exemple un acide tartrique, un acide dibenzoyltartrique, un acide mandélique ou un acide camphorsulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate, le mésylate, le bésylate, l'isotionate. En tant que base appropriée, on peut citer : la lysine, l'arginine, la méglumine, la bénéthamine, la benzathine et celles qui forment des sels physiologiquement acceptables, tels que des sels de sodium, de potassium, de calcium.

Comme composés sous forme hydratée, on peut citer, à titre d'exemple, les semi-hydrates, monohydrates et polyhydrates.

Par solvat, on entend une forme du composé associé à une ou plusieurs molécules de solvant, notamment utilisé lors de sa synthèse ou lors de sa purification, sans pour autant être en solution dans ce dernier.

Les différents composés de formule (I) selon l'invention peuvent se trouver sous toutes les formes d'isomères optiques possibles, éventuellement en mélange selon toutes proportions, à moins qu'il n'en soit spécifié autrement. Selon un mode de réalisation particulier, les composés selon l'invention comportant un carbone asymétrique se trouvent sous une forme racémique, les formes R et S se trouvant en proportions sensiblement égales. Selon un autre mode de réalisation, les composés de formule (I) de l'invention peuvent se trouver sous une forme enrichie en un diastéréoisomère ou énantiomère, avec un excès diastéréoisomérique ou énantiomérique supérieur à 80 %, voire même supérieure à 95%, voire sous une forme isomérique pure c'est-à-dire avec un excès diastéréoisomérique ou énantiomérique supérieur à 99 %.

Les composés (I) pourront être isolés sous une forme enrichie en un diastéréoisomère ou énantiomère par les techniques classiques de séparation : on pourra utiliser, par exemple des recristallisations fractionnées d'un sel du racémique avec un acide ou une base optiquement active dont le principe est bien connu ou, le plus souvent, les techniques classiques de chromatographies sur phase chirale ou non chirale.

Dans le cadre de (Invention, toutes les définitions et modes de réalisation préférés pour **A, R, X₁, X₂** et **SE** pourront être combinées.

En particulier, les sondes selon l'invention présenteront l'une ou l'autre des caractéristiques suivantes, voire toutes les caractéristiques suivantes :
- **X₂** = O,
- soit **X₁** = O et **SE** est un substrat de glycosidase ou glucuronidase et correspond à un groupe glycosyle lié par son carbone anomérique au reste de la molécule, ou **SE** est un substrat d'estérase et correspond à un groupe -C(O)Ri avec Ri qui représente, par exemple, un groupe alkyle de préférence de 1 à 20 atomes de carbone, alcényle de préférence de 1 à 20 atomes de carbone, un groupe benzyle, aryle ou hétéroaryle, soit **X₁** = NH et **SE** est un substrat de protéase ou peptidase et correspond à un groupe peptidyle lié par une fonction acyle portée par son carbone terminal ou par un chaînon latéral, **R** représente **-L-GP** avec :
   ∘ **L** qui représente, de préférence, **-(L1)m1-(L2)m2-(L'1)m'1** avec **L1** = - C(O)- **m1** = **m2** = 1, **m'1** = 1 ou 0 et **L2** et **L'1** tels que définis ci-dessus, et notamment **L** représente -C(0)- (CH₂)**p-L3-** avec p qui est égal à 1, 2, 3 ou 4 et **L3** qui est un groupe triazole et notamment un groupe 1H-1,2,3-triazole, et
   ∘ **GP** qui est, de préférence, une fonction F₁ choisie parmi les fonctions ammonium, carboxylate, sulfonate et phosphate et les polyéthylèneglycols,
- **A** est un des groupements spécifiquement décrits dans la présente demande de brevet.

Les sondes selon l'invention sont attractives pour plusieurs applications à haute sensibilité dans les sciences de la vie, et notamment : (1) le criblage à haut rendement d'une activité, en particulier d'une activité enzymatique, exprimée par des colonies bactériennes sur une plaque gélosée (analyse de colonies) ; (2) la détection In vitro d'une activité, en particulier d'une activité enzymatique, dans des liquides biologiques (hématologie et autres) ; (3) la détection d'une activité, en particulier d'une activité enzymatique, au niveau d'une cellule unique par cytométrie de flux; (4) la visualisation d'une activité, en particulier d'une activité enzymatique, intracellulaires dans des cellules cultivées (microscopie de fluorescence et microscopie confocale) ; (5) la détection histochimique d'une enzyme (à l'échelle tissulaire) ; et également (6) l'imagerie moléculaire *in vivo.*

Ainsi, les sondes selon la présente invention ont un grand nombre d'applications potentielles. Les exemples de telles applications incluent la conception d'analyses sur colonies bactériennes. Celles-ci sont actuellement réalisées sur une plaque gélosée (boîte de Pétri) où jusqu'à 3 000 colonies peuvent être distinguées sans avoir à les séparer activement dans des compartiments séparés comme les puits contenus dans une plaque à puits multiples. Il est, par exemple, possible de concevoir des tests sur échantillons cliniques permettant d'identifier parmi un ensemble de lignées bactériennes une lignée pathogène d'intérêt.

Les sondes selon l'invention peuvent servir aussi pour l'imagerie par fluorescence macroscopique, c'est-à-dire au niveau d'un organisme entier. Dans ce cas, la sonde pénétrera dans la paroi cellulaire pour atteindre l'activité d'intérêt (un stimulus tel qu'une enzyme, un composé chimique ou une caractéristique physicochimique du milieu dans lequel se trouve la sonde).

Les exemples suivants illustrent l'invention, mais n'ont aucun caractère limitatif.

### Abréviations utilisées

| | | | |
|---|---|---|---|
| DCM : | dichlorométhane | TFA : | acide trifluoroacétique |
| Boc : | tert-butytoxycarboxyl | Me : | méthyl |
| Ac : | acétyl | HOBt : | 1-hydroxybenzotriazole |
| sBu : | sec-butyl | HATU : | hexafluorophosphate de 1-[Bis(diméthylamino)methylène]-1H-1 ,2,3-Wazolo[4,5-b]pyridinium 3-oxyde |
| Et : | éthyl | TA : | Température Ambiante |
| DIAD | azodicarboxylate de diisopropyle | AMC : | 7-amino -4-rnéthylcoumarine |
| THF : | Tétrahydrofurane | DCC : | Dicyclohexylcarbodiimide |
| DM F : | N,N-diméthylformamide | iPr : | isopropyl |
| δ : | Déplacement Chimique (ppm) | RFU : | Unité de fluorescence arbitraire |
| A : | Unité d'absorbance arbitraire | U λₑₓ : | Longueur d'onde d'excitation (nm) |
| λₑₘ : | Longueur d'onde d'émission (nm) | RMN : | Résonance magnétique nucléaire |
| LC : | Chromatographie liquide | MS : | Spectroscopie de masse |
| ESI : | Ionisation par électrospray | Rf : | Rapport frontal |
| J: | Constante de couplage H-H (Hz) | s : | singulet |
| d : | doublet | t : | triplet |
| dd : | doublet de doublet | td : | triplet de doublet |
| m : | multiplet | br : | signal élargi |
| v : | volume | | |

### Appareillage utilisé pour les caractérisations

Les spectres RMN ont été obtenus à 297K grâce à un spectromètre Bruker AVANCE 300 (300 MHz & 75 MHz pour ¹H et ¹³C, respectivement) ou un Bruker AVANCE 500 (500 MHz & 125 MHz pour ^{I}H et ¹³C, respectivement).

Les mesures de Masse en basse résolution ont été obtenues grâce à un spectromètre de masse couplé à la chromatographie liquide AGILENT 1100 SL.

Les analyses par Chromatographie sur Couche Mince (CCM) ont été réalisées sur des plaques de gel de Silice 60Â déposé sur aluminium (Aldrich).

Les mesures de Masse en haute résolution ont été obtenues grâce à un spectromètre de masse MicrOTOFQ II, Bruker.

Les mesures de fluorescence ou d'absorbance ont été obtenues grâce à un fluorimètre à plaque EnSpire, Perkin-Elmer.

### A - Synthèses :

### I - Préparation de l'intermédiaire 8, le N-((4-benzylpiperazin-2-yl)méthyl)-2-phénylacétamide, sous la forme de sel avec l'acide trifluoacétique

### a) Préparation du composé 2: N-Boc-pipérazine

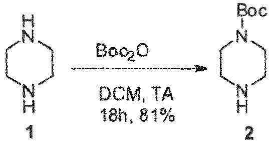

A une solution de pipérazine **1** (10,0g, 100mmol, 2,0eq.) dans du DCM (300mL) est ajoutée goutte à goutte une solution de Boc₂O (8,07g, 50mmol, I,0eq.) dans du DCM (150mL) sur une période de 3h à température ambiante (TA). A la fin de l'addition, le mélange réactionnel est agité à TA pendant 18h. Le mélange est ensuite concentré par évaporation, afin d'obtenir un volume final approximatif de 100mL Cette solution est ensuite lavée avec une solution aqueuse saturée de NaHCOs (2x100mL) et une fois avec de la saumure (100mL). La phase organique est séchée avec du Na₂S0₄, filtrée et évaporée pour obtenir le composé **2** sous forme d'une huile incolore qui cristallise avec le temps (7,57g, 40mmol, rendement: 81%).

¹H-RMN (300 MHz, CDCl₃): δ = 3.33 (t, J=5Hz, 4H), 2.75 (t, J=5Hz, 4H), 1.67 (s, 1H), 1.41 (s, 9H) ppm.

¹³C-RMN (75 MHz, CDCl₃): δ = 154.9, 79.5, 56.3, 46.0, 44.8, 30.4, 28.5 ppm. MS: ESI: [M+H]⁺ m/z trouvée 187.3, calculée 187.3

### b) Préparation du composé 3: N-Boc-N'-benzyl-pipérazine

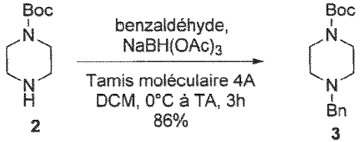

A une solution du composé **2** (10,0g, 5,7mmol, 1,0eq.), de benzaldéhyde (5,5mL, 53,7mmol, 1,0eq.) et de tamis moléculaire 4Â dans du DCM (200mL ) refroidie à 0°C est ajouté en plusieurs portions du NaBH(OAc)₃ (17,6g, 80,6mmol, 1 ,5eq.). Le milieu réaction est agité à 0°C pendant 1h puis à TA pendant 2h. A la fin de la réaction, le mélange est filtré et le filtrat est lavé trois fois avec une solution aqueuse saturée de NaHCOs (3x150mL). La phase organique est séchée avec du Na₂SO₄, filtrée et évaporée. Le brut réactionnel est purifié par colonne de chromatographie sur gel de silice (éluant : d'abord pur DCM puis DCM : MeOH / 95:5 / v:v) pour obtenir le composé **3** sous forme d'une huile incolore qui cristallise avec le temps (12,8g, 46mmol, rendement: 86%).

¹H-RMN (300 MHz, CDCl₃): δ = 7.29 (m, 5H ), 3.52 (s, 2H), 3.45 (t, J=5Hz, 4H), 2.40 (t, J=5Hz), 1.48 (s, 9H) ppm.

¹³C NMR (75 MHz, CDCl₃) δ = 154.90, 137.98, 129.25, 128.38, 127.26, 79.63, 63.18, 52.98, 43.61, 28.54 ppm.
MS: ESI: [M+H]⁺ m/z trouvée 277.3, calculée 277.3
Rf = 0.32 (éther de pétrole : acétate d'éthyle / 8:2 / v:v)

### c) Préparation du composé 4: tert-butyl 4-benzyl-2-(hydroxyméthyl)pipérazine-1 -carboxylate

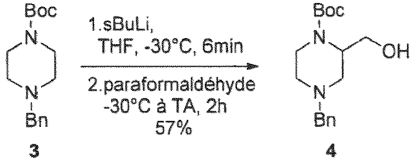

Dans un ballon préalablement séché et placé sous atmosphère Inerte d'Argon est dissout le composé **3** (1,0g, 3,6mmol, 1,0eq.) dans du THF anhydre (20mL). Cette solution est refroidie à -30°C et le sBuLi (1,3M dans du Cyclohexane, 4,2mL, 1,5eq.) est ajouté goutte à goutte. Le mélange réactionnel est agité à -30°C pendant 6 minutes avant que le paraformaldéhyde (350mg, 11,6mmol, 3,2eq.) ne soit introduit rapidement en une seule portion. Cette suspension est agitée à -30°C pendant 30minutes, et 1h30 à TA avant d'être quenchée grâce à une solution aqueuse saturée de NH₄Cl (20mL). La phase organique est séparée et la phase aqueuse est lavée deux fois avec Et₂O (2x20mL). Les phases organiques sont combinées, séchées avec du Na₂S0₄, filtrées et évaporées. Le brut réactionnel est purifié par colonne de chromatographie sur gel de silice (éluant : éther de pétrole : acétate d'éthyle / 7:3 / v:v) pour obtenir le composé **4** sous forme d'une huile jaune (625mg, 2,0mmol, rendement: 57%).

¹H-RMN (500 MHz, CDCl₃): δ= 7.32 (m, 5H), 4.12 (br s, 1H), 3.92 (m, 3H), 3.54 (s, 2H), 3.40 (br s, 1H), 3.04 (d, J=11.6Hz, 1H), 2.86 (d, J=8.7Hz, 1H), 2.33 (dd, J=U.6Hz, 3.9Hz, 1H), 2.13 (td, J=11.6Hz, 3.9Hz, 1H), 1.49 (s, 9H) ppm.

¹³C-RMN (125 MHz, CDCl₃): δ = 155.3, 137.3, 129.0, 128.6, 127.5, 80.0, 66.5, 63.1, 55.1, 52.6, 51.4, 41.7, 28.5 ppm.
MS: ESI: [M+H]⁺ m/z trouvée 307.2, calculée 307.2
Rf = 0.28 (éther de pétrole : acétate d'éthyle / 7:3 / v:v)

### d) Préparation du composé 5: tert-butyl 4-benzyl-2-((1 ,3-dioxoisoindolin-2-yl)méthyl)pipérazine-1 -carboxylate

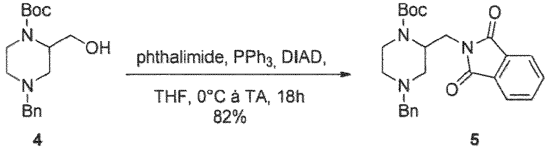

A une solution refroidie à 0°C du composé **4** (1,03g, 3,36mmol, 1,0eq.), de triphénylphosphine (1,07g, 4,03mmol, 1 ,2eq.) et de phthaiimide (600mg, 4,03mmol, 1 ,2eq.) dans du THF anhydre (20mL) est ajouté goutte à goutte le DIAD (845µL , 4,03mmol, 1 ,2eq.). Le mélange réactionnel est agité à 0°C pendant 30min puis à TA pendant 18h. Les volatiles sont évaporés et l'huile résultante est purifiée par colonne de chromatographie sur gel de silice (éluant : éther de pétrole : acétate d'éthyle / 8:2 / v:v) pour obtenir le composé 5 sous forme de solide blanc (1,20g, 2,76mmol, rendement: 82%).

¹H-RMN (500 MHz, CDCl₃): δ = 7.79 (m, 4H), 7.30 (m, 5H), 4.55(m, 1.5H), 4.45 (m, 0.5H), 3.98 (d, J=13Hz, 0.6H), 3.75 (d, J=12Hz, 0.4H), 3.48 (m, 4H), 2.28 (m, 2H), 2.18 (m, 1H), 2.05 (m, 1H), 1.08 + 1.03 (2xs, 9H) ppm.

¹³C-RMN (125 MHz, CDCl₃): δ = 168.5, 168.2, 167.9, 155.1, 154.4, 138.2, 134.5, 134.1, 133.7, 132.8, 132.4, 129.0, 128.7, 128.5, 127.4, 123.7, 123.4, 123.3, 79.7, 62.9, 54.5, 53.3, 50.3, 48.8, 40.1, 38.6, 37.8, 28.4, 27.9 ppm.
MS: ESI: [M+H]⁺ m/z trouvée 436.3, calculée 436.2
Rf = 0.32 (éther de pétrole : acétate d'éthyle / 8:2 / v:v)

### e) Préparation du composé 6: tert-butyl 2-(aminométhyl)-4- benzylpipérazine-1 - carboxylate

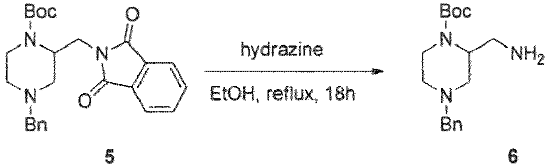

A une solution du composé **5** (1,20g, 2,76mmol, 1,0eq.) dans l'EtOH (30mL) est ajoutée l'hydrazine monohydrate (620mg, 12mmol, 4,0eq.) et le mélange réactionnel est porté à reflux pendant 18h. A la fin de la réaction, la suspension est refroidie à TA et est filtrée. Le filtrat est évaporé sous pression réduite pour donner un solide qui est re-suspendu dans du DCM (50mL), filtré une fois de plus et le filtrat est lavé deux fois avec une solution aqueuse saturée de NaHCO₃ (2x50mL) et une fois avec de la saumure (50mL). La phase organique est séchée avec du Ma₂S0₄, filtrée et évaporée pour obtenir le composé 6 (610mg) sous forme d'huile. Ce produit est utilisé dans l'étape suivante sans autre purification.

¹H-RMN (500 MHz, CDCl₃): δ = 7.35 (m,5H), 3.97 (br s, 2H), 3.57 (d, J = 13.2 Hz, 1H), 3.43 (d, J = 13.2 Hz, 1H), 3.07 (dd, J = 13.1, 7.0 Hz, 1H), 2.94 (dd, J = 13.1, 7.0 Hz, 1H), 2.81 (m, 2H), 2.10 (m, 2H), 1.50 (s, 9H) ppm.

¹³C-RMN (125 MHz, CDCl₃): δ = 155.33, 138.36, 128.76, 128.49, 128.35, 128.13, 127.19, 79.74, 62.82, 53.55, 53.30, 53.13, 48.55, 41.64, 28.48, 21.99 ppm.

MS: ESI: [M+H]⁺ m/z trouvée 306.3, calculée 306.2

### f) Préparation du composé 7: tert-butyl 4-benzyl-2-((2-phénylacétamido)méthyl)pipérazine-1-carboxylate

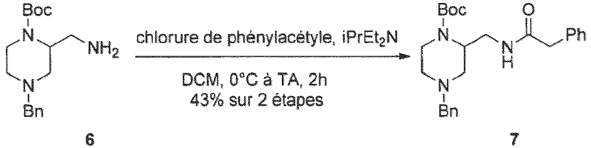

A une solution refroidie à 0°C du composé 6 (600mg, 1,96mmol, 1,0eq.) et de diisopropyléthylamine (DIPEA) (530µL, 3,0mmol, 1,5eq.) dans du DCM anhydre (15mL) est ajoutée goutte à goutte une solution de chlorure de phénylacétyle (318µL, 2,35mmol, 1,2eq.) dans du DCM anhydre (5mL). Le mélange réactionnel est agité à 0°C pendant 30min puis à TA pendant 1h30. A la fin de la réaction, le mélange est lavé trois fois avec une solution aqueuse saturée de NaHCOs (3x20mL). La phase organique est séchée avec du Na₂SO₄, filtrée et évaporée. Le brut réactionnel est purifié par colonne de chromatographie sur gel de silice (éluant : éther de pétrole : acétate d'éthyle / 55:45 / v:v) pour obtenir le composé 7 sous forme solide visqueux (500mg, 1.16mmol, rendement: 46% sur 2 étapes).

¹H-RMN (500 MHz, CDCl₃): δ = 7.27 - 7.06 (m, 10H), 6.12 (s, 1H), 4.08 (s, 1H), 3.43 - 3.36 (m, 3H), 3.21 (d, J = 13.0 Hz, 1H), 2.61 (d, J = 11.6 Hz, 1H), 2.57 (d, J= 11.6 Hz, 1H), 1.99 (dd, J = 11.6, 3.8 Hz, 1H), 1.92 (td, J = 11.6, 3.8 Hz, 1H), 1.36 (s, 9H) ppm. ¹³C-RMN (125 MHz, CDCl₃): δ = 171.0, 137.8, 135.1, 129.4, 128.9, 128.9, 128.8, 128.4, 127.3, 127.2, 80.1, 62.8, 54.2, 53.0, 49.7, 43.9, 41.0, 40.1, 28.5 ppm.
MS: ESI: [M+H]⁺ m/z trouvée 424.3, calculée 424.3
Rf = 0,23 (éther de pétrole : acétate d'éthyle / 6:4 / v:v)

### g) Préparation du composé 8: N-((4-benzylpipérazin-2-yl)méthyl)-2-phénylacétamide, sous la forme de sel avec l'acide trifluoroacétique

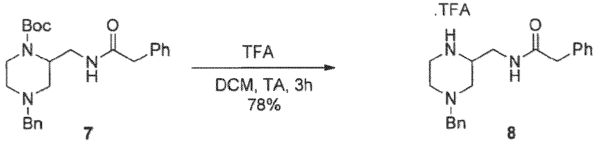

A une solution du composé 7 (500mg, 1.18mmol, 1.0eq.) dans du DCM (4mL) est ajouté de l'acide trifluoroacétique (TFA) (4mL) et le mélange réactionnel est agité à TA pendant 3h. A la fin de la réaction, te mélange réactionnel est évaporé sous pression réduite, redissout dans du DCM et évaporé une nouvelle fois. Cette opération est répétée deux autres fois. L'huile résultant est dissoute dans du MeOH et le produit est précipité dans de l'Et₂0. La suspension est filtrée et séchée à l'air pour obtenir le composé **8** sous forme d'une poudre blanche (401 mg, 0,92mmol, rendement: 78%).

¹H-RMN (500 MHz, CD₃OD): δ = 7.42 - 7.22 (m, 9H), 3.70 - 3.59 (m, 2H), 3.57 (s, 2H), 3.53 - 3.46 (m, 1H), 3.41 - 3.36 (m, 3H), 3.15 (td, J = 11.3, 3.5 Hz, 1H), 2.98 (d, J= 11.3 Hz, 2H), 2.44 (t, J= 11.3 Hz, 1H), 2.24 (t, J = 11.3 Hz, 1H) ppm.

¹³C-RMN (125 MHz, CDsOD): δ = 175.40, 136.40, 130.39, 130.26, 129.70, 129.62, 128.85, 128.13, 63.15, 56.87, 53.89, 50.29, 49.54, 44.80, 43.69, 40.74 ppm.

MS: ESI: [M+H]⁺ m/z trouvée 324.3, calculée 324.3

### II - Préparation de l'intermédiaire 10, le : 4-méthyl-2-oxo-2H-chromèn-7-yl carbonochloridate

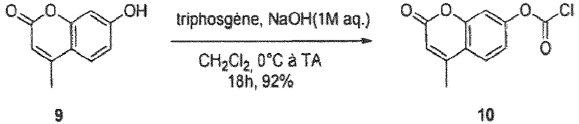

A une solution refroidie à 0°C de triphosgène (420 mg, 1,4 mmol, 0,7 eq) dans du DCM (10 mL) est ajoutée la 4-méthylumbelliférone **9** (360 mg, 2,0 mmol) en une portion. A cette suspension est additionnée goutte à goutte une solution aqueuse de NaOH (2M, 1,1 mL, 2,2 mmol, 1,1 eq) et le mélange réactionnel est agité à 0 °C pendant Ih puis à TA pendant 18h. La suspension est ensuite filtrée et le solide résultant est lavé deux fois avec du DCM et séché à l'air pour donner environ la moitié de la masse de composé **10** attendue (202 mg, 0,85 mmol). Le filtrat est alors lavé deux fois avec de l'eau et la phase organique est séchée avec du Na₂SO₄, filtrée et évaporée pour donner une autre portion de composé **10** (240 mg, 1,0 mmol) sous forme d'une poudre blanche. Le rendement global de cette réaction est de 93 %.

¹H-RMN (300 MHz, CDCl₃): δ = 7.69-7.62 (m, 1H), 7.33-7.19 (m, 2H), 6.31 (s, 1H), 2.46 (s, 3H,) ppm.

Rf = 0.47 (cyclohexane : acétate d'éthyle / 6:4 / v:v)

### III - Préparation du composé 12, l'hydrochlorure de 4-methyl-2-oxo-2H- chromen-7-yl 2-((2-phenylacetamido) methyl)piperazine-1-carboxylate (EXEMPLE 1)

### a) Préparation du composé 11 : 4-méthyl-2oxo-2H-chromen-7yl-4-benzyl-2-((2-phénylacétamido)méthyl)pipérazine-1-carbo-xylate

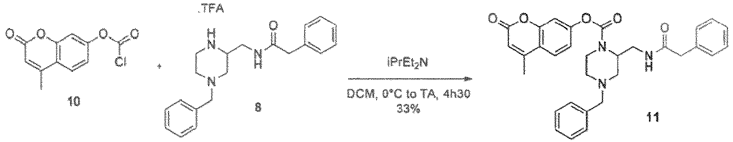

A une solution refroidie à 0°C du composé **10** (230mg, 0,96mmol, 1,05eq.) dans du DCM anhydre (10mL) sous une atmosphère inerte d'argon est additionnée goutte à goutte une solution du composé **8** (400mg, 0,91mmol, 1,0eq.) et de DIPEA (480µL 2,73mmol, 3,0eq.) dans du DCM anhydre (5mL). Le mélange réactionnel est ensuite agité à 0°C pendant 30min puis à TA pendant 4b. A la fin de la réaction, le mélange réactionnel est lavé trois fois avec une solution aqueuse saturée de NaHCOs (3x20mL) et la phase organique est séchée avec du Na₂S0₄, filtrée et évaporée. Le brut réactionnel est purifié par colonne de chromatographie sur gel de silice (éluant : gradient de DCM : MeOH / 99:1, 98:2, 97:3 / v:v) pour obtenir le composé **11** sous forme d'une huile incolore (160mg, 0,31mmol, rendement: 33%).

¹H-RMN (500 MHz, CDCl₃): δ = 7.46 (2xd, J - 8.5 Hz, 1H), 7.26 - 7.06 (m, 10H), 7.02 (d, J= 9.7 Hz, 1H), 6.97 (t, 7= 7.4 Hz, 1H), 6.25 (s, 0.5H), 6.13 (br s, 1H), 6.04 (s, 0.5H), 4.33 (s, 0.5H), 4.16 (s, 0.5H), 4.06 - 3.99 (m, 0.5H), 3.91 (t, J = 13.2 Hz, 1H), 3.70 - 3.61 (m, 0.5H), 3.50 - 3.34 (m, 4H), 3.28 (t, J = 12.6 Hz, 1H), 3.20 - 3.05 (m, 1H), 2.72 (d, J = 11.9 Hz, 1H), 2.65 (d, J = 11.8 Hz, 1H), 2.31 (d, J = 7.7 Hz, 3H), 2.13 (dd, J= 11.9, 4.0 Hz, 1H), 2.06 (t, J= 11.8 Hz, 1H) ppm.

¹³C-RMN (125 MHz, CDCl₃): δ = 171.23, 160.68, 154.08, 153.82, 153.40, 152.76, 152.12, 137.53, 134.76, 129.38, 129.31, 128.90, 128.88, 128.44, 127.40, 127.24, 125.22, 118.11, 117.96, 117.37, 117.24, 114.20, 114.12,110.29, 110.07, 62.63, 54.00, 53.85, 53.51, 52.74, 51.14, 43.79, 40.97, 40.46, 40.37, 18.72 ppm.
MS: ESI: [M+H]⁺ m/z trouvée 526.3, calculée 526.3
Rf = 0.38 (DCM : MeOH / 97:3 / v:v)

### b) Préparation du 12: hydrochlorure de 4-méthyl-2-oxo-2H-chromen-7-yl 2-((2-phénylacétamido)méthyl)pipérazine-1-carboxylate

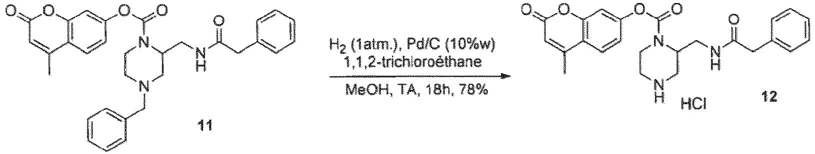

Un ballon contenant une solution du composé **11** (120mg, 0,23mmol, 1,0eq.) et du 1,1,2-trichloroéthane (25µL, 0,25mmol, 1,1leq.) dans du MeOH (5mL) est purgée pendant 5min avec du H₂. Le Pd/C (10%, 24mg) est ajouté et le ballon est de nouveau purgé avec du H₂ pendant 5min. Le mélange réactionnel est ensuite agité à TA pendant 18h sous une atmosphère de H₂. A la fin de la réaction, le mélange est filtré sur Celite^{®} et le filtrat est évaporé sous pression réduite jusqu'à un volume approximatif de 2mL Et₂O est alors ajouté pour faire précipiter le produit. La suspension résultante est filtrée et séchée à l'air pour donner le composé **12** sous forme d'une poudre blanche (85mg, 0.18mmol, rendement: 78%).

¹H-RMN (500 MHz, C¾00): δ = 7.80 (d, J = 8.4 Hz, 1H), 7.35 - 7.07 (m, 7H), 6.33 (s, 1H), 4.63 (2xs, 1H), 4.33 (2xs, 1H), 3.78 - 3.66 (m, 1H), 3.53 (s, 3H), 3.45 - 3.32 (m, 3H), 3.21 (s, 1H), 2.49 (s, 3H) ppm.

¹³C-RMN (125 MHz, CDsOD): δ = 175.07, 162.63, 155.25, 155.00, 136.46, 130.19, 129.71, 128.09, 127.15, 119.38, 119.02, 114.83, 111.09, 50.92, 50.42, 44.39, 44.04, 43.75, 38.60, 38.32, 37.44, 37.39, 18.72 ppm.

HRMS: C₂₄H₂₆N₃O₅ [M+H]⁺ m/z trouvée 436.1858 calculée 436.1867

### IV - Préparation de l'intermédiaire 14, le 2-phényl-N-(pipéridin-2-ylméthyl)acétamide :

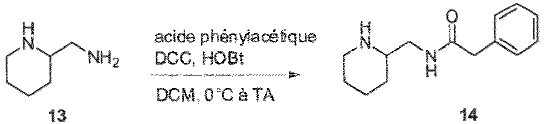

A une suspension d'acide phénylacétique (1,54g, 11 ,2mmol, 1,0eq.) et d'HOBt (1,66g, 12,3 mmol, 1,1 eq) dans du DCM (30 mL) est additionnée une solution de dicyclohexylcarbodiimide (2,56g, 12,3 mmol, 1,1 eq) dans du DCM (10 mL). Le mélange résultant est agité à TA pendant 30min avant que la 2-(aminométhyl)pipéridine **13** (1 ,4mL, 11,2 mmol, 1,0 eq) ne soit ajoutée. Le mélange réactionnel est ensuite agité à TA pendant 2h. La suspension résultante est filtrée et le filtrat est lavé deux fois avec une solution aqueuse saturée de NaHCOs (2x50 mL). La phase organique est ensuite extraite plusieurs fois avec une solution aqueuse à 10% de KH₂PO₄ jusqu'à ce que le pH de la phase aqueuse soit stable autour de pH 3. Du DCM (150 mL) est ensuite ajouté aux phases aqueuses recombinées et une solution aqueuse de NaOH (2M) est additionnée jusqu'à ce que le pH de la phase aqueuse atteigne pH 12. La phase organique est soutirée et la phase aqueuse basique est extraite deux fois avec du DCM (2x100 mL). Les phases organiques sont combinées, séchées avec du Na₂SO₄, filtrées et évaporées pour donner le composé **14** sous forme de solide blanc (2,07g, 8,9mmol, rendement: 80%).

¹H-RMN (200 MHz, CDCl₃): δ = 7.83 (m, 5H), 5.80 (s, 1H), 3.57 (s, 2H), 3.25 (m, 1H), 3.04 (m, 2H), 2.59 (m, 2H), 1.75 (m, 1H), 1.56 (m, 2H), 1.32 (m, 4H), 1.03 (m, 1H) ppm.

MS: ESI: [M+H]⁺ m/z trouvée 233.3, calculée 233.3

### V - Préparation du composé 15, le 4-méthyl-2-oxo-chromen-7-yl 2-((phénylacétomido)méthyl)pipéridine-1-carboxylate (comparatif 1)

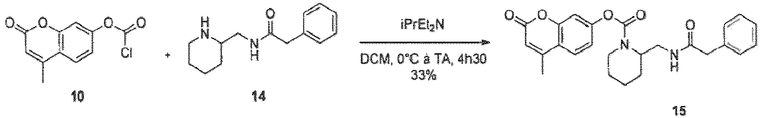

A une suspension refroidie à 0°C du composé **10** (39mg, 0,16mmol, 1,05eq.) dans du DCM anhydre (5mL) sous une atmosphère inerte d'argon, est additionné le composé **14** (25mg, 0,15mmol, 1,0eq.) suivi par la DIPEA (55µL, 0,30mmol, 2,0eq.). Le mélange réactionnel est agité à 0°C pendant 30min et à TA pendant 4h. A la fin de la réaction, le mélange réactionnel est lavé trois fois avec une solution aqueuse saturée de NaHCOs (3x20mL) et la phase organique est séchée avec du Na₂S0₄, filtrée et évaporée. Le brut réactionnel est purifié par colonne de chromatographie sur gel de silice (éluant : gradient de DCM : MeOH / 99:1, 98:2, 97:3/ v:v) pour obtenir le composé **15** sous forme d'une huile incolore (35mg, 0.31 mmol, rendement: 54%).

¹H-RMN (500 MHz, CDCl₃): δ = 7.49 (s, 1H), 7.30 - 7.04 (m, 5H), 7.04 - 6.88 (m, 2H), 6.16 (s, 1H), 5.90 (s, 1H), 4.48 - 4.23 (m, 1H), 4.03 (d, 7 = 13.5 Hz, 1H), 3.82 (s, 1H), 3.64 - 3.27 (m, 3H), 3.12 (s, 1H), 2.35 (s, 3H), 1.77 - 1.36 (m, 6H) ppm.

¹³C-RMN (125 MHz, CDCl₃): δ = 171.42, 160.73, 154.14, 153.99, 153.83, 152.12, 134.62, 129.75, 129.36, 129.00, 128.38, 127.35, 126.76, 125.19, 118.10, 117.32, 114.20, 110.34, 53.51, 51.09, 50.93, 46.72, 43.69, 41.28, 40.83, 40.64, 39.53, 38.94, 31.69, 26.53, 25.28, 24.39, 23.04, 19.09, 18.76 ppm.

HRMS: C₂₅H₂₆N₂NaO₅ [M+Na]⁺ m/z trouvée 457.1734 calculée 457.1734 R_{f} = 0.27 (DCM : MeOH / 98:2 / v:v)

### VI - Préparation de l'intermédiaire 19, le(4-benzylpipérazin-2-yl)méthyl octanoate sous la forme de sel avec l'acide trifluoroacétique

### a) Préparation du composé 17 : chlorure d'octanoyle

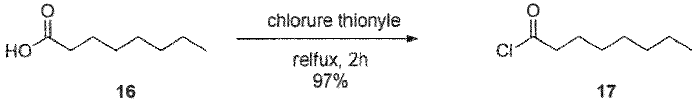

A de l'acide octanoïque **16** (10,0g, 69mmol, 1,0eq.) est additionné goutte à goutte du chlorure de thionyle (30mL, 410mmol, 6,0eq.). Ce mélange réactionnel est ensuite porté à reflux pendant 2h, puis refroidi à TA et les volatiles sont évaporés sous pression réduite. L'huile résultante est reprise dans du DCM et la solution est à nouveau évaporée. Cette procédure est répétée deux fois supplémentaires pour obtenir le composé **17** sous forme d'un liquide jaune très odorant (11,0g, 66,7mmol, rendement: 97%).

¹H-RNM (300 MHz, CDCl₃): δ = 2.87 (t, J = 7.3 Hz, 2H), 1.70 (q, J= 7.3 Hz, 2H), 1.42 - 1.22 (m, 8H), 0.88 (t, 7= 6.6 Hz, 3H) ppm.

¹³C-RNM (75 MHz, CDCl₃) δ = 173.89, 47.23, 31.64, 28.85, 28.51, 25.20, 22.65, 14.11 ppm.

### b) Préparation du composé 18 : tert-butyl 4-bensyl-2-((octanoyloxy)méthyl)pipérazine-1-carboxylate

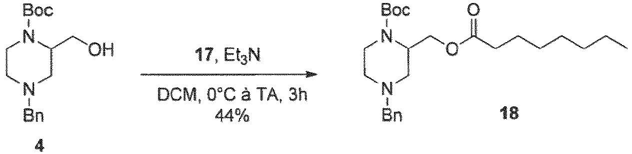

A une solution refroidie à 0°C du composé **4** (800mg, 3,72mmol, 1,0eq.) et de triéthylamine (780µL , 5,6mmol, 1 ,5eq.) dans du DCM anhydre (10mL) est additionnée goutte à goutte une solution du composé **17** (667mg; 4,lmmol, 1,1eq.) dans du DCM anhydre (5mL). Le mélange réactionnel est agité à 0°C pendant 30min puis à TA pendant 2h30. Il est ensuite dilué avec du DCM (35mL) et lavé une fois avec une solution aqueuse saturée de NaHCO₃ (50mL), une fois avec de l'eau (50mL), une fois avec une solution aqueuse d'HCl (1M - 50mL) et une fois avec de la saumure (50mL). La phase organique est séchée avec du Na₂SO₄, filtrée et évaporée. Le brut réactionnel est purifié par colonne de chromatographie sur gel de silice (DCM pur, puis DCM : acétate d'éthyle / 90:10) pour obtenir 18 sous forme d'une huile jaune (710mg, 1.64mmol, rendement : 44%).

¹H-RMN (500 MHz, CD₃OD): δ = 7.34 - 7.16 (m, 5H), 4.39 - 4.18 (m, 3H), 3.88 (s, 1H), 3.53 (d, 7 = 13.1 Hz, 1H), 3.37 (d, J = 13.1 Hz, 1H), 3.09 (t, J = 11.3 Hz, 1H), 2.77 (d, 7 = 11.5 Hz, 2H), 2.20 (t, J = 7.6 Hz, 2H), 2.14 - 1.95 (m, 2H), 1.60 - 1.49 (m, 2H), 1.44 (s, 9H), 1.25 (S, 8H), 0.87 (t J = 6.4 Hz, 3H) ppm.

¹³C-RMN (125 MHz, CDsOD): δ = 173.72, 154.91, 138.16, 128.93, 128.42, 127.29, 79.97, 62.83, 61.56, 53.10, 52.82, 34.36, 31.80, 29.25, 29.04, 28.52, 24.95, 22.73, 14.20 ppm.

MS: Kl: [M+H]⁺ m/z trouvée 433.3, calculée 433.3

### c) Préparation du composé 19 : (4-benzylpipérazin-2-yl)méthyl octanoate sous la forme de sel avec l'acide trifluoroacétique

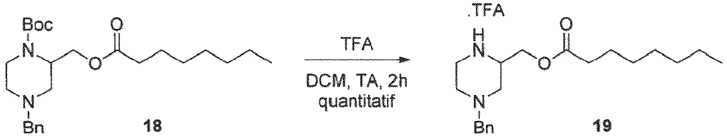

La procédure utilisée est la même que celle décrite pour la synthèse du composé **8,** mais en utilisant le composé **18** (700mg, 1,62mmol,) dans du DCM (5mL) et du TFA (5mL) pour donner le composé **19** sous forme d'une poudre blanche (775mg, rendement: quantitatif).

¹H-RMN (500 MHz, CD₃OD): δ = 7.41 - 7.27 (m, 5H), 4.30 (dd, J= 12.5, 3.9 Hz, 1H), 4.22 (dd, J = 12.5, 6.4 Hz, 1H), 3.70 - 3.63 (m, 1H), 3.46 (d, J = 13.2 Hz, 1H), 3.28 - 3.12 (m, 3H), 2.71 (td, J= 12.2, 12.2, 2.6 Hz, 1H), 2.61 (t, J = 11.8 Hz, 1H), 2.38 (t, J = 7.5 Hz, 2H), 1.66 - 1.52 (m, 2H), 1.30 (s, 8H), 0.89 (t, J= 6.7 Hz, 3H) ppm.

¹³C-RMN (125 MHz, CDsOD): δ = 174.57, 162.06, 161.77, 135.40, 130.95, 129.86, 129.62, 118.70, 116.40, 62.75, 62.55, 54.98, 52.20, 49.86, 43.97, 34.47, 32.84, 30.13, 30.07, 25.72, 23.65, 14.38 ppm.

MS: ESI: [M+H]⁺ m/z trouvée 333.3, calculée 333.2

### VII - Préparation du composé 21, l'hydrochlorure de 4-méthyl-2-oxo-2H-chromen-7-yl 2-((octanoyloxy)méthyl)pipérazine-1-carboxylate (EXEMPLE 2)

### a) Préparation du composé 20 : de 4-méthyl-2-oxo-2H-chromen-7-yl 2-((octanoyloxy)méthyl)pipérazine-1-carboxylate

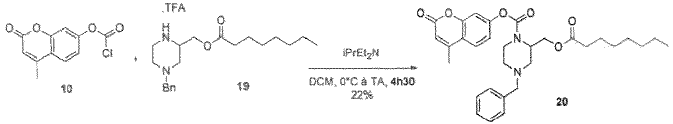

La procédure utilisée est la même que celle décrite pour la synthèse du composé **11,** avec le composé **10** (400mg, 1,67mmol, 1,0eq.), le composé **19** (750mg, 1,67mmol, 1,0eq.) et la DIPEA (880µL, 5,02mmol, 3,0eq.) dans du DCM anhydre (20mL). Après chromatographie sur gel de silice (éther de pétrole : acétate d'éthyle / 7:3 / v:v), on obtient le composé **20** sous forme d'un solide incolore (190mg, 0,36mmol, rendement: 22%).

¹H-RMN (500 MHz, CD₃OD): δ = 7.52 (d, J = 8.6 Hz, 1H), 7.24 - 7.16 (m, 1H), 7.11 - 7.00 (m, 2H), 6.18 (S, 1H), 4.57 - 4.38 (m, 2H), 4.27 (s, 1H), 4.09 - 3.95 (m, 1H), 3.54 (d, J = 13.1 Hz, 1H), 3.39 (d, J = 13.0 Hz, 1H), 3.23 (t, J = 11.9 Hz, 0.5H), 2.82 (d, J = 11.3 Hz, 2H), 2.36 (s, 3H), 2.21 - 2.10 (m, 3H), 1.55 - 1.43 (m, 2H), 1.26 - 1.12 (m, 8H), 0.87 - 0.75 (m, 3H) ppm.

¹³C-RMN (125 MHz, CDsOD): δ 173.46, 160.56, 154.10, 153.82, 152.88, 152.00, 137.65, 128.76, 128.35, 127.29, 125.17, 118.11, 117.90, 117.29, 114.14, 110.25, 110.05, 62.57, 61.57, 61.28, 52.75, 52.62, 51.16, 50.57, 41.02, 40.21, 34.13, 31.58, 29.03, 28.86, 24.76, 22.54, 18.65, 14.03 ppm.
MS: ESI: [M+H]⁺ m/z trouvée 535.3, calculée 535.3
Rf = 0.26 (éther de pétrole : acétate d'éthyle / 7:3 / v:v)

### b) Préparation du composé 21 : de 4-méthyl-2-oxo-2H-chromen-7-yl 2-((octanoyloxy)méthyl)pipérazine-1-carboxylate

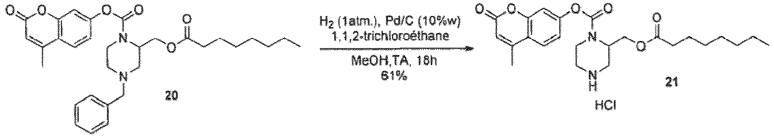

La procédure utilisée est la même que celle décrite pour la synthèse du composé **12,** avec le composé **20** (180mg, 0,33mmol, 1,0eq.), du 1,1,2- trichloroéthane (37µL, 0,39mmol, 1,1eq.), du Pd/C (38mg, 20%w) dans du MeOH (5mL) pour obtenir le composé 21 sous forme d'une poudre blanche (95mg, 0,20mmol, rendement: 61%).

¹H-RMN (500 MHz, CD₃OD): δ = 7.82 (d, J = 8.6 Hz, 1H), 7.32 - 7.13 (m, 2H), 6.34 (s, 1H), 4.67 (s, 1H), 4.43 - 4.22 (m, 2H), 3.53 (d, J = 13.4 Hz, 2H), 3.45 (d, J = 11.7 Hz, 3H), 3.26 (s, 1H), 2.49 (s, 3H), 2.35 (t J = 7.5 Hz, 2H), 1.57 (s, 2H), 1.26 (dd, J = 28.7, 21.9 Hz, 9H), 0.84 (t, J = 6.8 Hz, 3H) ppm.

¹³C-RMN (125 MHz, CDsOD): δ = 174.84, 162.51, 155.27, 154.96, 154.90, 154.05, 127.20, 119.25, 119.06, 114.87, 111.01, 61.14, 43.94, 43.50, 34.83, 32.80, 30.17, 30.07, 25.89, 23.63, 18.67, 14.36 ppm.

HRMS: C₂₄H₃₃N₂O₆ [M+H]⁺ m/z trouvée 445.2318 calculée 445.2333

### VIII - Préparation du composé 22, l'acide 4-(4-(((4-methyl-2-oxo-2H-chromen-7-yl)oxy)carbonyl)-3-((octanoyloxy)méthyl)pipérazin-1-yl)butane-1-sulfonique

### (EXEMPLE 3)

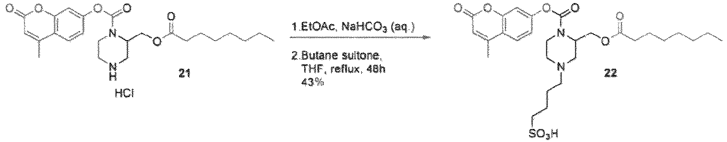

A une suspension du composé **21** (25mg, 0,052mmol, 1,0eq.) dans l'acétate d'éthyle (10mL) est ajoutée une solution aqueuse de NaHCO (1M, 10mL). La phase organique est soutirée et la phase aqueuse est lavée trois fois avec de l'acétate d'éthyle (3x10mL). Les phases organiques combinées sont séchées avec du Na₂S0₄, filtrées et évaporées pour donner une huile incolore. Cette huile est dissoute dans du THF anhydre (2,5mL), et de la butane sultone est ajoutée (2 gouttes) et le mélange réactionnel est chauffé à reflux pendant 48h. A la fin de la réaction, il est dilué avec du THF (2,5mL) et de l'Et₂O (5mL) et de l'eau est ajoutée (10mL). La phase aqueuse est séparée et lyophilisée pour donner le composé **22** sous forme d'un solide blanc très hygroscopique (13mg, 0,022mmol, rendement: 43%).

¹H-RMN (500 MHz, CD₃OD): δ = 7.72 (d, J = 8.6 Hz, 1H), 7.21 - 7.03 (m, 2H), 6.23 (d, J = 1.1 Hz, 1H), 4.59 (s, 1H), 4.38 - 4.12 (m, 2H), 3.62 - 3.24 (m, 4H), 3.12 - 2.83 (m, 3H), 2.80 (t, J = 6.9 Hz, 2H), 2.42 - 2.35 (m, 3H), 2.29 - 2.17 (m, 2H), 1.91 - 1.73 (m, 4H), 1.47 (s, 2H), 1.25 - 0.98 (m, 10H), 0.74 (t, J- 6.9 Hz, 3H) ppm.

¹³C-RMN (125 MHz, CDsOD): δ = 162.54, 155.30, 155.02, 154.93, 127.23, 119.30, 118.88, 114.88, 34.89, 32.83, 30.21, 30.10, 25.92, 23.67, 23.24, 18.71, 14.40 ppm. HRMS: C₂₈H₄₀N₂NaO₉S [M+Na]⁺ m/z trouvée 603.2339 calculée 603.2347

### IX - Préparation du composé 27, 4-méthyl-2-oxo-2H-chromen-7-yl 2-((octanoyloxy)méthyl)pipérazine-1-carboxylate (comparatif 2)

### a) Préparation du composé 24 : N-Boc-2-(hydroxymétthyl)pipéridine

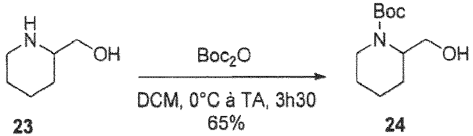

A une solution refroidie à 0°C du composé **23** (5g, 43mmol, 1,0eq.) dans du DCM (20mL) est ajoutée goutte à goutte une solution de Boc₂0 (6,6g, 47mmol, 1,1leq.) dans du DCM (10mL). Le mélange réactionnel est agité à 0°C pendant 30min, puis à TA pendant 3h. Il est ensuite dilué avec du DCM (30mL) et lavé une fois avec une solution aqueuse saturée de NaHCOs (3x60mL). La phase organique est séchée avec du Na₂SO₄, filtrée et évaporée pour obtenir le composé **24** (6,02g, 28mmo1, rendement: 65%) sous forme d'un solide jaune.

¹H-RMN (300 MHz, CDCl₃) δ = 4.36 - 4.22 (m, 1H), 3.94 (d, J = 12.3 Hz, 1H), 3.88 - 3.75 (m, 1H), 3.67 - 3.53 (m, 1H), 2.87 (t, J = 12.3 Hz, 1H), 2.01 (br s, 1H), 1.73 - 1.52 (m, 4H), 1.52 - 1.38 (m, 11H) ppm.

¹³C-RMN (75 MHz, CDCl₃) δ = 156.43, 79.92, 61.86, 52.68, 40.14, 28.57, 25.39, 25.35, 19.76 ppm.

### b) Préparation du composé 25 : N-Boc-2-((octanoyloxy)méthyl)pipéridine

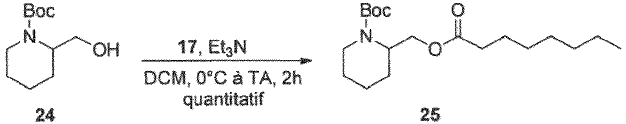

A une solution refroidie à 0°C du composé **17** (6,0g, 27,8mmol, 1,0eq.) et de triéthylamine (6mL, 42mmol, 1,5eq.) dans du DCM sec (40mL) est ajoutée goutte à goutte une solution du composé **24** (5,0g, 30,6mmol, 1,1 eq.) dans du DCM sec (20mL). Le mélange réactionnel est agité à 0°C pendant 30min et à TA pendant 3h. Il est ensuite dilué avec du DCM (40mL) et lavé une fois avec de l'HCl 1M (100mL), une fois avec de l'eau (50mL), une fois avec NaOH 1M (100mL), et une fois avec de la saumure (100mL). La phase organique est séchée avec du Na₂SO₄, filtrée et évaporée à sec pour donner le composé **25** (9,82g, rendement quantitatif) sous la forme d'un liquide jaune utilisé sans autre purification.

¹H-RMN (300 MHz, CDCl₃) δ = 4.40 (s, 0.6H), 4.26 - 4.11 (m, 0.9H), 4.10 - 3.99 (m, 1.3H), 3.99 - 3.80 (m, 0.8H), 3.76 - 3.64 (m, 0.2H), 3.59 - 3.47 (m, 0.2H), 2.86 - 2.65 (m, 0.8H), 2.48 (s, 0.2H), 2.37 (t, J = 7.5 Hz, 0.2H), 2.21 (t, J = 7.5 Hz, 2H), 1.68 - 1.43 (m, 6H), 1.43 - 1.30 (m, 9H), 1.30 - 1.08 (m, 10H), 0.80 (t, 7= 5.9 Hz, 3H) ppm. ¹³C-RMN (75 MHz, CDCl₃) δ = 173.77, 155.15, 79.54, 61.71, 34.38, 31.75, 29.21, 28.99, 28.52, 25.50, 25.35, 24.98, 22.67, 19.42, 14.13 ppm.

### c) Préparation du composé 26 : pipéridine-2-ylméthyl octanoate sous la forme de sel avec de l'acide trifluoroacétique

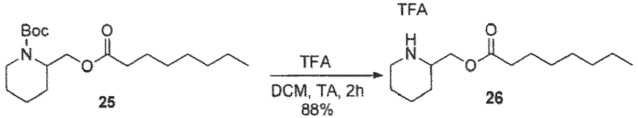

La procédure utilisée est la même que celle décrite pour la synthèse du composé **8,** mais en utilisant le composé **25** (700mg, 1,62mmol, 1,0eq.) dans du DCM (3mL) et du TFA (3mL) pour donner le composé **26** sous forme d'une poudre blanche (910mg, 2,56mmol, rendement : 88%).

¹H-RMN (300 MHz, CDCl₃) δ= 8.93 (s, 1H), 8.25 (s, 1H), 4.54 (d, 7= 5.4 Hz, 0.1H), 4.32 (d, J = 3.4 Hz, 0.3H), 4.28 (d, J= 3.4 Hz, 0.6H), 4.23 - 4.07 (m, 1H), 3.50 (d, J = 12.7 Hz, 1H), 3.43 - 3.26 (m, 1H), 3.03 - 2.83 (m, 1H), 2.30 (t, J= 7.6 Hz, 1H), 2.06 - 1.46 (m, 8H), 1.39 - 1.17 (m, 9H), 0.87 (t, J = 5.7 Hz, 3H) ppm.

¹³C-RMN (75 MHz, CDCl₃) δ = 173.81, 161.67, 117.60, 63.67, 56.40, 45.11, 33.69, 31.71, 29.06, 28.95, 25.16, 24.67, 22.66, 22.06, 21.97, 14.10 ppm. MS: ESI: [M+H]⁺ m/z trouvée 241.2, calculée 241.2

### d) Préparation du composé 27: 4-méthyl-2-oxo-2H-chromen-7-yl 2-((octanoyloxy)méthyl)pipérazine-1-carboxylate

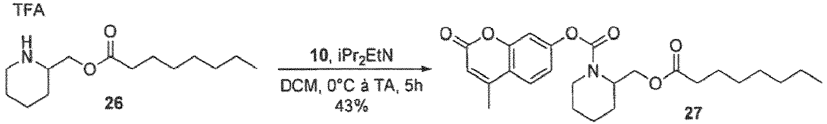

La procédure utilisée est la même que celle décrite pour la synthèse du composé **11,** avec le composé **10** (190mg, 0,79mmol, 1,0eq.) le composé **26** (285mg, 0,79mmol, 1,0eq.) et la DIPEA (700µL , 3,95mmol, 4,0eq.) dans du DCM anhydre (lOmL). Après chromatographie sur gel de silice (éther de pétrole : acétate d'éthyle / 75:25 / v:v), on obtient le composé **27** sous forme d'une huile incolore (150mg, 0,34mol, rendement 43%).

¹H-RMN (500 MHz, COCl₃) δ= 7.57 (d, J= 8.6 Hz, 1H), 7.16 - 7.05 (m, 2H), 6.24 (s, 1H), 4.68 (s, 1H), 4.41 (t, 7= 9.9 Hz, 1H), 4.22 - 4.10 (m, 2H), 3.13 (br s, 0.5H), 2.98 (br s, 0.5H), 2.42 (s, 3H), 2.28 (t, J= 6.8 Hz, 2H), 1.85 - 1.66 (m, 4H), 1.65 - 1.50 (m, 4H), 1.24 (br s, 8H), 0.84 (t, J = 6.6 Hz, 3H) ppm.

¹³C-RMN (125 MHz, CDCl₃) δ = 173.77, 160.82, 154.33, 154.20, 153.29, 152.12, 125.22, 118.25, 117.39, 114.31, 110.39, 61.67, 50.35, 50.00, 40.89, 40.09, 34.37, 31.75, 29.22, 29.03, 25.81, 25.12, 24.99, 22.70, 19.32, 18.85, 14.17 ppm.
HRMS: C₂₅H₃₃NNaO₆ [M+Na]⁺ m/z trouvée: 466.2179 calculée 466.200
Rf = 0.34 (éther de pétrole : acétate d'éthyle / 75:25 / v:v)

### X - Préparation du composé 31, le 4-méthyl-2-oxo-2H-chromen-7-ylm 4-(3-(1-(2-(2-méthoxyéthoxy)éthoxy)éthyl)-1H-1,2,3-triazol-4-yl)propanoyl)2-2((2-phenylacétamido)méthyl)pipéarzine-1-carboxylate (EXEMPLE 4)

### a) Préparation du composé 29, 1-azido-2-(2-méthoxyéthoxy)éthoxy)éthane

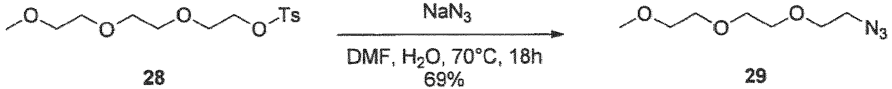

A une solution du composé **28** (2,0g, 6,28mmol, 1,0eq.) dans un mélange DMF (15mL) et eau (8mL) est additionné le NaN₃ (2,04g, 31,40mmol, 5,0eq.) en une portion. Ce mélange réactionnel est ensuite chauffé à 70°C pendant 18h. Les volatiles sont ensuite évaporés et le solide résultant est repris dans l'acétate d'éthyle (50mL). La suspension est filtrée et le filtrat est lavé deux fois avec une solution aqueuse saturée de NaHCOs (2x50mL), puis une fois avec de la saumure (50mL). La phase organique est séchée avec du Na₂S0₄, filtrée et évaporée pour donner le composé **29** sous forme d'une huile incolore (820mg, 4,33mmol, rendement : 69%).

¹H-RMN (300 MHz, CDCl₃) δ = 3.68 - 3.57 (m, 8H), 3.55 - 3.48 (m, 2H), 3.38 - 3.32 (m, 5H) ppm.

¹³C-RMN (75 MHz, CDCl₃) δ = 71.94, 70.71, 70.68, 70.63, 70.05, 59.04, 50.69 ppm. Rf = 0.56 (éther de pétrole : acétate d'éthyle/ 1:1 / v:v)

### b) Préparation du composé 30, 4-méthyl-2-oxo-2H-chromen-7-yl 4-(pent-4-ynoyl)-2-((2-phénylacétmido)méthyl)pipérazine-1-carboxylate

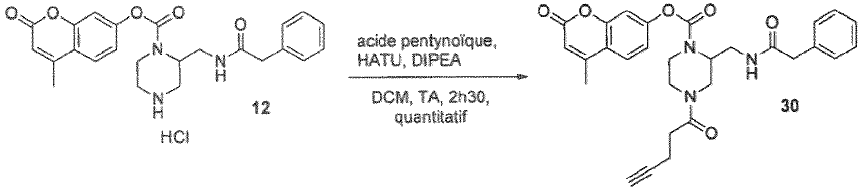

A une suspension du composé **12** (50mg, 0,106mmol, 1,0eq.), d'acide pentynoïque (12mg, 0,117mmol, 1,1eq.) et de HATU (46mg, 0,117m mol, 1,1eq.) dans du DCM anhydre (5mL) sous atmosphère inerte d'argon, est additionnée la DIPEA (42µL , 0,240mmol, 2,2eq.) et le mélange réactionnel est agité à TA pendant 2h30. A la fin de la réaction, le mélange réactionnel est dilué avec du DCM (20mL) et lavé deux fois avec une solution aqueuse saturée de NaHCOs (2x20mL) et une fois avec de la saumure (20mL). La phase organique est séchée avec du Na₂SO₄, filtrée et évaporée. Le brut réactionnel est purifié par colonne de chromatographie sur alumine neutre (Ether de pétrole : Acétate d'éthyle / 1:9 / v:v) pour obtenir le composé **30** sous forme d'une huile incolore (60mg, rendement: quantitatif).

¹H-RMN (500 MHz, CDCl₃) δ = 7.59 (dd, J = 14.8, 8.6 Hz, 1H), 7.36 - 7.27 (m, 2H), 7.27 - 7.21 (m, 2H), 7.21 - 7.02 (m, 2H), 6.73 - 6.45 (m, 1H), 6.32 - 6.04 (m, 1H), 4.67 - 4.25 (m, 2H), 4.17 - 3.69 (m, 3H), 3.66 - 3.47 (m, 3H), 3.45 - 3.18 (m, 2H), 3.18 - 2.89 (m, 2H), 2.81 (s, 3H), 2.74 - 2.63 (m, 1H), 2.60 - 2.47 (m, 3H), 2.44 (s, 3H), 2.20 (S, 1H), 2.13 - 1.96 (m, 1H) ppm.

¹³C-RMN (125 MHz, CDCl₃) δ = 171.52, 171.36, 170.69, 170.37, 170.27, 165.73, 160.62, 154.03, 153.52, 153.23, 152.78, 152.16, 134.78, 134.65, 129.23, 128.97, 128.88, 127.33, 127.21, 125.35, 117.97, 117.58, 117.45, 114.33, 114.22, 110.28, 110.16, 83.32, 83.05, 69.34, 69.03, 53.52, 51.33, 45.59, 45.20, 45.03, 43.62, 42.37, 42.19, 41.21, 40.48, 40.19, 39.98, 38.62, 38.05, 37.50, 31.77, 18.74, 14.66, 14.36 ppm.
MS: ESI: [M+H]⁺ m/z trouvée 515.3, calculée 515.2
Rf = 0.20 (éther de pétrole : acétate d'éthyle/ 1:9 / v:v)

### c) Préparation du composé 31, 4-méthyl-2-oxo-2H-chromen-7-yl 4-(3-(1-(2-(2-méthoxyéthoxy)éthoxy)éthyl)1-H-1,2,3-triazol-4-yl)propanoyl)-2-((2-phenylacétamido)méthyl)pipérazine-1-carboxylate

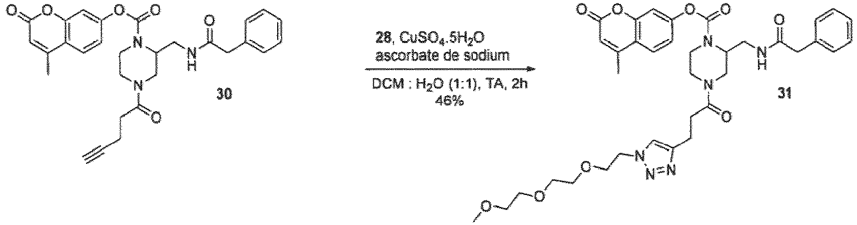

A une solution du composé **30** (30mg, 0,058mmol, 1,0eq.) et du composé 28 (24mg, 0,128m mol, 2,2eq.) dans un mélange DCM (1mL) et eau (1mL) sont additionnés le CuSO₄.5H₂O (9mg, 0,04mmol, 0,6eq.) et l'ascorbate de sodium (16mg, 0,08mmol, 1 ,2eq.) en une portion. Ce mélange réactionnel est ensuite agité à TA pendant 2h. A la fin de la réaction, le mélange réactionnel est dilué avec du DCM (10mL) et de l'eau (10mL). La phase organique est soutirée et la phase aqueuse est lavée trois fois avec du DCM (3x10mL). Les phases organiques combinées sont séchées avec du Na₂SO₄, filtrées et évaporées. Le brut réactionnel est purifié par colonne de chromatographie sur gel de silice (éluant : gradient de DCM : MeOH / 100 : 0, 99:1, 98:2, 97:3, % : 4, 95 : 5 / v:v) pour donner le composé **31** sous forme d'une poudre blanche (19mg, 0,027mmol, rendement : 46%).

¹H-RMN (500 MHz, CDCl₃) δ = 7.96 (s, 0.25H), 7.47 (m 1.5H), 7.32 - 6.94 (m, 7H), 6.61 (s, 0.2H), 6.21 (m, , 1H), 5.86 (s, 0.2H), 4.73 (s, 0.25H), 4.55 - 4.17 (m, 3H), 4.01 - 3.69 (m, 4.5H), 3.65 - 3.61 (m, 1.5H), 3.59 - 3.50 (m, 6.5H), 3.50 - 3.38 (m, 3H), 3.31 (d, J = 5.4 Hz, 2.5H), 3.26 - 2.55 (m, 6.5H), 2.36 (s, 3H), 1.74 (s, 1H) ppm.

¹³C-RMN (125 MHz, OX½) δ = 172.66, 171.54, 170.71, 160.77, 154.23, 153.64, 152.93, 152.10, 135.49, 134.96, 134.70, 129.42, 129.03, 128.85, 127.35, 127.14, 125.36, 122.%, 118.17, 117.60, 114.42, 110.45, 72.04, 70.64, 69.56, 67.21, 59.18, 51.27, 50.71, 50.21, 45.48, 45.21, 44.77, 44.02, 43.86, 42.43, 42.22, 41.15, 40.55, 40.24, 40.00, 39.36, 38.12, 37.72, 37.42, 32.04, 31.66, 29.81, 28.81, 21.34, 20.57, 18.86 ppm.
MS: ESI: [M+H]⁺ m/z trouvée 704.5, calculée 704.3
Rf = 0.30 (DCM : MeOH/ 95:5 / v:v)

### XI - Préparation de l'intermédiaire 34, N-((4-(pent-4-ynoyl)pipérazin-2-yl)méthyl)-2-phénylacétamide sous forme de sel avec l'acide trifluoroacétique

### a) Préparation du composé 32, tert-butyl 2-((2-phénylacétamido)méthyl)pipérazine-1-carboxulate sel d'hydrochlorure

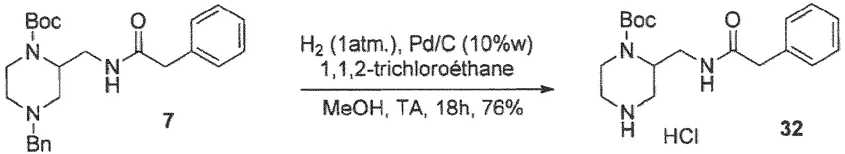

La procédure utilisée est la même que celle décrite pour la synthèse du composé **12,** avec le composé 7 (605mg, 1,53mmol, 1,0eq.), du 1,1,2-trichloroéthane (161µL, 1,70mmol, 1,1eq.), du Pd/C (121mg, 20%massique) dans du MeOH (30mL) pour obtenir le composé **32** sous forme d'une poudre blanche (430mg, 1,16mmol, rendement: 76%).

¹H-RMN (300 MHz, CDCl₃) δ = 7.43 - 7.15 (m, 5H), 6.25 (s, 1H), 4.47 - 4.15 (m, 2H)ᵣ 4.00 - 3.57 (m, 2.7H), 3.52 (d, J - 2.9 Hz, 2H), 3.33 (m, 0.7H), 3.28 - 2.85 (m, 2.8H), 2.68 - 2.29 (m, 4.5H), 2.00 (d, J= 8.4 Hz, 1H), 1.47 (s, 9H) ppm.

¹³C-RMN (75 MHz, CDCl₃) δ = 171.53, 171.33, 170.51, 170.26, 165.70, 154.91, 134.84, 129.22, 128.92, 128.78, 127.26, 127.09, 83.36, 83.06, 80.81, 69.19, 68.90, 53.49, 49.97, 45.37, 43.60, 42.10, 41.32, 31.77, 31.69, 28.29, 14.55, 14.29 ppm. MS: ESI: [M+H]⁺ m/z trouvée 333.2, calculée 333.2

### b) Préparation du composé 33, tert-butyl 4-(pent-4-ynoyl)-2-((2-phénylcétamido)méhtyl)pipérazine-1-carboxylate

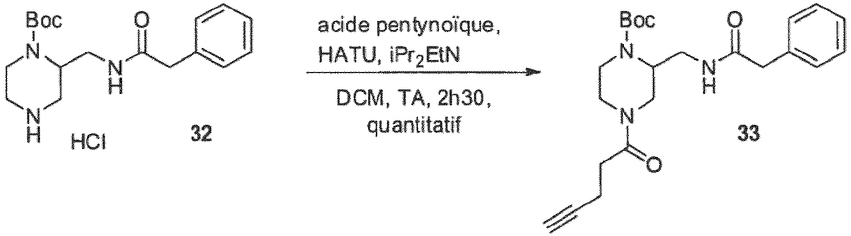

A une suspension refroidie à 0°C du composé **32** (410mg, 1,11mmol, 1,0eq.), d'acide pentynoïque (122mg, 1,22mmol, 1,1eq.) et de HATU (473mg, 1,22mmol, 1,1eq.) dans du DCM anhydre (30mL) sous atmosphère inerte d'argon, est additionnée la DIPEA (395µL , 2,25mmol, 2eq.) et le mélange réactionnel est agité à 0°C pendant 15min, puis à TA pendant 2h15. A la fin de la réaction, le mélange réactionnel est dilué avec du DCM (20mL) et lavé deux fois avec une solution aqueuse saturée de NaHCOs (2x50mL) et une fois avec de la saumure (50mL). La phase organique est séchée avec du Na₂S0₄, filtrée et évaporée. Le brut réactionnel est purifié par colonne de chromatographie sur alumine neutre (éther de pétrole : acétate d'éthyle / 15:85 / v:v) pour obtenir le composé **33** sous forme d'un solide légèrement jaune (552mg, rendement: quantitatif).

¹H-RMN (300 MHz, CDCl₃) δ = 7.43 - 7.15 (m, 5H), 6.25 (s, 1H), 4.47 - 4.15 (m, 2H), 4.00 - 3.57 (m, 2.7H), 3.52 (d, J = 2.9 Hz, 2H), 3.33 (m, 0.7H), 3.28 - 2.85 (m, 2.8H), 2.68 - 2.29 (m, 4.5H), 2.00 (d, .7 = 8.4 Hz, 1H), 1.47 (s, 9H) ppm.

¹³C-RMN (75 MHz, CDCl₃) δ = 171.53, 171.33, 170.51, 170.26, 165.70, 154.91, 134.84, 129.22, 128.92, 128.78, 127.26, 127.09, 83.36, 83.06, 80.81, 69.19, 68.90, 53.49, 49.97, 45.37, 43.60, 42.10, 41.32, 31.77, 31.69, 28.29, 14.55, 14.29 ppm.
MS: ESI: [M+H]⁺ m/z trouvée 413.3, calculée 413.2
Rf = 0.29 (éther de pétrole : acétate d'éthyle/ 2:8 / v:v)

### c) Préparation du composé 34, N- N-((4-(pent-4-ynoyl)pipérazin-2-yl)méthyl)-2-phénylacétamide sous forme de sel avec l'acide trifluoroacétique

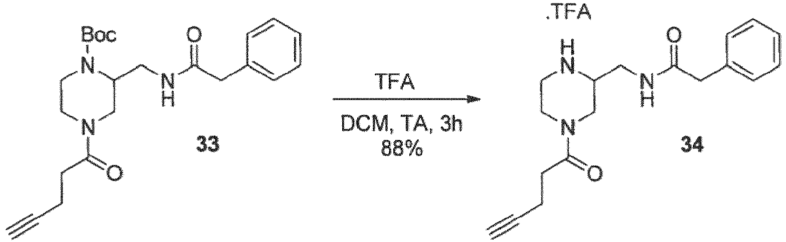

La procédure utilisée est la même que celle décrite pour la synthèse du composé 8, mais en utilisant le composé **33** (550mg, 1 ,33mmol, 1,0eq.) dans du DCM (5mL) et du TFA (5mL) pour donner le composé **34** sous forme d'une poudre blanche (500mg, 1,17mmol rendement: 88%).

¹H-RMN (500 MHz, MeOD) δ = 7.33 - 7.22 (m, 4H), 7.22 - 7.15 (m, 1H), 4.45 (t, J= 13.4 Hz, 1H), 3.97 (t, J = 13.4 Hz, 1H), 3.45 - 3.11 (m, 6.5H), 3.06 (t, J= 10.6 Hz, 0.5H), 3.01 - 2.81 (m, 2H), 2.67 - 2.46 (m, 2H), 2.42 (m, 2H), 2.24 (d, 7= 1.6 Hz, 1H), 1.34 - 1.26 (m, 1H) ppm.

¹³C-RMN (125 MHz, MeOD) δ = 175.33, 172.22, 172.09, 163.50, 163.23, 162.96, 162.68, 136.48, 136.39, 130.30, 130.27, 130.17, 129.70, 129.65, 128.09, 128.03, 83.85, 70.32, 70.26, 56.37, 56.22, 55.75, 54.81, 46.53, 44.58, 44.48, 43.72, 43.66, 43.55, 43.26, 42.64, 40.34, 40.19, 40.08, 39.63, 39.42, 32.73, 32.59, 18.65, 15.11, 13.09 ppm.

MS: ESI: [M+H]⁺ m/z trouvée 313.2, calculée 313.2

### XII - Préparation du composé 36, 4-nitrophényl 4-(3-(1-(2-(2-(2-méthoxyéthoxy)éthoxy)éthyl)-1H-1,2,3-triazol-4-yl)propanoyl)1 -2-((2-phénylacétamido)méhtyl)pipérazine-1-carboxylate (EXEMPLE 5)

### a) Préparation du composé 35, 4-nitrophényl 4-(pent-4-ynoyl)-2-((2-phénylacétamido)méthyl)pipérazine-1-carboxylate

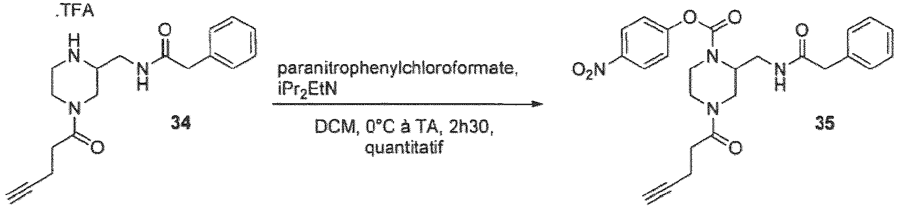

A une solution refroidie à 0°C et sous atmosphère inerte d'argon du composé **34** (50mg, 0,117mmol, 1,0eq.) et de DIPEA (55µL, 0,30mmol, 2,5eq.) dans du DCM anhydre (2mL) est ajouté le para- nitrophénolchlorofomiate (28mg, 0,130mmol, 1,1eq.) en une fois. Le mélange réactionnel est agité à 0°C pendant 30min puis à TA pendant 2h. A la fin de la réaction, le mélange réactionnel est dilué avec du DCM (20mL) et lavé deux fois avec une solution aqueuse saturée de NaHCOs (2x25mL) et une fois avec de la saumure (25mL). La phase organique est séchée avec du Na₂SO₄, filtrée et évaporée. Le brut réactionnel est purifié par colonne de chromatographie sur gel de silice (éther de pétrole : acétate d'éthyle / 2:8 / v:v) pour obtenir le composé **35** sous forme d'un solide incolore (60mg, rendement: quantitatif).

¹H-RMN (500 MHz, CDCl₃) δ= 8.15 (t, 7 = 9.8 Hz, 2H), 7.33 - 7.01 (m, 7H), 6.23 (s, 0.4H), 6.08 - 5.82 (m, 0.6H), 4.60 - 4.36 (m, 1.5H), 4.32 - 4.17 (m, 0.5H), 4.05 - 3.88 (m, 1.4H), 3.84 (m, 0.8H), 3.77 - 3.62 (m, 0.8H), 3.62 - 3.16 (m, 4.5H), 3.12 (t, J = 12.0 Hz, 0.5H), 2.96 - 2.65 (m, 1.8H), 2.65 - 2.26 (m, 4.2H), 2.08 - 1.86 (m, 1.5H), 1.29 - 1.13 (m, 0.5H) ppm.

¹³C-RMN (125 MHz, CDCl₃) δ = 171.59, 171.35, 170.80, 170.40, 170.28, 155.85, 152.84, 152.40, 145.05, 144.94, 134.69, 134.48, 129.35, 129.25,129.08, 128.96, 127.50, 127.33, 125.17, 122.27, 122.13, 121.85, 83.28, 83.03, 69.37, 69.10, 53.54, 51.50, 51.25, 45.58, 45.23, 45.03, 43.68, 42.43, 42.09, 41.20, 40.59, 40.18, 38.70, 38.10, 37.48, 31.86, 31.75, 14.71, 14.40 ppm.
MS: ESI: [M+H]⁺ m/z trouvée 478.3, calculée 478.3
Rf = 0.32 (éther de pétrole : acétate d'éthyle/ 2:8 / v:v)

### b) Préparation du composé 36, 4-nitrophényl 4-(3-(1-(2-(2-(2-méthoxyéthoxy)éthoxy)éthyl)-1H-1,2,3-triazol-4-yl)propanoyl)1-2-((2-phénylacétamido)méhtyl)pipérazine-1-carboxylate

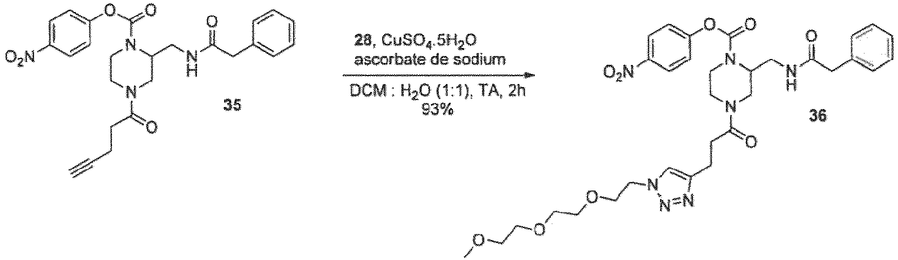

La procédure utilisée est la même que celle décrite pour la synthèse du composé **31,** avec le composé **35** (30mg, 0,063mmol, 1,0eq.), le composé 28 (26mg, 0,138mmol, 2,2eq.), CuSO₄.5H₂O (9mg, 0,04mmol, 0,6eq.) et l'ascorbate de sodium (16mg, 0,08mmol, 1,2eq.) dans un mélange DCM (1mL) et eau (1mL). Après purification, on obtient le composé **36** sous forme d'une huile légèrement jaune (39mg, 0,058mmol, rendement : 93%).

¹H-RMN (500 MHz, CDCl₃) δ = 8.29 - 8.01 (m, 2H), 7.54 - 7.01 (m, 7H), 4.56 - 4.24 (m, 5H), 4.05 - 3.66 (m, 5H), 3.66 - 3.37 (m, 12H), 3.31 (d, J= 4.7 Hz, 3H), 3.25 - 2.55 (m, 7H), 2.18 - 1.84 (m, 1H) ppm.

¹³C-RMN (125 MHz, CDCl₃) δ = 172.78, 171.62, 156.11, 155.95, 152.46, 145.10, 144.97, 135.45, 134.92, 129.38, 128.97, 128.80, 127.31, 127.10, 125.18, 122.21, 72.00, 70.64, 70.60, 69.43, 59.14, 51.47, 51.26, 50.69, 50.44, 50.18, 45.45, 45.20, 44.63, 44.03, 43.80, 42.40, 42.09, 41.06, 40.63, 40.25, 40.10, 39.39, 38.05, 37.72, 37.48, 31.85, 31.39, 30.40, 29.77, 29.51, 28.59, 26.99, 21.36, 20.53 ppm.
MS: ESI: [M+H]⁺ m/z trouvée 667.3, calculée 667.3
Rf = 0.31 (DCM : MeOH/ 95:5 / v:v)

### XIII - Préparation du composé 39, 4-chloro-2-(6-chloro-4-oxo-3,4-dihydroquinazolin-2-yl)phényl 4-(3-(1-(2-(2-(2-méthoxyéthoxy)éthoxy)éthyl)-1H-1,2,3-triazol-4-yl)propanoyl)1-2-((2-phénylacétamido)méhtyl)pipérazine-1-carboxylate (EXEMPLE 6)

### a) Préparation du composé 38, 4-chloro-2-(6-chloro-4-oxo-3,4-dihydroquinazolin-2-yl)phényl 4-(pent-4-ynoyl)-2-((2-phénylcétamido)méthyl)pipérazine1-carboxylate

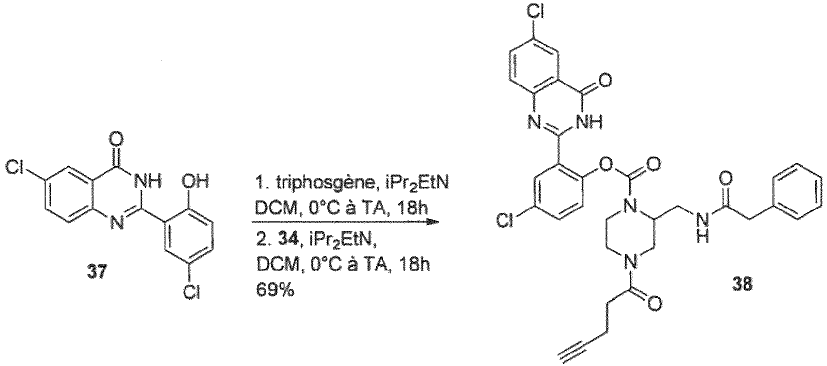

A une suspension refroidie à 0°C et sous une atmosphère inerte d'argon du composé **37** (40mg, 0,13mmol, 1,1eq) dans du DCM anhydre (5mL) est ajoutée une solution de triphosgène (130mg, 0,43mmol, 3,3eq.) dans du DCM anhydre (2mL) suivie par l'ajout de DIPEA (80µL , 0,43mmol, 3,3eq.). Ce mélange réactionnel est agité à 0°C pendant 30min puis à TA pendant 18h. Les volatiles sont alors enlevés par évaporation sous pression réduite et le solide résultant est repris dans du DCM anhydre (5mL). A cette nouvelle suspension est ajoutée une solution du composé **34** (50mg, 0,1 17mmol, 1,0eq.) dans du DCM anhydre (2mL), suivi par de la DIPEA (85µL , 0,47mmol, 4,0eq.)., et ce mélange réactionnel est agité à TA pendant 18h. A la fin de la réaction, le mélange réactionnel est dilué avec du DCM (20mL) et lavé deux fois avec une solution aqueuse saturée de NaHC0₃ (2x25mL) et une fois avec de la saumure (25mL). La phase organique est séchée avec du Na₂SO₄, filtrée et évaporée. Le brut réactionnel est purifié par deux colonnes de chromatographie sur gel de silice successives (éther de pétrole : acétate d'éthyle / 2:8 / v:v ; et gradient DCM : MeOH / 99:1, 98:2, 97:3 / v:v) pour obtenir le composé 38 sous forme d'un solide blanc (52mg, 0,080mmol, rendement: 69%).

¹H-RMN (500 MHz, CDCl₃) δ= 11.12 - 10.16 (m, 0.8H), 8.14 (S, 0.7H), 8.01 - 7.70 (m, 0.9H), 7.64 (s, 1.5H), 7.41 (s, 0.9H), 7.30 - 6.88 (m, 5.3H), 6.82 - 6.46 (m, 0.9H), 6.10 (s, 0.3H), 4.56 - 4.01 (m, 2H), 4.05 - 2.99 (m, 7.1H), 2.99 - 2.60 (m, 1.8H), 2.60 - 2.15 (m, 3.6H), 2.10 - 1.59 (m, 1.6H), 1.23 (m, 1.3H) ppm.

¹³C-RMN (125 MHz, CDCl₃) δ = 173.05, 171.80, 170.88, 170.50, 170.31, 161.41, 153.40, 153.05, 152.46, 149.57, 149.45, 147.43, 147.20, 147.09, 135.64, 135.41, 135.19, 134.83, 134.72, 134.55, 133.55, 133.39, 133.14, 132.11, 131.72, 130.88, 130.52, 130.29, 129.61, 129.43, 129.27, 129.20, 129.05, 128.85, 127.99, 127.66, 127.45, 127.20, 127.16, 125.96, 125.80, 124.81, 124.35, 124.16, 122.27, 122.06, 121.85, 83.37, 83.08, 82.95, 69.53, 69.39, 69.06, 68.97, 51.86, 51.67, 45.26, 45.09, 44.96, 43.58, 43.38, 42.44, 41.78, 41.11, 40.58, 40.37, 39.74, 39.33, 38.86, 38.41, 38.02, 31.76, 29.72, 14.68, 14.33 ppm.
MS: ESI: [M+H]⁺ m/z trouvée 645.3, calculée 645.2
Rf = 0.19 (DCM : MeOH/ 97:3 /v:v)

### b) Préparation du composé 39, 4-chloro-2-(6-chloro-4-oxo-3,4-dihydroquinazolin-2-yl)phényl 4-(3-(1-(2-(2-(2-méthoxyéthoxy)éthoxy)éthyl)-1H-1,2,3-triazol-4-yl)propanoyl)1-2-((2-phénylacétamido)méhtyl)pipérazine-1-carboxylate

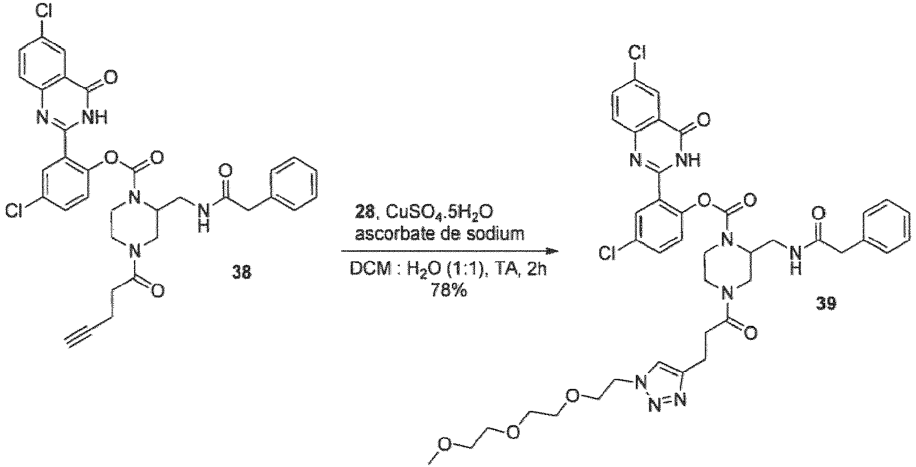

La procédure utilisée est la même que celle décrite pour la synthèse du composé 31, avec le composé **38** (50mg, 0,077mmol, 1,0eq.), le composé **28** (31mg, 0,170mmol, 2,2eq.), CuSO₄.5H₂O (12mg, 0,05mmol, 0,6eq.) et l'ascorbate de sodium (20mg, 0,10mmol, 1,2eq.) dans un mélange DCM (2m L) et eau (2mL). Après purification, on obtient le composé 39 sous forme d'une huile légèrement jaune (50mg, 0,060mmol, rendement : 78%).

¹H-RMN (500 MHz, CDCl₃) δ = 11.12 (s, 0.4H), 10.89 (s, 0.2H), 10.14 - 9.90 (m, 0.2H), 8.33 - 7.94 (m, 2H), 7.91 - 7.71 (m, 2H), 7.70 - 7.48 (m, 1.5H), 7.43 - 7.32 (m, 2H), 7.30 - 7.12 (m, 3.5H), 4.54 (m, 4H), 3.93 (m, 4H), 3.77 - 3.50 (m, 10H), 3.45 - 2.72 (m, 10H), 2.39 (s, 0.5H), 1.66 (s, 3H) ppm.

¹³C-RMN (125 MHz, CDCl₃) δ = 174.00, 173.14, 172.10, 171.24, 161.27, 152.45, 152.24, 149.84, 149.71, 147.59, 147.54, 147.30, 147.19, 135.53, 135.30, 135.16, 135.11, 134.89, 133.08, 133.02, 132.14, 132.09, 131.98, 131.67, 131.54, 131.04, 129.79, 129.44, 129.35, 128.84, 128.78, 128.68, 127.64, 127.19, 126.97, 126.04, 125.83, 125.11, 124.32, 124.22, 122.54, 72.01, 70.64, 70.59, 70.43, 69.27, 59.15, 52.06, 51.86, 51.50, 50.90, 50.58, 45.62, 45.47, 45.38, 45.17, 44,54, 44.01, 43.88, 43.72, 43.53, 41.87, 41.17, 40.84, 40.42, 40.00, 39.20, 39.11, 38.30, 38.28, 30.34, 29.70, 29.43, 21.39, 20.64 ppm.
MS: ESI: [M+H]⁺ m/z trouvée 834.5, calculée 834.3
Rf = 0.13 (DCM : MeOH/ 96:4 / v:v)

### B - Evaluation de l'hydrosolubilité des composés :

L'hydrosolubilité des composés a été évaluée en fonction de la présence ou de l'absence de précipité dans des solutions tamponnées des composés en question à différentes concentrations. Ces solutions ont été préparées comme suit :
Une quantité connue de produit est dissoute dans un volume de DMSO approprié afin d'obtenir une solution mère limpide à 100mM dans le DMSO (sauf dans le cas de l'AMC-Leucine commerciale (notée AMC-Leu) qui a donné une solution trouble dans ces conditions et la concentration de la solution mère dans le DMSO a dû être abaissée à 50mM). Ces solution mères sont ensuite diluées dans un tampon PBS commercial afin d'obtenir un éventail de concentrations finales comprises entre 1mM et 50µM. Un composé est déclaré insoluble si un précipité apparaît et persiste après dilution.

Le **Tableau 2** ci-dessous présente les résultats de ces tests de solubilité.

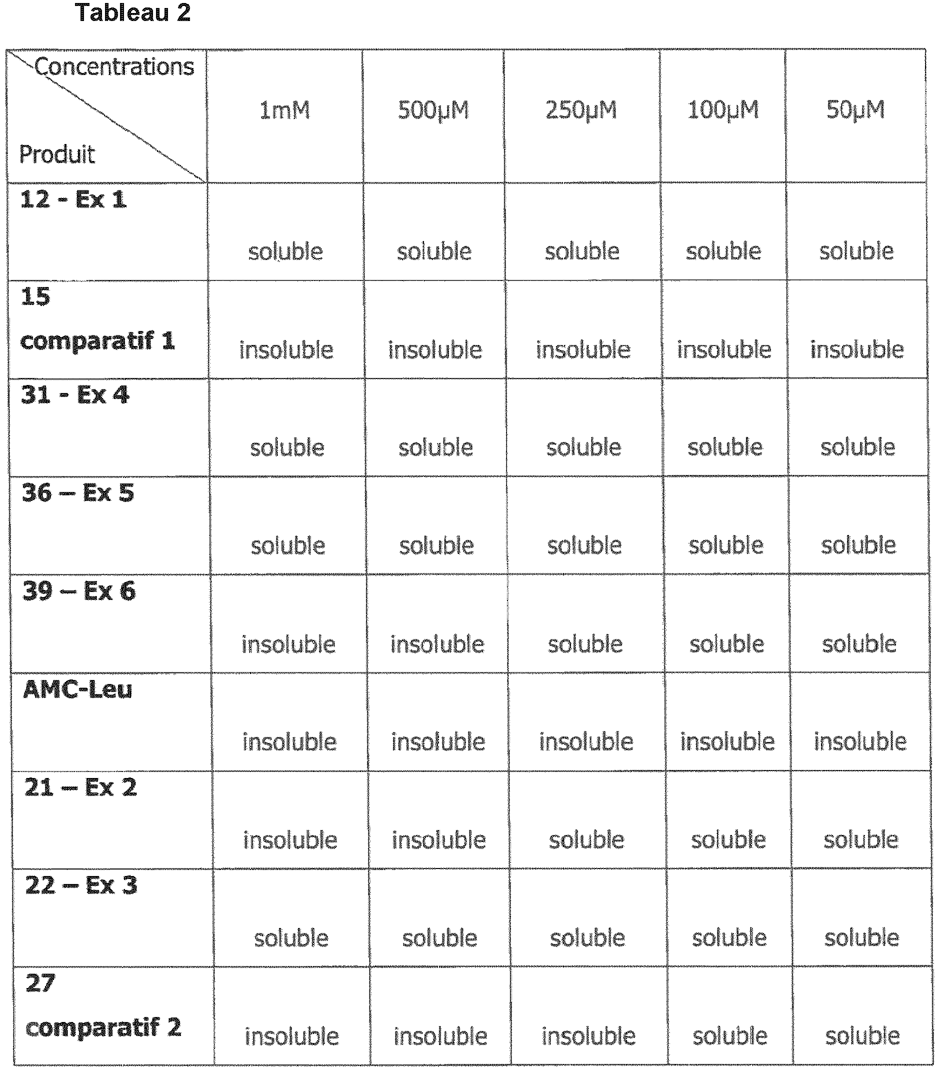

Conclusions : Dans tous les cas présentés, les composés selon l'invention présentent une meilleure solubilité en solution aqueuse, par rapport à un substrat commercial sans bras espaceur, ou par rapport à un substrat incorporant un bras espaceur décrit dans les demandes WO 2013/045854 et WO 2014/020285.

### Activation enzymatique :

Certains des composés ont été testés pour évaluer la fluorescence détectée sous activité enzymatique.

A une solution de sonde selon l'invention dans du PBS dans une plaque 96-puits (noire pour la fluorescence ou transparente pour l'absorbance) est ajoutée une solution de PGA (Penicillin G Amidase de Escherichia Coll, Waterstone Tech.) dans du tampon PBS ou une solution de Upase (Lipase de Candida Rugosa, Sigma-Aldrich) dans du tampon PBS.
Concentration finale de sonde: comprise entre 10µM et 50µM
Concentration finale de PGA: 5U
Ou concentration finale de lipase : 4U

La plaque est ensuite incubée à 37 °C et la fluorescence ou l'absorbance est enregistrée au cours du temps avec un lecteur de plaque multi-puits (EnSpire, Perkin Elmer). Les courbes résultantes sont le résultat de triplicats.

Pour la 4-méthylumbéliférone (composés **12, 15, 31, 21 et 27**) : Fluorescence : λₑₓ - 370 nm, λₑₘ= 445 nm.

Pour la 6-chloro-2-(5-chloro-2-hydroxyphenyl)quinazolin-4(3H)-one
(composé **39**): Fluorescence : λₑₓ = 365 nm, λₑₘ= 530 nm.

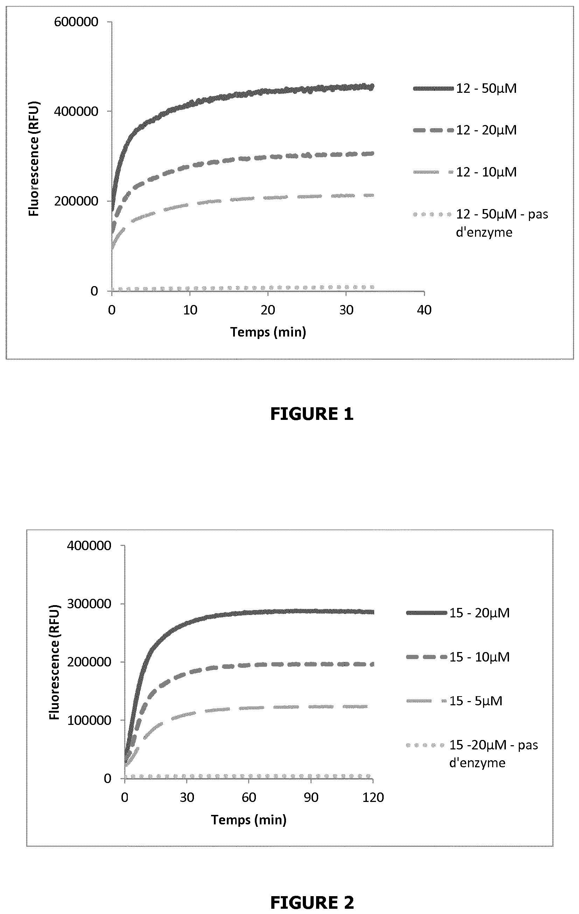

Pour la paranitrophénolate (composé **36**): Absorbance : λ_{abs} = 405 nm. Les résultats sont présentés sur les **FIGURES 1 à 8** annexées.

L'évolution de la fluorescence suite à l'activation du composé **12** par la PGA est présentée **FIGURE 1****.**

L'évolution de la fluorescence suite à l'activation du composé **15** par la PGA est présentée **FIGURE 2****.**

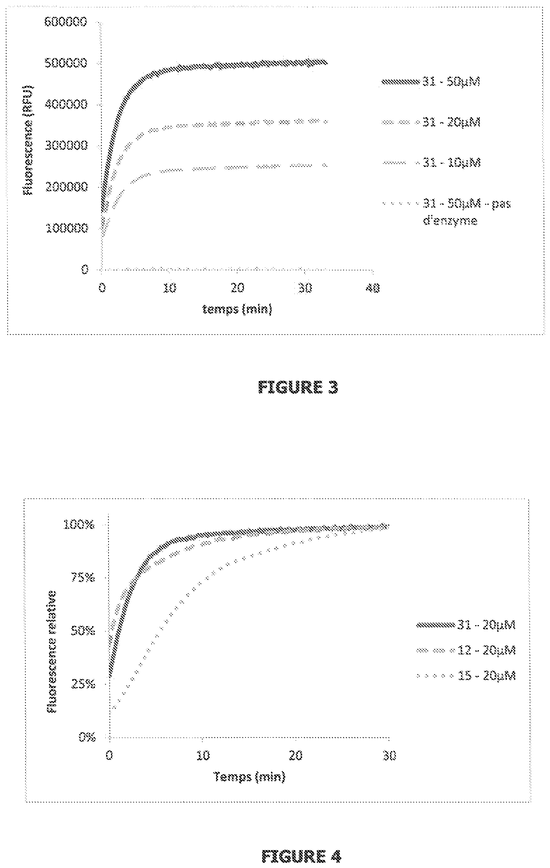

L'évolution de la fluorescence suite à l'activation du composé **31** par la PGA est présentée **FIGURE 3****.**

La comparaison des vitesses de réponse des composés **12** et **31** selon l'invention, et du composé **15** utilisé en tant que comparatif, suite à leur activation par la PGA est présentée en **FIGURE 4****.**

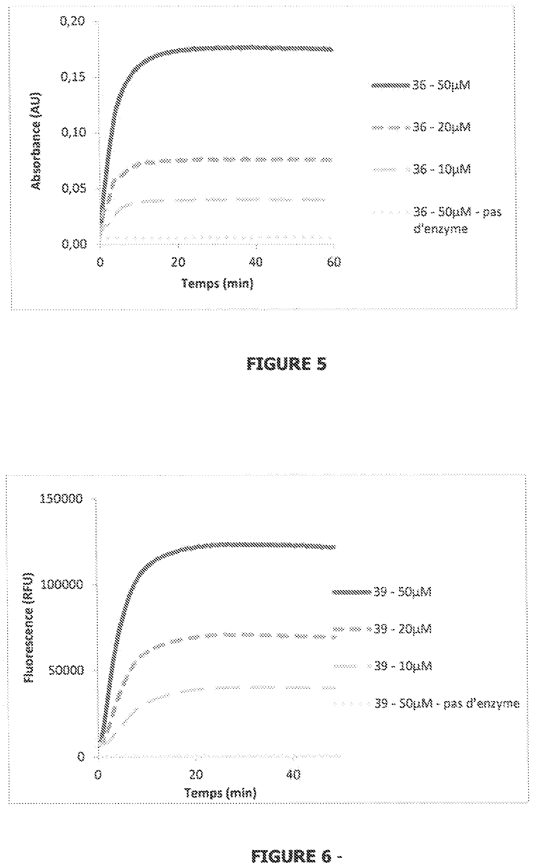

L'évolution de l'absorbance suite à l'activation du composé **36** par la PGA est présentée **FIGURE 5****.**

L'évolution de la fluorescence suite à l'activation du composé **39** par la PGA est présentée **FIGURE 6****.**

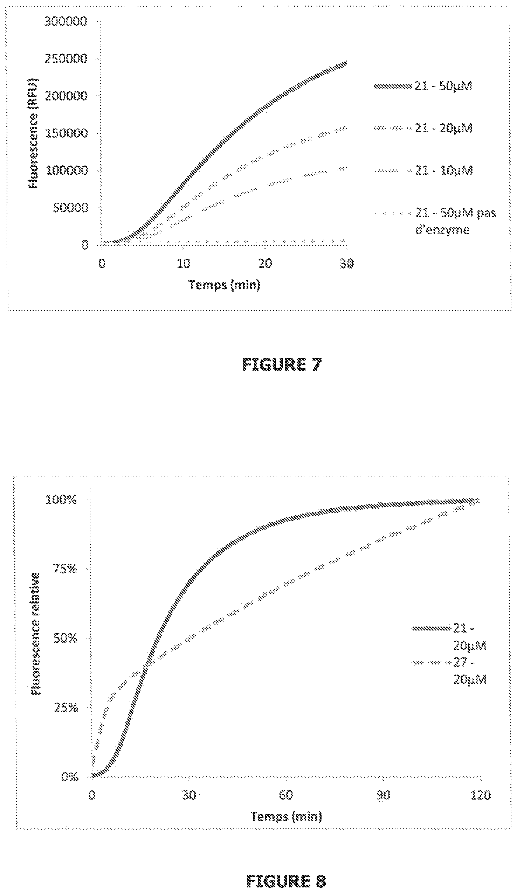

L'évolution de la fluorescence suite à l'activation du composé **21** par la lipase est présentée **FIGURE 7****.**

La comparaison des vitesses de réponse du composé **21** selon l'invention, et du composé **27** utilisé en tant que comparatif, suite à leur activation par la Lipase est présentée en **FIGURE 8****.**

Conclusions : les composés selon l'invention permettent effectivement de détecter la présence d'une activité enzymatique grâce à des mesures de fluorescence ou d'absorbance. La reconnaissance enzymatique est toujours bonne malgré (Incorporation d'une charge ou d'un groupement relativement encombrant d'un point de vue stérique sur le bras espaceur.

## Revendications

1. - Sondes de formule (I) : 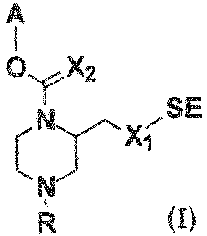 dans laquelle :
- soit X₁ est un oxygène et SE est un substrat d'estérase, glycosidase, phosphatase, sulfatase, ou glucuronidase,
- soitXi représente NH et SE est un substrat de protéase, notamment d'amidase, de peptidase ou transférase,
- X₂ = O ou S,
- A est un groupe aromatique, qui après clivage en solution aqueuse de la liaison C(X₂)-0, conduit à la libération d'un chromophore ou d'un fluorophore, ledit groupe aromatique étant un groupe comprenant un ou plusieurs cycles aromatiques substitués ou non substitués, lesdits cycles pouvant comprendre un ou plusieurs hétéroatomes choisis parmi les atomes d'azote, d'oxygène ou de soufre et/ou un ou plusieurs atomes de carbone sous la forme d'un carbonyle C=O,
- R représente un atome d'hydrogène ou -(L)n-GP, avec n qui est égal à 0 ou 1,
- L est un bras de liaison,
- GP est un groupe hydrosolubilisant qui est une fonction F₁ choisie parmi les aminés primaires, tertiaires et secondaires, amidine, la guanidine, le tétrazole ; une fonction F₂ anionique du type, carboxylate, sulfonate ou phosphate ; un groupement comprenant une ou plusieurs de ces fonctions F₁ et/ou F₂ ; un polyéthylèneglycol ; un sucre ou polysaccharide tel que le glucose, le galactose et le mannose ; un groupe peptidique tel que la poly-lysine, la poly-arginine et les TAT-peptides,
ainsi que leurs sels physiologiquement acceptables,
ledit groupe aromatique A étant choisi parmi le paranitrophénol et ses dérivés, les indigoïdes, la cyclohexenoescutetine, l'alizarin et l'hydroxyflavone,
ou ledit groupe A étant un composé aromatique avec -OA choisi parmi 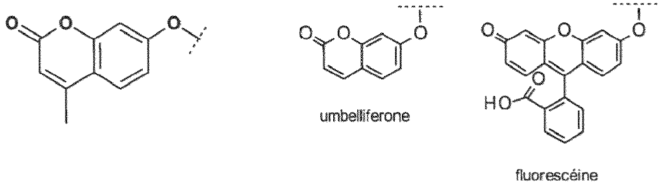 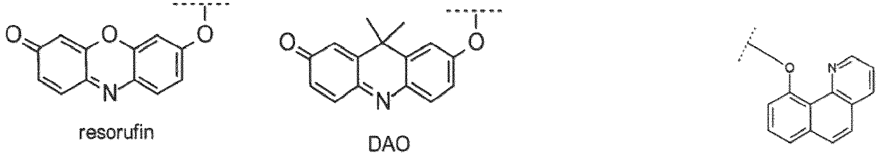 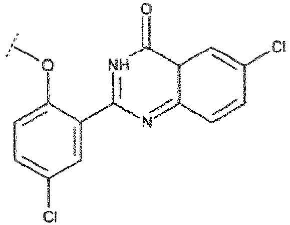 , ou,
ou ledit groupe A étant un composé aromatique avec -OA qui répond à la formule (AA): 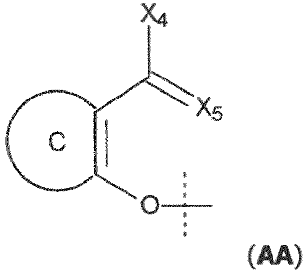
dans laquelle :
soit X₅ est un atome d'oxygène et X₄ est un groupe -NH₂, -OH, -SH, alkyle, aryle, -O-alkyle , -O-phényle, -NH-alkyle, -NH-phényle, -S-alkyle ou -S-aryle, lesdits groupes alkyle et phényle pouvant être substitués ou non substitués,
soit X₅ représente un atome d'azote et est lié à X₄ qui représente alors CH, O, S, N ou NH pour former un hétéroaryle substitué ou non substitué,
et 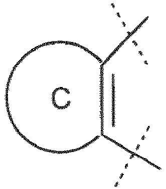 représente un aryle ou un hétéroaryle, substitué ou non substitué, par exemple choisi parmi les groupes phényle, naphthyle, et 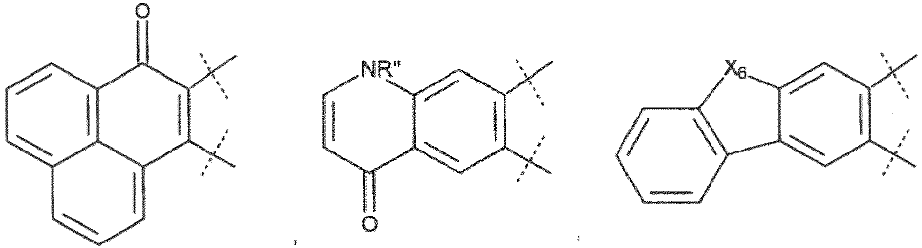
lesdits groupes pouvant être substitués ou non substitués,
avec X₆ qui représente S, O ou NR", et R" qui représente un atome d'hydrogène ou un groupe (C₁-C₄)alkyle.

2. - Sondes selon la revendication 1 **caractérisées en ce que** SE est choisi parmi les groupes glycosyle liés par leur carbone anomérique au reste de la molécule ou SE est un substrat de protéase ou peptidase et correspond à un groupe peptidyl ou aminoacide lié au reste de la molécule par une fonction acyle portée par leur carbone terminal ou par un chaînon latéral.

3. - Sondes selon l'une quelconque des revendications 1 à 2 **caractérisées en ce que** SE est un substrat de glycosidase par exemple de la N-acétyl-β-galactosaminidase ; la N-acétyl-p-glucosaminidase ; la α-amylase ; la α-arabinofuranosidase ; la α-arabinosidase ; la β-cellobiosidase ; la β-chitobiosidase ; la α-galactosidase ; la β-galactosidase ; la α-glucosidase ; la β-glucosidase ; la β-glucuronidase ; la α-maltosidase ; la α-mannosidase ; la β-mannosidase ; la β-xylosidase ; la β-D-fucosidase ; la α-L-fucosidase ; la β-L-fucosidase ; la L-iduronidase ou de la cellulase ; et correspond à groupement glycosyle lié par son carbone anomérique au reste de la molécule, et notamment, SE est un groupement mono-glycosylé choisi parmi le galactosyle, le glucosyle, le mannosyte, le gulosyle, l'altosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle ou un groupement polyglycosylé constitué de plusieurs, par exemple de 2 à 20, de préférence de 3 à 10, et plus particulièrement de 4 à 6, de ces groupements monoglycosylés identiques ou différents.

4. - Sondes selon l'une quelconque des revendications 1 à 2 **caractérisées en ce que** SE est un substrat de de galactosidase, par exemple de la β-galactosidase, de l'induronidase, de la glucosidase, de la N-acétyl-D-glucosaminidase, de la N-acétyl-D-galactosaminidase, de la mannosidase, de la fucosidase ou de la glucuronidase, notamment de la β -glucuronidase ; et correspond à groupement glycosyle lié par son carbone anomérique au reste de la molécule, et notamment, SE est un groupement mono-glycosylé choisi parmi le D-glucuronyle, le L-iduronyle, le D-glucopyranosyle, le D-galactopyranosyle, le N-acétyl-D-glucosaminyle, le N-acétyl-D-galactosaminyle, le D-mannopyranosyle, le L-fucopyranosyle ou un groupement polyglycosylé constitué de plusieurs, par exemple de 2 à 20, de préférence de 3 à 10, et plus particulièrement de 4 à 6, de ces groupements monoglycosylés identiques ou différents.

5. - Sondes selon l'une quelconque des revendications 1 à 2 **caractérisées en ce que** SE est :
- soit un substrat d'une protéase ou d'une peptidase, qui est un substrat de cathepsine ou de métalloprotéase, notamment de la PSMA (Prostate Specific Membrane Antigen) ; et SE correspond à un groupe peptidyle ou aminoacide lié au reste de la molécule par une fonction acyle portée par son carbone terminal ou par un chaînon latéral ;
- soit un substrat d'une transférase, notamment un substrat de la glutathione transférase ou de la gamma-glutamyl transférase, et SE correspond à un groupe peptidyle ou aminoacide lié au reste de la molécule par une fonction acyle portée par son carbone terminal ou par un chaînon latéral ou SE correspond à un groupe sulfonyle formant une sulfonamide avec X₁ qui représente alors NH.

6. - Sondes selon l'une quelconque des revendications 1 à 5 **caractérisées en ce que** A est un groupe aromatique avec -OA qui est du type phénoxy, et correspond, aux structures préférées (BB) ou (CC) suivantes : 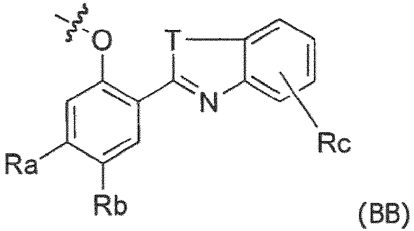 dans laquelle
- T est -NH-C(O)-, -O-, -NH, N-alkyle ou N-aryle,
- Ra est l'hydrogène ou un substituant carboné attracteur d'électrons, comme -CN ou - COORd, avec Rd qui représente un groupe (C₁-C₄)alkyle, ou bien Ra est -CONReRf, avec Re et Rf, identiques ou différents, qui représentent l'hydrogène ou un groupe (C₁-C₄)alkyle, ou bien Ra est -CF₃ ou un groupe 2-oxazolyle, 2-thiazolyle, 2-imidazolyel, 2-benzoimidazolyle, 4-pyrimidinon-2-yle ou quinazolinon-2-yle,
- Rb est l'hydrogène, un atome de chlore, brome, iode ou fluor, -OH, -NH₂, -NRgRh, - NHRg ou -ORg, avec Rg et Rh qui représentent chacun indépendamment un (C₁-C₄)alkyle,
- ou bien Ra et Rb sont liés entre eux pour former une chaîne hydrocarbonée comprenant 4 ou 5 chaînons, saturée ou Insaturée, substituée ou non substituée, éventuellement interrompue par un ou plusieurs hétéroatomes choisis parmi N, S et O,
- Rc est l'hydrogène, Br, Q, I ou F, 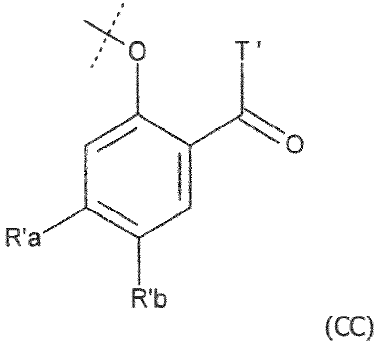
dans laquelle :
- T' est NH₂, OH, un groupe aryle, un groupe (C₁-C₄)alkyle, SH, NHR'c, OR'c, NR'cR'd ou SR'c, avec R'c et R'd, identiques ou différents, qui représentent un groupe (C₁-C₄)alkyle ou aryle,
- R'a est l'hydrogène ou un substituant carboné attracteur d'électrons comme -CN, ou
- COOR'e, avec R'e qui représente un groupe (C₁-C₄)alkyle, ou R'a est -CONRfR'g, avec R'f et R'g, Identiques ou différents, qui représentent l'hydrogène ou un groupe (C₁-C₄)alkyle, ou bien R'a est -CF₃ ou un 2-oxazolyle, 2-thiazolyle, 2-imidazolyle, 2-benzoimidazolyle, 4-pyrimidinon-2-yle ou quinazolinon-2-yle,
- R'b est l'hydrogène, un atome de chlore, brome, iode ou fluor, -OH, -NH₂, -NR'hR'i ou
- OR'h, avec R'h et R'i, identiques ou différents, qui représentent un groupe (C₁-C₄)alkyle,
- ou bien R'a et R'b sont liés entre eux pour former une chaîne hydrocarbonée comprenant 4 ou 5 chaînons, saturée insaturée, substituée ou non substituée, éventuellement interrompue par un ou plusieurs hétéroatomes choisis parmi N, S et O.

7. - Sondes selon l'une quelconque des revendications 1 à 6 **caractérisées en ce que** R représente -(L)n-GP avec n = 1 et L est un bras de liaison, et notamment un bras -(L1)m1-(L2)m2-(L'1)m'1-, défini dans le sens pipérazine -> groupe GP avec :
- L1 et L'1, identiques ou différents, qui sont choisis parmi -O-, -NH, -N(C₁₋₆ alkyl)-, - N(phényl)-, -N(aryl)-, C(O)-, -C(O)O, -OC(O)- -OC(O)-O-, -NHC(O)-O- -OC(O)-NH-, - NHC(O)-NH- -S-, -SO₂, -N=N-, -NHC(O)- et-CONH- ;
- L2 qui est choisi parmi les groupes bivalents suivants : (C₁₋₂₀)alkyle, (C₁-₂₀)alcényle, (C₁₋₂₀)alcynyle, (C₆₋₂₄)aryle, (C₇₋₄₄)alkylaryle, (C₇₋₄₄)alcénylaryle, (C₇₋₄₄)alcynylaryle, (C₇₋₄₄)alkylcycloalkyle, (C₇₋₄₄)alcénylcycloalkyle, (C₇₋₄₄)alcynylcycloalkyle, (C₇₋₄₄)alkylhétérocycloallcyle, (C₇₋₄₄)alcénylhétérocycloalkyle, (C₇₋₄₄)alcynylhétérocycloalkyle ; lesdits groupes pouvant être interrompus ou se terminer par un groupe triazole et pouvant être non substitués ou substitués, notamment par un ou plusieurs substituants choisis parmi les (C₁₋₁₀)alcoxy, (C₁₋₁₀)alkyle, (C₆₋₁₀)aryle, amido, imido, phosphido, nitrido, (C₁₋₁₀)alcényle, (C₁₋₁₀)alcynyle et -OH ;
- m1, m'1 et m2, identiques ou différents, qui sont égaux à 0 ou 1 et
- GP étant tel que défini à la revendication 1.

8. - Sondes selon la revendication 7 **caractérisées en ce que** L représente -(L1)m1-(L2)m2-(L '1)m'1 avec L1 = -C(O)-, m1 = m2 = 1, m'1 = 1 ou 0 et L2 et L'1 tels que définis à la revendication 14, et notamment L représente -C(O)-(CH₂)p-L3- avec p qui est égal à 1, 2, 3 ou 4 et L3 qui est un groupe triazole et notamment un groupe 1H- 1,2,3-triazole.

9. - Sondes selon l'une quelconque des revendications 1 à 8 **caractérisées en ce que** X₂ est un atome d'oxygène.

10. - Sondes selon l'une quelconque des revendications 1 à 9 **caractérisées en ce que** :
- X₂ est un atome d'oxygène,
- soit X₁ est un atome d'oxygène et SE est un substrat de glycosidase ou glucuronidase et correspond à un groupe glycosyle lié par son carbone anomérique au reste de la molécule ou SE est un substrat d'estérase et correspond à un groupe -C(O)Ri avec Ri qui représente, par exemple, un groupe alkyle de préférence de 1 à 20 atomes de carbone, un groupe alcényle de préférence de 1 à 20 atomes de carbone, un groupe benzyle, aryle ou hétéroaryle,
- soit X₁ représente NH et SE est un substrat de protéase ou peptidase et correspond à un groupe peptidyle ou aminoacide lié au reste de la molécule par une fonction acyle portée par son carbone termina! ou par un chaînon latéral; et
- R représente -L-GP avec :
• L qui représente -(L1)m1-(L2)m2-(L'1)m'1 avec L1 = - C(O)-, m1 = m2 = 1, m'1 = 1 ou 0 et L2 et L'1 tels que définis à la revendication 10, et notamment L représente - C(O)-(CH₂)p-L3- avec p qui est égal à 1, 2, 3 ou 4 et L3 qui est un groupe triazole et notamment un groupe 1H-1,2,3- triazole, et
• GP qui est tel que défini à la revendication 1.

11. - Sondes selon la revendication 1 choisies parmi :
hydrochlorure de 4-méthyl-2-oxo-2H-chromen-7-yl 2-((2- phénylacétamido)méthyl)pipérazine-I-carboxylate (Composé **12**) : 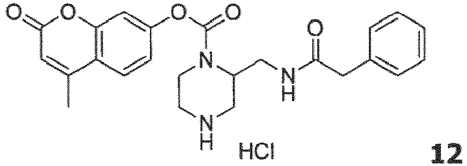
hydrochlorure de 4-methyl-2-oxo-2H-chromen-7-yl 2- ((octanoyloxy)méthyl)pipérazine- 1 - carboxylate (Composé **21**) 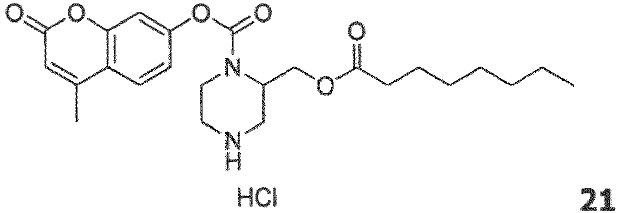
acide 4-4-(((4-méthyl-2-oxo-2H-chromen-7-yl)oxy)carbonyl)-3- ((octanoloxy)methyl)pipéra2in-1-yl)butane-1-sulfonique (Composé **22**) 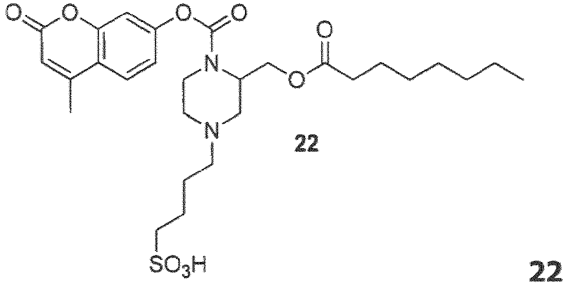
4-méthyl-2-oxo-2H-chromen-7-yl 4-(3-(1-(2-(2-(2- methoxyéthoxy)éthyl)-1H-1,2,3-triazol-4-yl)propanoyl)-2-((2- phenylacétamido)méthyl)pipérazine-1-carboxylate (Composé **31**) 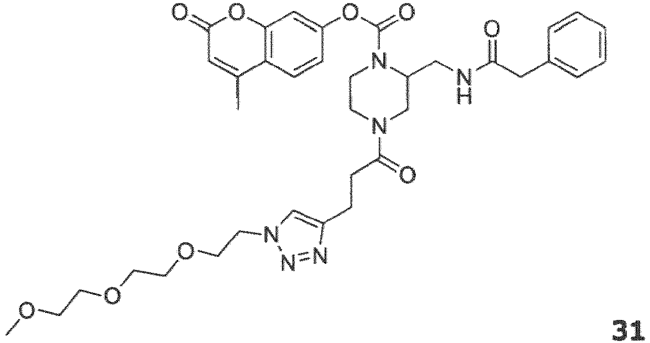
4-nitrophényl 4-(3-(1-(2-(2-(2-méthoxyéthoxy)éthoxy)éthyl)-1H-1,2,3-triazol- 4-yl)propanoyl)-2-((2-phénylacetamido)methyl)piperazine-1-carboxylate (Composé **36**) 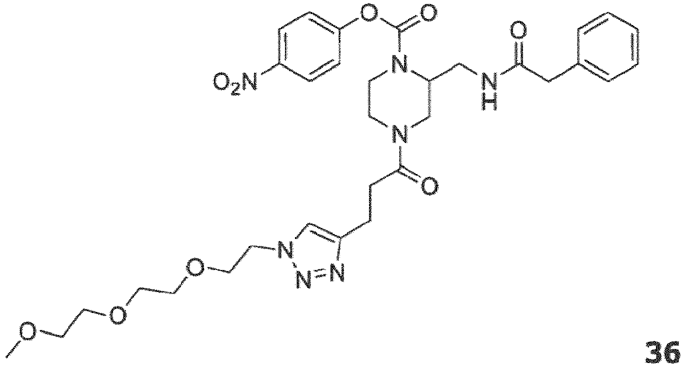
4-chloro-2-(6-chloro-4-oxo-3,4-dihydroquinazolin-2-yl)phényl 4-(3-(I-(2-(2- (2-méthoxyéthoxy)éthoxy)éthyl)-1H-1,2,3-triazol-4-yl)propanoyl)-2-((2-phénylacétamido)méthyl)pipérazine-1-carboxylate (Composé **39**)

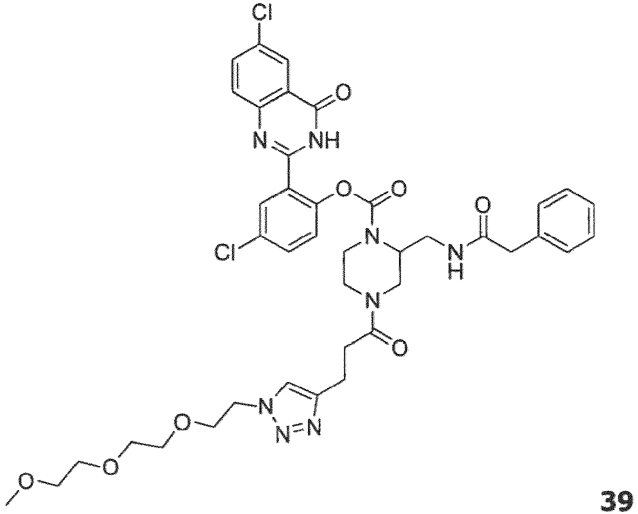

12. - Intermédiaires de formule : 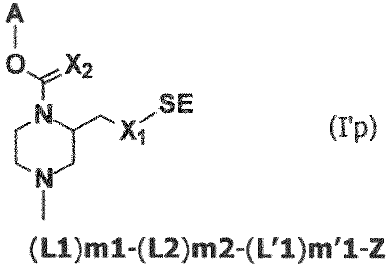 et 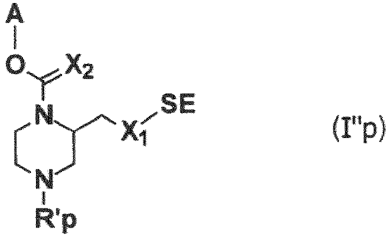 dans lesquelles :
A, X₁, X₂ SE, L1, L'1, L2, m1, m'1, et m2 sont tels que définis pour (I) aux revendications 1 à 10,
Z représente C=CH, N₃, une fonction N-oxysuccinimide ou maléimide, et
R'p représente un groupe protecteur des fonctions aminés, de préférence choisi parmi les groupes benzyle, tes groupes -C(O)OR', avec R'₁ qui représente un groupe alkyle ou alcényle de 1 à 12 atomes de carbone ou un groupe -(CH₂)ₘ₃R"₁ avec R", qui représente un groupe aryle, cycloalkyle ou fluorényle et m3 qui est égal à 0, 1, 2 ou 3, tel que les groupes carbobenzyloxy, ter-butyloxycarbonyl, 9-fluorénylmethyloxycarbonyl, et allyloxycarbonyle, ainsi que leurs sels, solvats ou hydrates.

13. - Sondes selon l'une quelconque des revendications 1 à 11, pour la détection *in vivo,* chez l'homme ou l'animal, d'une enzyme, dont la présence entraîne le clivage de la liaison SE-X₁

14. - Procédé in vitro pour détecter, au moyen d'une sonde selon l'une quelconque des revendications 1 à 11, la présence d'une enzyme, dont la présence entraîne le clivage de la liaison SE-X₁.

15. - Procédé selon la revendication 14 comprenant les étapes successives suivantes :
- la mise en contact d'un échantillon, suspecté de contenir ladite enzyme, avec une sonde selon l'une quelconque des revendications 1 à 11;
- l'application de conditions appropriées pour permettre le clivage de la liaison covalente entre X₁ et SE, sous l'action de l'enzyme, qui est suivi d'un clivage de la liaison entre te C(X₂) et le groupe -OA suite à une cyclisation de l'espaceur présent dans la sonde de formule (I), et
- l'analyse quantitative ou qualitative du chromophore ou fluorophore libéré.

16. - Procédé selon la revendication 14 ou 15 **caractérisé en ce qu'**il est mis en oeuvre dans des conditions physiologiques, notamment dans un milieu aqueux, de préférence tamponné à un pH appartenant à la gamme allant de 4 à 9, et par exempte à un pH de l'ordre de 7,4.

17. - Procédé selon l'une quelconque des revendications 14 à 16 **caractérisé en ce qu'**un fluorophore est libéré et son analyse comprend les étapes suivantes :
- exposition du fluorophore à une source lumineuse capable de produire une lumière à une longueur d'onde d'absorption du fluorophore ; et
- détection de la fluorescence résultante.

18. - Procédé selon l'une quelconque des revendications 14 à 16 **caractérisé en ce qu'**un chromophore est libéré et son analyse comprend les étapes suivantes :
- exposition du chromophore à une source lumineuse capable de produire une lumière à une longueur d'onde d'absorption du chromophore ; et
- détection de la lumière absorbée par le chromophore, correspondant à un changement de couleur.

19. - Procédé selon l'une quelconque des revendications 14 à 18 **caractérisé en ce que** le fluorophore ou le chromophore libéré, en particulier sous la forme d'un composé A-OH, A-O⁻ ou d'une forme obtenue après réarrangement, ou d'une forme tautomérique ou polymérisée de A-OH ou A-O⁻, forme un précipité en solution aqueuse.

## Patentansprüche

1. - Sonden der Formel (1) : 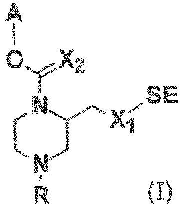 worin:
- entweder X₁ ein Sauerstoff ist und SE ein Substrat von Esterase, Glycosidase, Phosphatase, Sulfatase oder Glucuronidase ist,
- oder X1 NH ist und SE ein Substrat von Protease ist, insbesondere Amidase, Peptidase oder Transferase
- X₂ = O oder S,
- A eine aromatische Gruppe ist, die nach Spaltung der C(X₂)-O-Bindung in wässriger Lösung zur Freisetzung eines Chromophors oder eines Fluorophors führt, wobei diese aromatische Gruppe eine Gruppe ist, die einen oder mehrere substituierte oder unsubstituierte aromatische Ringe umfasst, wobei diese Ringe möglicherweise ein oder mehrere Heteroatome, ausgewählt aus Stickstoff-, Sauerstoff- oder Schwefelatomen, und/oder ein oder mehrere Kohlenstoffatome in Form eines C=O-Carbonyls umfassen,
- R ein Wasserstoffatom oder -(L)n-GP ist, wobei n gleich O oder 1 ist,
- L ein Verbindungsarm ist,
- GP eine hydrosoubilisierende Gruppe ist, die eine F₁-Funktion, ausgewählt aus primären, tertiären und sekundären Aminen, Amidin, Guanidin, Tetrazol; eine anionische F₂-Funktion vom Carboxylat-, Sulfonat- oder Phosphattyp; eine Gruppe, die eine oder mehrere dieser Funktionen F₁ und/oder F₂ umfasst; ein Polyethylenglykol; ein Zucker oder Polysaccharid wie Glucose, Galactose und Mannose; eine Peptidgruppe wie Polylysin, Polyarginin und TAT-Peptide, ist,
sowie deren physiologisch verträgliche Salze,
wobei diese aromatische Gruppe A ausgewählt ist aus Paranitrophenol und seinen Derivaten, Indigoiden, Cyclohexenoescutetin, Alizarin und Hydroxyflavon,
oder diese Gruppe A eine aromatische Verbindung ist, mit -OA ausgewählt aus 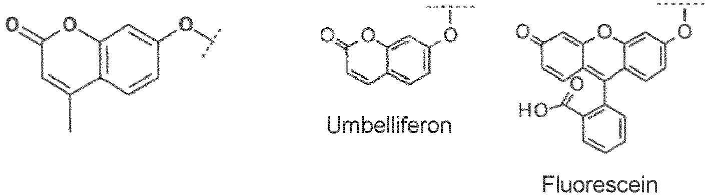 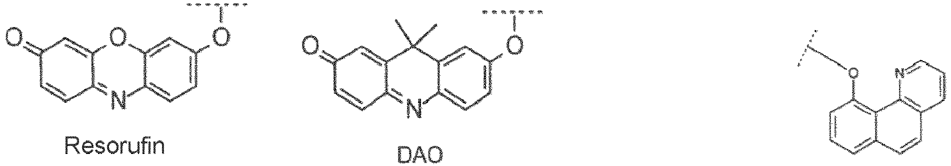 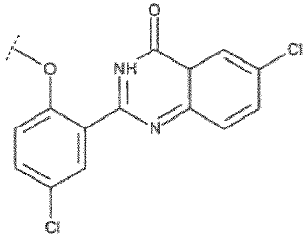 , oder
oder diese Gruppe A eine aromatische Verbindung ist, mit OA, das der Formel (AA) entspricht: 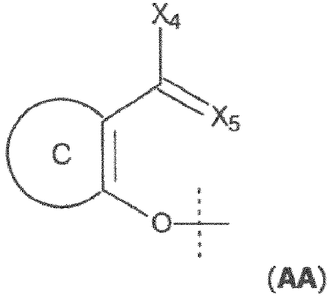
worin:
entweder X₅ ein Sauerstoffatom ist und X₄ eine NH₂-, OH-, SH-, Alkyl-, Aryl-, O-Alkyl-, O-Phenyl-, NH-Alkyl-, NH-Phenyl-, S-Alkyl- oder S-Aryl-Gruppe, wobei diese Alkyl- und Phenyl-Gruppen substituiert oder unsubstituiert sein können,
oder X₅ ein Stickstoffatom ist und an X₄ verknüpft ist, das entsprechend CH, O, S, N oder NH ist, zur Bildung eines substituierten oder unsubstituierten Heteroaryls,
und 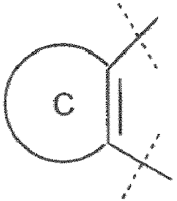 ein Aryl oder ein Heteroaryl ist, substituiert oder unsubstituiert, zum Beispiel ausgewählt aus Phenyl-, Naphthyl-Gruppen, und 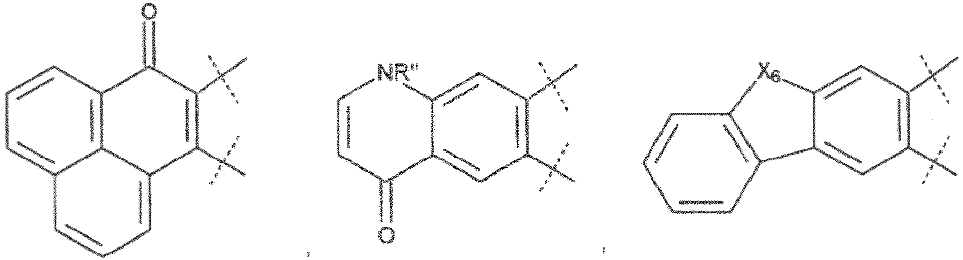
wobei diese Gruppen substituiert oder unsubstituiert sein können,
mit X6, das S, O oder NR" ist, und R", das ein Wasserstoffatom oder eine (C₁-C₄)-Alkyl-Gruppe ist.

2. - Sonden nach Anspruch 1, **dadurch gekennzeichnet, dass** SE aus den Glykosylgruppen ausgewählt ist, die über ihren anomeren Kohlenstoff mit dem Rest des Moleküls verbunden sind, oder SE ein Substrat von Protease oder Peptidase ist und einer Peptidyl- oder Aminosäuregruppe entspricht, die mit dem Rest des Moleküls durch eine Acylfunktion verbunden ist, die an ihrem terminalen Kohlenstoff oder durch eine Seitenbindung getragen wird.

3. - Sonden nach einem der Ansprüche 1 bis 2, **dadurch gekennzeichnet, dass** SE ein Glycosidase-Substrat ist, beispielsweise N-Acetyl-3-galactosaminidase; N-Acetyl-p-Glucosaminidase; a-Amylase; a-Arabinofuranosidase; a-Arabinosidase; 13-Cellobiosidase; 13-Chitobiosidase; a-Galactosidase; 13-Galactosidase; a-Glucosidase; 13-Glucosidase; 13-Glucuronidase; a-Maltosidase; a-Mannosidase; 13-Mannosidase; 13-Xylosidase; 13-D-Fucosidase; a-L-Fucosidase; 13-L-Fucosidase; L-Iduronidase oder Cellulase; und entspricht einer Glycosylgruppe, die über ihren anomeren Kohlenstoff mit dem Rest des Moleküls verbunden ist, und insbesondere SE eine monoglycosylierte Gruppe ist, ausgewählt aus Galactosyl, Glucosyl, Mannosyt, Gulosyl, Altosyl, Altrosyl, Idosyl, Talosyl, Fucosyl, Fructosyl, Arabinosyl, Lyxosyl, Ribosyl, Xylosyl, Glucuronyl und N-Acetylhexosaminyl-Gruppe oder eine polyglykosylierte Gruppe bestehend aus mehreren, beispielsweise 2 bis 20, vorzugsweise 3 bis 10 und insbesondere 4 bis 6 dieser gleichen oder verschiedenen monoglykosylierten Gruppen.

4. - Sonden nach einem der Ansprüche 1 bis 2, **dadurch gekennzeichnet, dass** SE ein Substrat von Galactosidase ist, beispielsweise β-Galactosidase, Induronidase, Glucosidase, N-Acetyl-D-Glucosaminidase, N-Acetyl-D-Galactosaminidase, Mannosidase, Fucosidase oder Glucuronidase, insbesondere β--Glucuronidase; und einer Glycosylgruppe entspricht, die über ihren anomeren Kohlenstoff mit dem Rest des Moleküls verbunden ist, und insbesondere SE eine monoglycosylierte Gruppe ist, ausgewählt aus D-Glucuronyl, L-Iduronyl, D-Glucopyranosyl, D-Galactopyranosyl, N-Acetyl- D-Glucosaminyl, N-Acetyl-D-Galactosaminyl, D-Mannopyranosyl, L-Fucopyranosyl oder eine polyglykosylierte Gruppe bestehend aus mehreren, beispielsweise 2 bis 20, vorzugsweise 3 bis 10 und insbesondere 4 bis 6, derselben oder verschiedene monoglykosylierte Gruppen.

5. - Sonden nach einem der Ansprüche 1 bis 2, **dadurch gekennzeichnet, dass** SE ist:
- entweder ein Substrat einer Protease oder einer Peptidase, die ein Substrat von Cathepsin oder Metalloprotease ist, insbesondere PSMA (Prostataspezifisches Membranantigen; Prostate Specific Membrane Antigen); und SE einer Peptidyl- oder Aminosäuregruppe ist, die über eine Acylfunktion, die von ihrem terminalen Kohlenstoff oder einer Seitenbindung getragen wird, mit dem Rest des Moleküls verbunden ist;
- oder ein Substrat einer Transferase, insbesondere ein Substrat von Glutathiontransferase oder der Gamma-Glutamyltransferase, und SE eine Peptidyl- oder Aminosäuregruppe ist, die über eine Acylfunktion, die von ihrem terminalen Kohlenstoff oder der einer Seitenbindung getragen wird, mit dem Rest des Moleküls verbunden ist, oder SE eine Sulfonylgruppe ist, die mit X₁ ein Sulfonamid bildet, das dann NH darstellt.

6. - Sonden nach einem der Ansprüche 1 bis 5, **dadurch gekennzeichnet, dass** A eine aromatische Gruppe mit -OA vom Phenoxytyp ist und den folgenden bevorzugten Strukturen (BB) oder (CC) entspricht: 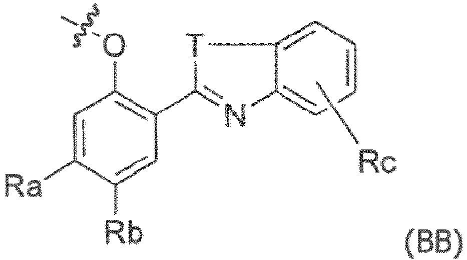 worin
- T -NH-C(O)-, -O-, -NH, N-Alkyl oder N-Aryl ist,
- Ra Wasserstoff oder ein elektronenziehender Kohlenstoffsubstituent ist, wie -CN oder -COORd, wobei Rd eine (C₁-C₄)-Alkylgruppe ist, oder Ra -CONReRf ist, wobei Re und Rf, gleich oder verschieden, Wasserstoff oder eine (C₁-C₄)Alkylgruppe sind, oder Ra -CF₃ oder eine 2-Oxazolyl-, 2-Thiazolyl-, 2-Imidazolyl-, 2-Benzimidazolyl-, 4-Pyrimidinon- 2-yl- oder Chinazolinon-2-yl-Gruppe ist,
- Rb Wasserstoff, ein Chlor-, Brom-, Jod- oder Fluor-Atom, -OH, -NH2, -NRgRh, -NHRg oder -ORg ist, wobei Rg und Rh jeweils unabhängig voneinander ein (C₁-C₄)-Alkyl sind,
- oder Ra und Rb miteinander verbunden sind, um eine Kohlenwasserstoffkette zu bilden, die 4 oder 5 Glieder umfasst, gesättigt oder ungesättigt, substituiert oder unsubstituiert, gegebenenfalls unterbrochen durch ein oder mehrere Heteroatome, ausgewählt aus N, S und O,
- Re Wasserstoff, Br, Cl, I oder F ist, 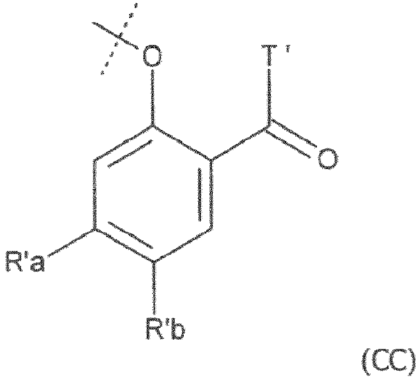
worin:
- T' NH₂, OH, eine Arylgruppe, eine (C₁-C₄)Alkylgruppe, SH, NHR'c, OR'c, NR'cR'd oder SR'c ist, wobei R'c und R'd, gleich oder verschieden, eine (C1-C4)Alkyl- oder Arylgruppe sind,
- R'a Wasserstoff oder ein elektronenziehender Kohlenstoffsubstituent, wie -CN oder - COOR'e, ist, wobei R'e eine (C1-C4)-Alkylgruppe ist, oder R'a -CONRfR'g ist, wobei R'f und R'g gleich oder verschieden, Wasserstoff oder eine (C₁-C₄)-Alkylgruppe sind, oder R'a -CF₃ oder eine 2-Oxazolyl-, 2-Thiazolyl-, 2-lmidazolyl-, 2-Benzimidazolyl-, 4-Pyrimidinon-2-yl- oder Chinazolinon-2-yl-Gruppe ist,
- R'b Wasserstoff, ein Chlor-, Brom-, Jod- oder Fluor-Atom, -OH, -NH2, -NR'hR'i oder
- OR'h ist, wobei R'h und R'i, gleich oder verschieden, eine (C₁-C₄)Alkylgruppe sind,
- oder R'a und R'b miteinander verbunden sind, um eine Kohlenwasserstoffkette zu bilden, die 4 oder 5 Glieder umfasst, gesättigt oder ungesättigt, substituiert oder unsubstituiert, gegebenenfalls unterbrochen durch ein oder mehrere Heteroatome, ausgewählt aus N, S und O.

7. - Sonden nach einem der Ansprüche 1 bis 6, **dadurch gekennzeichnet, dass** R für -(L)n-GP, wobei n = 1 und L ein Verbindungsarm und insbesondere ein Arm -(L1)m1-(L2)m2-(L'1)m'1-ist, definiert in Richtung Piperazin -> GP-Gruppe, wobei:
- L1 und L'1, gleich oder verschieden, ausgewählt sind aus -O-, -NH, -N(C₁₋₆-Alkyl)-, - N(Phenyl)-, -N(Aryl)-, C(O)- , -C(O)O, -OC(O)- -OC(O)-O-, -NHC(O)-O- -OC(O)-NH-, - NHC(O)-NH- -S -, -SO₂, -N=N-, -NHC(O)- und -CONH-;
- L2 aus den folgenden zweiwertigen Gruppen ausgewählt ist: (C₁₋₂₀)Alkyl, (C₁₋₂₀)Alkenyl, (C₁₋₂₀)Alkinyl, (C₆₋₂₄)Aryl, (C₇₋₄₄)Alkylaryl, (C₇₋₄₄) Alkenylaryl, (C₇₋₄₄)Alkinylaryl, (C_{7- 44})Alkylcycloalkyl, (C₇₋₄₄)Alkenylcycloalkyl, (C₇₋₄₄)Alkinylcycloalkyl, (C₇₋₄₄)Alkylheterocycloalkyl, (C₇₋₄₄)Alkenylheterocycloalkyl, (C₇₋₄₄)Alkinylheterocycloalkyl; wobei diese Gruppen mit einer Triazolgruppe unterbrochen oder enden können und unsubstituiert oder substituiert sein können, insbesondere durch einen oder mehrere Substituenten ausgewählt aus (C₁₋₁₀)-Alkoxy, (C₁₋₁₀)-Alkyl, (C₆₋₁₀)-Aryl, Amido, Imido, Phosphido, Nitrido, (C₁₋₁₀)Alkenyl, (C₁₋₁₀)Alkinyl und -OH;
- m1, m'1 und m2, , gleich oder verschieden, gleich 0 oder 1 sind und
- GP wie in Anspruch 1 definiert ist.

8. - Sonden nach Anspruch 7, **dadurch gekennzeichnet, dass** L -(L1)m1-(L2)m2-(L '1)m'1 ist, wobei L1 = -C(O)-, m1 = m2 = 1, m'1 = 1 oder 0 ist und L2 und L'1 wie in Anspruch 14 definiert, sind, und insbesondere L -C(O)-(CH₂)p-L3- ist, wobei p gleich 1, 2, 3 oder 4 ist und L3 eine Triazolgruppe, und insbesondere eine 1H-1,2,3-Triazolgruppe, ist.

9. - Sonden nach einem der Ansprüche 1 bis 8, **dadurch gekennzeichnet, dass** X₂ ein Sauerstoffatom ist.

10. - Sonden nach einem der Ansprüche 1 bis 9, **dadurch gekennzeichnet, dass**:
- X₂ ein Sauerstoffatom ist,
- X₁ entweder ein Sauerstoffatom ist und SE ein Substrat von Glycosidase oder Glucuronidase ist und einer Glycosylgruppe entspricht, die über ihren anomeren Kohlenstoff mit dem Rest des Moleküls verbunden ist, oder SE ein Substrat von Esterase ist und einer -C(O)Ri-Gruppe entspricht, wobei Ri beispielsweise eine Alkylgruppe mit vorzugsweise 1 bis 20 Kohlenstoffatomen, eine Alkenylgruppe mit vorzugsweise 1 bis 20 Kohlenstoffatomen, eine Benzyl-, Aryl- oder Heteroarylgruppe ist,
- oderX1 für NH ist und SE ein Substrat von Protease- oder Peptidase ist und einer Peptidyl- oder Aminosäuregruppe entspricht, die über eine Acylfunktion an ihrem terminalen Kohlenstoff oder über einen Seitenlink mit dem Rest des Moleküls verbunden ist; und
- R für -L-GP steht, wobei:
• L -(L1)m1-(L2)m2-(L'1)m'1 ist, wobei L1 = -C(O)-, m1 = m2 = 1, m'1 = 1 oder 0 ist und L2 und L'1 wie in Anspruch 10 definiert sind, und insbesondere L -C(O)-(CHz)p-L3- ist, wobei p gleich 1, 2, 3 oder 4 ist und L3 eine Triazolgruppe ist und insbesondere 1H-1,2,3-Triazol-Gruppe und
• GP so ist, wie in Anspruch 1 definiert.

11. - Sonden nach Anspruch 1 ausgewählt aus:
4-Methyl-2-oxo-2H-chromen-7-yl-2-((2-phenylacetamido)methyl)piperazin-I-carboxylat-hydrochlorid (Verbindung **12**): 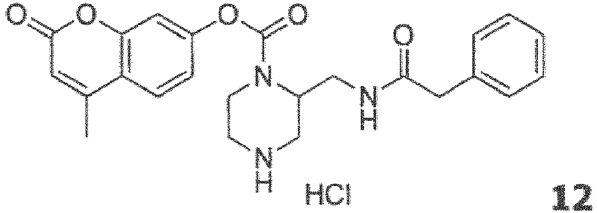
4-Methyl-2-oxo-2H-chromen-7-yl-2-((octanoyloxy)methyl)piperazin-1-carboxylathydrochlorid (Verbindung **21**) 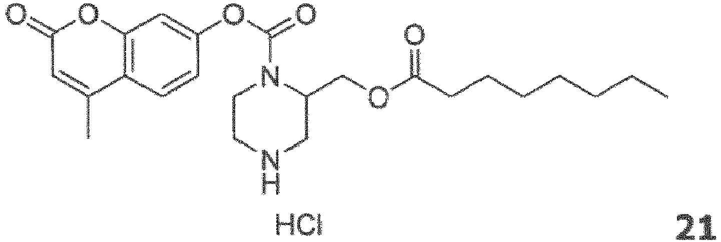
4-4-(((4-Methyl-2-oxo-2H-chromen-7-yl)oxy)carbonyl)-3-((octanoloxy)methyl)pipera2in-1-yl)butan-1-sulfonsäure (Verbindung **22**) 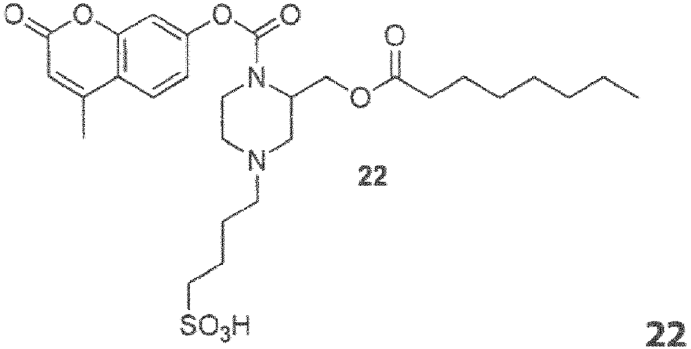
4-Methyl-2-oxo-2H-chromen-7-yl 4-(3-(1-(2-(2-(2-methoxyethoxy)ethyl)-1H-1,2,3-triazol-4-yl )Propanoyl)-2-((2-phenylacetamido)methyl)piperazin-1-carboxylat (Verbindung **31**) 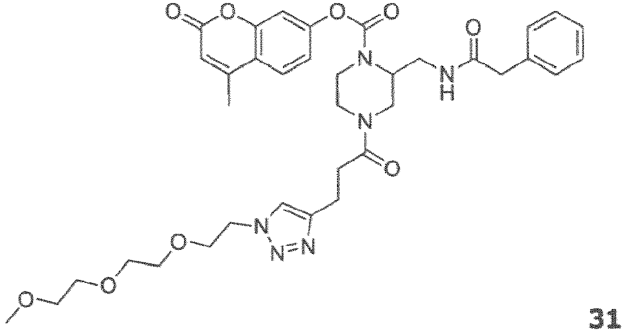
4-Nitrophenyl 4-(3-(1-(2-(2-(2-methoxyethoxy)ethoxy)ethyl)-1H-1,2,3-triazol-4-yl)propanoyl)-2-((2-Phenylacetamido)methyl)piperazin-1-carboxylat (Verbindung **36**) 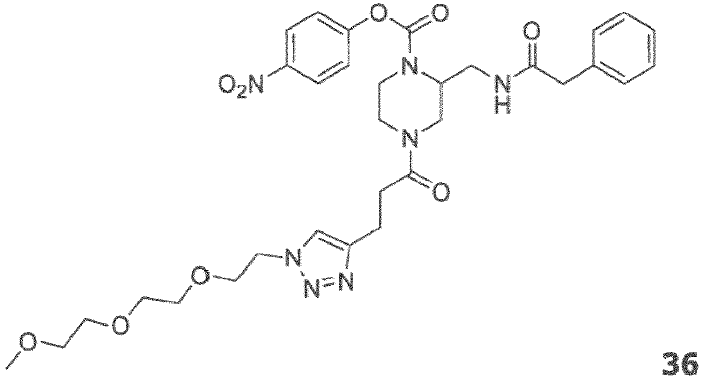
4-Chlor-2-(6-chlor-4-oxo-3,4-dihydrochinazolin-2-yl)phenyl 4-(3-(1-(2-(2- (2-methoxyethoxy)ethoxy)ethyl)- 1H-1,2,3-Triazol-4-yl)propanoyl)-2-((2-phenylacetamido)methyl)piperazin-1-carboxylat (Verbindung **39**)

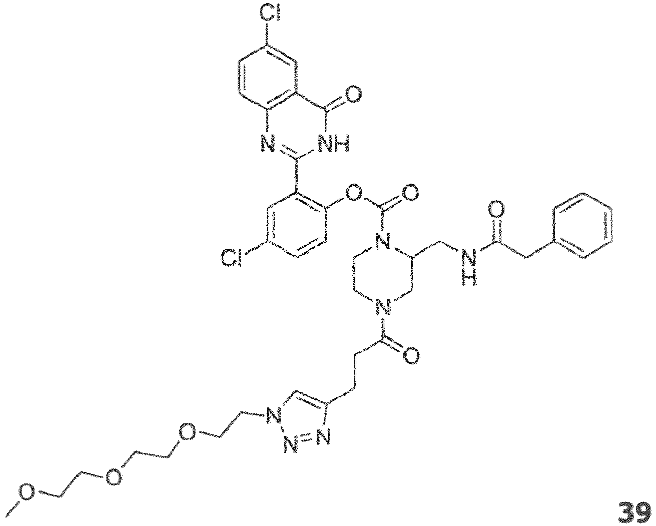

12. - Intermediate der Formel: 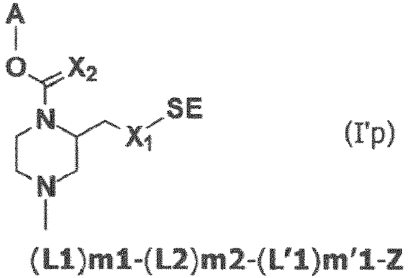 und 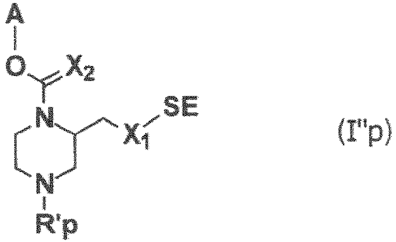 worin:
A, X₁, X₂ SE, L1, L'1, L2, m1, m'1 und m2 wie unter (1) in den Ansprüchen 1 bis 10 definiert sind,
Z C≡CH, N₃, eine N-Oxysuccinimid- oder Maleimidfunktion ist und
R'p eine Gruppe ist, die die Aminofunktionen schützt, vorzugsweise ausgewählt aus Benzylgruppen, Ihren -C(O)OR'₁-Gruppen, wobei R'₁ eine Alkyl- oder Alkenylgruppe mit 1 bis 12 Kohlenstoffatomen oder eine Gruppe -(CH2)ₘ₃R"₁, wobei R"₁ eine Aryl-, Cycloalkyl- oder Fluorenylgruppe darstellt und m3 gleich 0, 1, 2 oder 3 ist, wie Carbobenzyloxy-, ter-Butyloxycarbonyl-, 9-Fluorenylmethyloxycarbonyl- und Allyloxycarbonylgruppen,
sowie deren Salze, Solvate oder Hydrate.

13. - Sonden nach einem der Ansprüche 1 bis 11 für den in-vivo-Nachweis eines Enzyms bei Menschen oder Tieren, dessen Anwesenheit die Spaltung der SE-X₁-Bindung verursacht

14. - *In-vitro-*Verfahren zum Nachweis der Anwesenheit eines Enzyms, dessen Anwesenheit die Spaltung der SE-X₁-Bindung verursacht, mittels einer Sonde nach einem der Ansprüche 1 bis 11.

15. - Verfahren nach Anspruch 14, umfassend die folgenden aufeinanderfolgenden Schritte :
- das Inkontaktbringen einer Probe, von der vermutet wird, dass sie dieses Enzym enthält, mit einer Sonde nach einem der Ansprüche 1 bis 11;
- die Anwendung geeigneter Bedingungen, um die Spaltung der kovalenten Bindung zwischen X1 und SE unter der Wirkung des Enzyms zu ermöglichen, gefolgt von einer Spaltung der Bindung zwischen dem C(X₂) und der -OA-Gruppe infolge einer Zyklisierung des Spacers, der in der Sonde der Formel (1) vorhanden ist, und
- die quantitative oder qualitative Analyse des freigesetzten Chromophors oder Fluorophors.

16. - Verfahren nach Anspruch 14 oder 15, **dadurch gekennzeichnet, dass** es unter physiologischen Bedingungen durchgeführt wird, insbesondere in einem wässrigen Medium, vorzugsweise gepuffert bei einem pH-Wert im Bereich von 4 bis 9, beispielsweise bei einem pH-Wert um 7,4.

17. - Verfahren nach einem der Ansprüche 14 bis 16, **dadurch gekennzeichnet, dass** ein Fluorophor freigesetzt wird und seine Analyse die folgenden Schritte umfasst:
- Aussetzen des Fluorophors einer Lichtquelle, die Licht bei einer Absorptionswellenlänge des Fluorophors erzeugen kann; und
- Detektion der resultierenden Fluoreszenz.

18. - Verfahren nach einem der Ansprüche 14 bis 16, **dadurch gekennzeichnet, dass** ein Chromophor freigesetzt wird und seine Analyse die folgenden Schritte umfasst:
- Aussetzen des Chromophors einer Lichtquelle, die Licht bei einer Absorptionswellenlänge des Chromophors erzeugen kann; und
- Erkennung des vom Chromophor absorbierten Lichts, das einer Farbänderung entspricht.

19. - Verfahren nach einem der Ansprüche 14 bis 18, **dadurch gekennzeichnet, dass** das freigesetzte Fluorophor oder Chromophor, das insbesondere in Form einer A-OH-, A-O⁻-Verbindung oder einer nach Umlagerung erhaltenen Form oder einer tautomeren oder polymerisierten Form von A -OH oder A-O⁻ vorliegt, in wässriger Lösung einen Niederschlag bildet.

## Claims

1. Probes with formula (1): 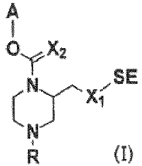 in which:
- either X₁ is an oxygen and SE is an esterase, glycosidase, phosphatase, sulfatase, or glucuronidase substrate,
- or X₁ represents NH and SE is a protease, in particular amidase, peptidase or transferase substrate,
- X₂ = O or S,
- A is an aromatic group that, after cleavage of the C(X₂)-O bond in aqueous solution, leads to the liberation of a chromophore or a fluorophore, said aromatic group being a group comprising one or more substituted or unsubstituted aromatic rings, said rings possibly comprising one or more heteroatoms selected from nitrogen, oxygen or sulfur atoms and/or one or more carbon atoms in the form of a carbonyl C=O,
- R represents a hydrogen atom or -(L)n-GP, with n equal to 0 or 1,
- L is a linker arm,
- GP is a hydrosolubilizing group which is a function F₁ selected from primary, tertiary and secondary amines, amidine, guanidine, tetrazole; an anionic function F₂ of the carboxylate, sulfonate or phosphate type; a group comprising one or more of these functions F₁ and/or F₂; a polyethylene glycol; a sugar or polysaccharide such as glucose, galactose and mannose; a peptide group such as poly-lysine, poly-arginine and TAT-peptides,
as well as their physiologically acceptable salts,
said aromatic group A being selected from para-nitrophenol and its derivatives, indigoids, cyclohexenoescutetin, alizarin and hydroxyflavone,
or said group A being an aromatic compound with -OA selected from 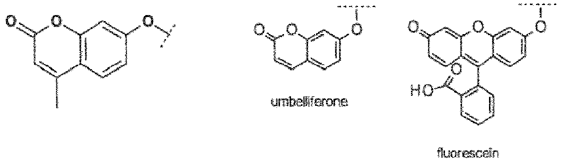 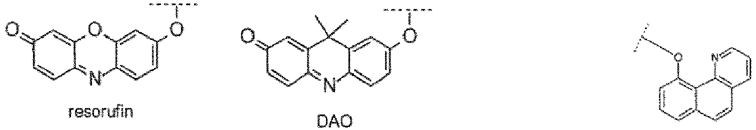 or 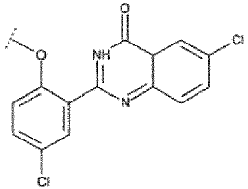
or said group A being an aromatic compound with -OA which corresponds to the formula (AA): 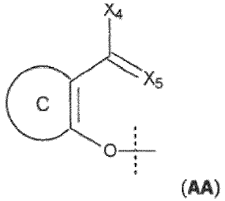
in which:
either X₅ is an oxygen atom and X₄ is a -NH₂, -OH, -SH, alkyl, aryl, -O-alkyl, -O-phenyl, -NH-alkyl, -NH-phenyl, -S-alkyl or -S-aryl group, said alkyl and phenyl groups possibly being substituted or unsubstituted,
or X₅ represents a nitrogen atom and is bonded to X₄ which then represents CH, O, S, N or NH to form a substituted or unsubstituted heteroaryl,
and 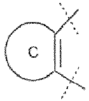 represents an aryl or a heteroaryl, substituted or unsubstituted, for example selected from phenyl, naphthyl groups, and 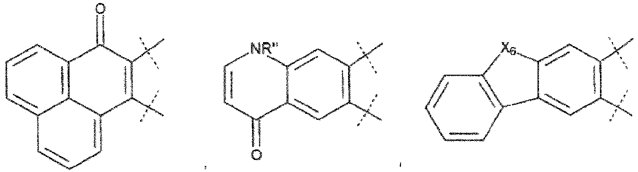
said groups possibly being substituted or unsubstituted,
with X₆ which represents S, O or NR", and R" which represents a hydrogen atom or a (C₁-C₄)alkyl group.

2. The probes according to claim 1 **characterized in that** SE is selected from the glycosyl groups bonded to the remainder of the molecule via their anomeric carbon or SE is a protease or peptidase substrate and corresponds to a peptidyl or amino acid group bonded to the remainder of the molecule via an acyl function carried by their terminal carbon or by a side chain.

3. The probes according to any one of claims 1 to 2 **characterized in that** SE is a glycosidase substrate, for example N-acetyl-β-galactosaminidase; N-acetyl-β-glucosaminidase; α-amylase; α-arabinofuranosidase; α-arabinosidase; β-cellobiosidase; β-chitobiosidase; α-galactosidase; β-galactosidase; α-glucosidase; β-glucosidase; β-glucuronidase; α-maltosidase; α-mannosidase; β-mannosidase; β-xylosidase; β-D-fucosidase; α-L-fucosidase; β-L-fucosidase; L-iduronidase or cellulase; and corresponds to a glycosyl group bonded to the remainder of the molecule via its anomeric carbon, and in particular, SE is a monoglycosylated group selected from galactosyl, glucosyl, mannosyte, gulosyl, altosyl, altrosyl, idosyl, talosyl, fucosyl, fructosyl, arabinosyl, lyxosyl, ribosyl, xylosyl, glucuronyl and N-acetyl-hexosaminyl or a polyglycosylated group constituted by a plurality, for example from 2 to 20, preferably 3 to 10, and more particularly 4 to 6, of these identical or different monoglycosylated groups.

4. The probes according to any one of claims 1 to 2 **characterized in that** SE is a substrate of galactosidase, for example β-galactosidase, induronidase, glucosidase, N-acetyl-D-glucosaminidase, N-acetyl-D-galactosaminidase, mannosidase, fucosidase or glucuronidase, in particular β-glucuronidase; and corresponds to a glycosylated group bonded to the remainder of the molecule via its anomeric carbon, and in particular, SE is a monoglycosylated group selected from D-glucuronyl, L-iduronyl, D-glucopyranosyl, D-galactopyranosyl, N-acetyl-D-glucosaminyl, N-acetyl-D-galactosaminyl, D-mannopyranosyl, L-fucopyranosyl or a polyglycosylated group constituted by a plurality, for example from 2 to 20, preferably 3 to 10, and more particularly 4 to 6, of these identical or different monoglycosylated groups.

5. The probes according to any one of claims 1 to 2 **characterized in that** SE is:
- either a substrate of a protease or a peptidase, which is a substrate of cathepsin or metalloprotease, in particular PSMA (Prostate Specified Membrane Antigen); and SE corresponds to a peptidyl or amino acid group bonded to the remainder of the molecule via an acyl function carried by its terminal carbon or by a side chain;
- or a substrate of a transferase, in particular a substrate of glutathione transferase or gamma-glutamyl transferase, and SE corresponds to a peptidyl or amino acid group bonded to the remainder of the molecule via an acyl function carried by its terminal carbon or by a side chain or SE corresponds to a sulfonyl group forming a sulfonamide with X₁ which then represents NH.

6. The probes according to any one of claims 1 to 5 **characterized in that** A is an aromatic group with -OA which is phenoxy in type, and corresponds to the following preferred structures (BB) or (CC): 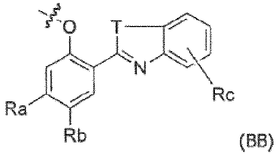 in which
- T is -NH-C(O)-, -O-, -NH, N-alkyl or N-aryl,
- Ra is hydrogen or an electron-attracting carbon-containing substituent, such as - CN or -COORd, with Rd which represents a (C₁-C₄)alkyl group, or Ra is -CONReRf, with Re and Rf, identical or different, which represent hydrogen or a (C₁-C₄)alkyl group, or Ra is -CF₃ or a 2-oxazolyl, 2-thiazolyl, 2-imidazolyel, 2-benzoimidazolyl, 4-pyrimidinon-2-yl or quinazolinon-2-yl group,
- Rb is hydrogen, a chlorine, bromine, iodine or fluorine atom, -OH, -NH₂, -NRgRh, -NHRg or -ORg, with Rg and Rh which each independently represent a (C₁-C₄)alkyl,
- or Ra and Rb are bonded together to form a hydrocarbon chain comprising 4 or 5 links, saturated or unsaturated, substituted or unsubstituted, optionally interrupted by one or more heteroatoms selected from N, S and O,
- Re is hydrogen, Br, Q, I or F, 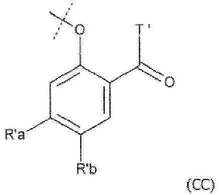
in which:
- T' is NH₂, OH, an aryl group, a (C₁-C₄)alkyl group, SH, NHR'c, OR'c, NR'cR'd or SR'c, with R'c and R'd, identical or different, which represent a (C₁-C₄)alkyl or aryl group,
- R'a is hydrogen or an electron-attracting carbon-containing substituent such as - CN, or -COOR'e, with R'e which represents a (C₁-C₄)alkyl group, or R'a is -CONRfR'g, with R'f and R'g, identical or different, which represent hydrogen or a (C₁-C₄)alkyl group, or R'a is -CF₃ or a 2-oxazolyl, 2-thiazolyl, 2-imidazolyl, 2-benzoimidazolyl, 4-pyrimidinon-2-yl or quinazolinon-2-yl,
- R'b is hydrogen, a chlorine, bromine, iodine or fluorine atom, -OH, -NH₂, - NR'hR'i or -OR'h, with R'h and R'i, identical or different, which represent a (C₁-C₄)alkyl group,
- or R'a and R'b are bonded together to form a hydrocarbon chain comprising 4 or 5 links, saturated unsaturated, substituted or unsubstituted, optionally interrupted by one or more heteroatoms selected from N, S and O.

7. The probes according to any one of claims 1 to 6 **characterized in that** R represents -(L)n-GP with n = 1 and L is a linker arm, and in particular a -(L1)m1-(L2)m2-(L'1)m'1- arm, defined in the direction piperazine -> group GP with:
- L1 and L'1, identical or different, which are selected from -O-, -NH, -N(C₁₋₆) alkyl, -N(phenyl)-, -N(aryl)-, C(O)-, -C(O)O, -OC(O)- -OC(O)-O-, -NHC(O)-O- - OC(O)-NH-, -NHC(O)-NH- -S-, -SO₂, -N=N-, -NHC(O)- and -CONH-;
- L2 which is selected from the following bivalent groups: (C₁₋₂₀)alkyl, (C₁₋₂₀)alkenyl, (C₁₋₂₀)alkynyl, (C₆₋₂₄)aryl, (C₇₋₄₄)alkylaryl, (C₇₋₄₄)alkenylaryl, (C₇₋₄₄)alkynylaryl, (C₇₋₄₄)alkylcycloalkyl, (C₇₋₄₄)alkenylcycloalkyl, (C₇₋₄₄)alkynylcycloalkyl, (C₇₋₄₄)alkylheterocycloalkyl, (C₇₋₄₄)alkenylheterocycloalkyl, (C₇₋₄₄)alkynylheterocycloalkyl; said groups possibly being interrupted by or terminating in a triazole group and possibly being unsubstituted or substituted, in particular with one or more substituents selected from (C₁-₁₀)alkoxy, (C₁-₁₀)alkyl, (C₆₋₁₀)aryl, amido, imido, phosphide, nitrido, (C₁-₁₀)alkenyl, (C₁-₁₀)alkynyl and -OH;
- m1, m'1 and m2, identical or different, which are equal to 0 or 1 and
- GP being as defined in claim 1.

8. The probes according to claim 7 **characterized in that** L represents - (L1)m1-(L2)m2-(L'1)m'1 with L1 = -C(O)-, m1 = m2 = 1, m'1 = 1 or 0 and L2 and L'1 as defined in claim 14, and in particular L represents -C(O)-(CH₂)p-L3- with p which is equal to 1, 2, 3 or 4 and L3 which is a triazole group and in particular a 1H-1,2,3-triazole group.

9. The probes according to any one of claims 1 to 8 **characterized in that** X₂ is an oxygen atom.

10. The probes according to any one of claims 1 to 9 **characterized in that**:
- X₂ is an oxygen atom,
- either X₁ is an oxygen atom and SE is a glycosidase or glucuronidase substrate and corresponds to a glycosyl group bonded to the remainder of the molecule via its anomeric carbon or SE is an esterase substrate and corresponds to a -C(O)Ri group with Ri which represents, for example, an alkyl group preferably containing 1 to 20 carbon atoms, an alkenyl group preferably containing 1 to 20 carbon atoms, a benzyl, aryl or heteroaryl group,
- or X₁ represents NH and SE is a protease or peptidase substrate and corresponds to a peptidyl or amino acid group bonded to the remainder of the molecule via an acyl function carried by its terminal carbon or by a side chain; and
- R represents -L-GP with:
• L which represents -(L1)m1-(L2)m2-(L' 1)m'1 with L1 = -C(O)-, m1 = m2 = 1, m'1 = 1 or 0 and L2 and L'1 as defined in claim 10, and in particular L represents - C(O)-(CH₂)p-L3- with p which is equal to 1, 2, 3 or 4 and L3 which is a triazole group and in particular a group 1H-1,2,3-triazole, and
• GP which is as defined in claim 1.

11. The probes according to claim 1 selected from:
4-methyl-2-oxo-2H-chromen-7-yl2-((2-phenylacetamido)methyl)piperazine-1-carboxylate hydrochloride (Compound 12): 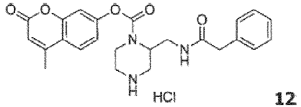
4-methyl-2-oxo-2H-chromen-7-yl2-((octanoyloxy)methyl)piperazine-1-carboxylate hydrochloride (Compound 21) 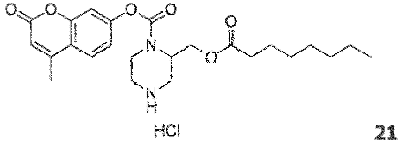
4-4-(((4-methyl-2-oxo-2H-chromen-7-yl)oxy)carbonyl)-3-((octanoloxy)methyl)piperazin-1-yl)butane-1-sulfonic acid (Compound 22) 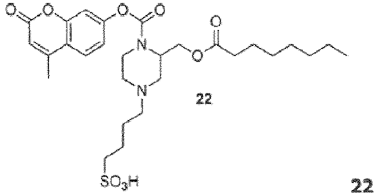
4-methyl-2-oxo-2H-chromen-7-yl 4-(3-(1-(2-(2-(2-methoxyethoxy)ethyl)-1H-1,2,3-triazol-4-yl)propanoyl)-2-((2-phenylacetamido)methyl)piperazine-1-carboxylate (Compound 31) 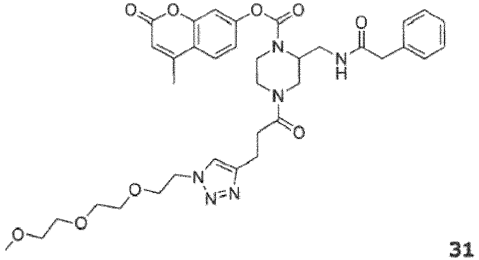
4-nitrophenyl 4-(3-(1-(2-(2-(2-methoxyethoxy)ethoxy)ethyl)-1H-1,2,3-triazol-4-yl)propanoyl)-2-((2-phenylacetamido)methyl)piperazine-1-carboxylate (Compound 36) 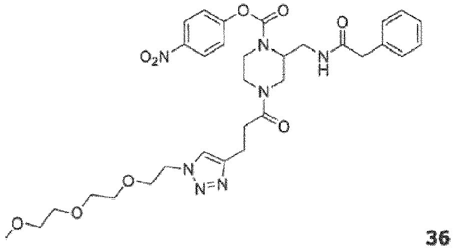
4-chloro-2-(6-chloro-4-oxo-3,4-dihydroquinazolin-2-yl)phenyl 4-(3-(1-(2-(2-(2-methoxyethoxy)ethoxy)ethyl)-1H-1,2,3-triazol-4-yl)propanoyl)-2-((2-phenylacetamido)methyl)piperazine-1-carboxylate (Compound 39)

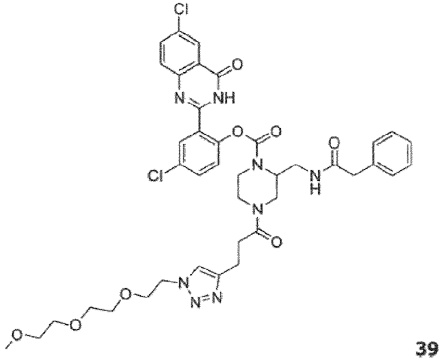

12. Intermediate compounds with formula: 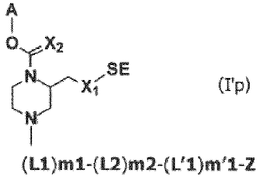 and 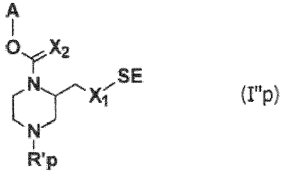 in which:
A, X₁, X₂ SE, L1, L'1, L2, m1, m'1, and m2 are as defined for (I) in claims 1 to 10,
Z represents C=CH, N3, a N-oxysuccinimide or maleimide function, and
R'p represents a protective group for the amine functions, preferably selected from benzyl groups, -C(O)OR' 1 groups with R'₁ which represents an alkyl or alkenyl group containing 1 to 12 carbon atoms or a -(CH₂)ₘ₃R"₁ group with R"₁ which represents an aryl, cycloalkyl or fluorenyl group and m3 which is equal to 0, 1, 2 or 3, such as carbobenzyloxy, ter-butyloxycarbonyl, 9-fluorenylmethyloxycarbonyl, and allyloxycarbonyl groups, as well as their salts, solvates or hydrates.

13. The probes according to any one of claims 1 to 11, for in vivo detection, in a human or animal, of an enzyme, the presence of which causes cleavage of the SE-X₁ bond.

14. An in vitro method of detecting the presence of an enzyme, the presence of which causes cleavage of the SE-X₁ bond, by means of a probe according to any one of claims 1 to 11.

15. The method according to claim 14 comprising the following successive steps:
- bringing a sample, suspected of containing said enzyme, into contact with a probe according to any one of claims 1 to 11;
- applying appropriate conditions to permit cleavage of the covalent bond between X₁ and SE, under the action of the enzyme, which is followed by a cleavage of the bond between the C(X₂) and the -OA group following cyclization of the linker present in the probe with formula (I), and
- quantitatively or qualitatively analyzing the liberated chromophore or fluorophore.

16. The method according to claim 14 or 15 **characterized in that** it is carried out under physiological conditions, in particular in an aqueous medium, preferably buffered to a pH in the range 4 to 9, and for example to a pH of the order of 7.4.

17. The method according to any one of claims 14 to 16 **characterized in that** a fluorophore is liberated and its analysis comprises the following steps:
- exposing the fluorophore to a source of light that is capable of producing light at an absorption wavelength of the fluorophore; and
- detecting the resulting fluorescence.

18. Method according to any one of claims 14 to 16 **characterized in that** a chromophore is liberated and its analysis comprises the following steps:
- exposing the chromophore to a source of light that is capable of producing light at an absorption wavelength of the chromophore; and
- detecting the light absorbed by the chromophore, corresponding to a change in color.

19. The method according to any one of claims 14 to 18 **characterized in that** the liberated fluorophore or chromophore, in particular in the form of a compound A-OH, A-O- or a form obtained after rearrangement, or a tautomeric or polymerized form of A-OH or A-O- forms a precipitate in aqueous solution.
